# EUROPEAN PATENT SPECIFICATION

(11) **EP 0 520 443 B1**
(45) Date of publication and mention of the grant of the patent: **08.01.1997**
(21) Application number: 92110713.2
(22) Date of filing: 25.06.1992
(51) Int. Cl.: A61B 5/14, G01N 27/416, G01N 33/49

(54) **Electrochemical sensor assembly**
Elektrochemischer Sensoraufbau
Construction d'un capteur électrochimique

(30) Priority: 26.06.1991 US 721025; 26.06.1991 US 721027; 26.06.1991 US 721028; 26.06.1991 US 721030; 18.05.1992 US 885096
(43) Date of publication of application: 30.12.1992
(73) Proprietor: PPG INDUSTRIES, INC., Pittsburgh Pennsylvania 15272 (US)
(72) Inventor: Betts, Ronald Eugene, La Jolla, California 92037 (US); Graves, Jeffrey Arthur, San Juan Capistrano, California 92675 (US); Hillier, Douglas Raymond, San Juan Capistrano, California 92675 (US); Leader, Matthew James, Laguna Niguel, California 92677 (US); Koerner, Richard John, San Diego, California 92109 (US); Savage, Douglas Robert, Del Mar, California 92014 (US); Sherman, Marshall Lee, Cardiff, California 92007 (US); Sin van, Kee, Lino Lakes, Minn.55014 (US)
(74) Representative: Sternagel, Hans-Günther, Dr.

(56) References cited:
- WO-A-90/04351
- AT-B- 376 117
- US-A- 3 049 118
- US-A- 4 734 784
- US-A- 4 929 429
- US-A- 5 046 496
- US-A- 5 096 669

## Description

The present invention is directed to a sensor apparatus having hydrated membranes present on a nonconducting substrate that also has present an electrical circuit means. More particularly, in this aspect the present invention is directed to the sensor apparatus having hydrated membranes on a ceramic substrate that has an integrated circuit produced by such techniques as silk screening, thick film and/or thin film processing.

Also the present invention is related to an improved electronic wiring substrate like a wiring board used in the above defined sensor apparatus.

Numerous methods and apparatus exist in the art for measuring chemical components of fluids. For instance, when the fluid is a liquid or liquid with a dissolved gas with or without the presence of solids, it may be necessary with current technology to transport a sample to a location for testing. With centralized testing, the bulky, stationary, elaborate and sophisticated equipment performs the analysis on a practically endless number of samples. An example of this is the qualitative and/or quantitative measurement of constituents or analytes of blood. For instance, the measurement of blood gases, usually a measure of the partial pressures of oxygen and carbon dioxide, along with the pH from a sample of arterial blood gives the state of the acid base balance or the effectiveness of both the respiratory and cardiovascular systems of the human or vertebrate body. For measuring constituents of blood, the blood sample is drawn from the patient and usually, as in the case of blood gases, transported to a central location for testing.

This technique of transporting the sample to stationary measuring equipment can lead to problems. Ingenious technology has broached solutions to maintain the original composition of the fluid during transportation. Elaborate designs for syringes used in taking the blood samples overcame some problems that resulted in inaccurate readings of the particular chemical constituent being measured. For instance, for determining blood gas composition, the problem of air contamination in the collected sample was solved by the use of liquid heparin as an anticoagulant. Unfortunately, this introduced a sample dilution problem. Subsequent development resulted in the use of heparin in the dry state as opposed to the liquid state to avoid this dilution. Also, elaborate designs are provided for proper mixing of the sample after transportation but before testing. Even with these improvements, there are many reports in the literature that suggest that the values obtained in the measurement of blood gases depend on the type of measuring equipment and the technique for sample collection.

The art also has attempted to develop more portable measuring equipment rather than the fairly expensive nonportable equipment that engender the elaborate and cumbersome transportation techniques. Devices that are very portable could shorten or overcome transporting the sample altogether so that a patient's blood gases could be measured at the bedside in a manner similar to measuring a patient's temperature. U.S. Patents 3,000,805 and 3,497,442 show two such devices. The former has electrodes located on a syringe plunger and the latter has electrodes placed on the syringe well to conduct the measurements. The electrodes are the sensing devices for the blood gases. In U.S. Patent 5,046,496 (Betts et al.), Applicants' assignee describes and claims a portable blood gas sensor which includes electrodes fabricated from a conventional silk screening process where the electrodes are screened on to a ceramic substance. Typically, these electrodes are used along with an electrolyte and analyte permeable membrane that covers the sensor. Some of these membranes may be hydratable membranes that can be stored in a dry state and hydrated Just prior to use.

The utilization of portable equipment to obtain accurate analysis reports while using a disposable device could be advanced with improvements in electronic circuit board design. Accurate sensing of the ambient temperature of the wiring board can precisely control the heater to ultimately maintain, within a narrow distribution of temperatures, the desired operating surface temperature on the wiring board in the region of the one or several sensors. Also the accurate sensing of temperature is important in the area of measuring two phase calibrant liquids so that the calibrant values can be corrected for the most recent storage temperature.

Placement of the all of the components, including the heater, on the wiring board can result in the maximum utility and capability of these components and minimize power consumption.

Also, as with any device for detecting and measuring the analytes in fluid, the device must be calibrated in some manner to obtain accurate values for the amounts of the analytes in the fluid. In the large stationary equipment, calibration occurs through the use of reference fluids that are analyzed before and sometimes during the analysis process of the analyte. Such reference fluids can also be used with portable analyte measuring devices but portable devices should be made as user friendly as possible so their use actually can be more portable.

In EP-A-399 227 a sensor assembly for measuring analytes in fluids in disclosed whereby the sensor assembly comprising a housing in the form of a flow-through cell containing one or more microsensors containing hydratable membranes kept in a dry state and which have to be conditioned prior to use.

It is the object of the present invention to provide a sensor assembly apparatus that utilizes at least one hydratable membrane that can be useful in portable measuring devices or can be placed in catheter lines or actually utilized with stationary equipment where the apparatus allows for the preconditioned state of the membrane.

This object has been attained by the apparatus of claim 1.

Preferred embodiments are described in the dependent claims.

This gives the advantages of: ready-to-use sensors, establishment of a stable electronic operation with stable potential for potentiometric type sensors and maintenance of electrolytic contact between electrodes in amperometric sensors, electrolyte present for reference electrodes, and/or reduced voltage drift like that experienced during a hydration step for dry sensors with hydratable membranes.

An additional advantage of the apparatus of the present invention is that it can be used in a fluid collection and sensor assembly apparatus that has a calibration device resulting in ready-to-use sensors and assistance in the generation of results in a more timely, accurate and inexpensive manner.

Still another advantage of the present invention is that a sensor is provided in a ready-to-use state for example for stationary analysis equipment and especially in a ready-to-use state for a portable analysis device to make such a device more user friendly and and actually more portable.

Also, it is another advantage of the apparatus of the present invention that it can be used in a portable, fluid analyte measuring device for multiple sequential samples where the device has a ready-for-use, multiple use, disposable cartridge to facilitate use of the device at or near the site of sample generation.

The foregoing objects and others gleaned from the following disclosure are accomplished by the sensor assembly of the present invention.

The preconditioned electrochemical sensor assembly of the present invention has a sensor element that is a nonconducting substrate having at least one hydrophilic-membrane-containing analyte sensor and electrical circuitry, a housing to enclose the sensor element where the housing allows for at least one channel to pass over at least one sensor, a hydrating fluid occupying the portion or portions of the channel or channels over the sensor or sensors, and seals that are substantially impervious to at least moisture placed in or on the channel to maintain the hydrating fluid in contact with the hydrophilic membrane. The sensor and the electrical circuitry means are in electrical contact with the sensor at least to convey the electrical impulses from the sensor to an instrument to read the electrical signals. The housing encloses the sensor element, and the electrical circuitry of the sensor element is electrically isolated from the hydrating fluid in the channel or channels to avoid leakage current or short circuiting of the electrical circuitry. The channel is constructed to provide fluid flow to, over, and from the one or more sensors and to allow for ingress and egress from the housing.

In a narrower aspect of the present invention, the preconditioned disposable electrochemical sensor assembly for measuring analytes in fluids has: a) a housing, b) sensor element that has a nonconducting substrate with more than one hydrophilic-membrane-containing analyte sensors and with electrical circuitry in electrical contact with the sensors at least to convey the electrical impulses from the sensor, c) a hydrating fluid positioned in fluid contact with the hydrophilic polymeric membrane of the sensor, d) seals that are substantially impervious to at least moisture to maintain the hydrating fluid in contact with the hydrophilic polymeric membrane of the sensor, e) electrical isolating means to maintain electrical separation between the hydrating fluid and electrical circuitry of the sensor element.

In this aspect of the invention, the housing has a first and second opposing section where each section has an exterior and interior surface. The sections when matched together form an interior space and at least one channel. The former allows for placement of the sensor element within the housing while the latter allows for fluid contact between the hydrating fluid and the hydrophilic polymeric membrane or membranes of the sensors. The channel has two opposing openings to allow fluid flow through the channel from a receiving opening before to an exit opening after the sensors. The receiving opening is suitable for attachment to a sample receiving means and the exit opening is suitable for attachment to a collection means such as a syringe or reservoir in general. The interior space of the housing communicates with the channel to contain the sensor element so that the sensor or sensors that are on the substrate are so disposed to lie in the path of the channel for fluid contact with the hydrating fluid. The first and second sections can be adhesively connected to improve their attachment to each other. The housing also allows for communication from the electrical circuitry means to the reading instrument. Such an instrument could be one that takes the signals from the sensor through the electrical circuitry means and as a self-contained, hand-held, preferably battery-powered monitoring instrument or analyzer, hereinafter "Analyzer") processes the signals and displays the information in a digital or paper mode to the operator.

The other components of the invention are arranged in or on the housing in a manner to allow the sensor's use in detecting the component of interest in the fluid and to maintain the hydrophilic polymeric membranes of the electrochemical sensor in a hydrated state prior to use, and to isolate the hydrating fluid and the electrical circuitry means. The sensor element is configured, arranged, and placed in the interior space of the housing to assist in maintaining electrical isolation between the electrical circuit means and the hydrating fluid. The hydrating fluid is chiefly an aqueous fluid with an effective composition to hydrate at least to a partial degree but better to a substantial degree the hydrophilic polymeric membranes. The sealing means covers the receiving opening of the channel and the exit opening of the channel and can be two separate seals in adhering association to the housing so as to cover these openings. The seal can have one or more surfaces where at least one surface is substantially a non-oxidizing metal such as aluminum that is useful with an adhesive-type polymer. The adhesive-type polymer can be used either as an application to the surface to be sealed or as another surface of the seal. The seal is fixedly attached to the housing by a chemical means and/or by a mechanical means. The electrical isolating means occupies an effective portion of the interior space of the housing not occupied by the sensor element or the channel and not interfering with the contact between the sensor element and the channel in order to obtain electrical isolation of the electrical circuitry means from the hydrating fluid in the channel.

The electrical circuitry means for use with the sensor element with the aforediscussed sensors and with the housing or other sensors and housings known to those skilled in the art can be an improved wiring board that includes the nonconducting substrate. In addition to the at least one analyte sensor and the substrate the electronic wiring board preferrably has: 1) a thermistor in close relation to the at least one analyte sensor supported on or to the substrate, and 2) a heater, also supported on the substrate. The heater provides heat in response to the temperature sensed by the thermistor to at least the region where the thermistor and the analyte sensor are positioned on the board. This arrangement controls the temperature of the region of the board within a narrow distribution of temperatures and thereby increases the sensor's accuracy, and connecting means supported on the board for connecting the board to an external electrical source.

In a narrower aspect, the improved electronic wiring board is manufactured using thick film or thin film layered circuit technique or a combination of these, and the thermistor and the one or more analyte sensors are supported in the same plane on the substrate wherein the analyte sensors are blood gas sensors of one or more of the following types: an oxygen sensor, a carbon dioxide sensor, and a pH sensor. Also, the connecting means includes plurality of external leads, and a resistor is supported on the substrate on the same side as the heater and commonly connected to one of the external leads with the thermistor, dividing the voltage therebetween. Although it is possible to have the resistor and the heater each electrically connected to external leads, the temperature coefficient of the thermistor can be positive or negative and the temperature coefficient of the resistor is substantially zero. Also, the thermistor and resistor values are allowed to vary over several orders of magnitude as long as the two can be made equal at the calibration temperature. Additionally, the connecting means further includes a plurality electronic conducting pathways individually and electrically connecting each of the sensors and the thermistor with external leads provided on the substrate at the end of the pathways.

Also, the heater can be powered by a controlled DC voltage whereby the heater is regulated by a combination of proportional, integral and/or derivative controls thereby reducing the amount of overshooting or undershooting by the heater of a predetermined temperature. The external leads are positioned on the same side of the substrate as the resistor and the heater. The electronic conducting pathways of improved electronic wiring substrate can individually and electrically connect each of the sensors and the thermistor on one side of the board with external leads provided on the other side of the board through a plurality of holes in the board. Additionally, the temperature sensor including the thermistor and the resistor can be calibrated by laser trimming of the resistor to produce a ratiometric output proportional or inversely proportional to temperature.

Also the improved electronic wiring board wherein the oxygen sensor is an electrochemical cell can have an anode and a cathode, each connected to an external lead. Also the oxygen sensor can include an oxygen permeable membrane covering, in a fluid tight manner, and an opening in the board can contain an electrolyte, and the anode can be grounded on the board to thereby assure that the potential of the electrolyte is the same as the anode potential.

Additionally, the improved electronic wiring board can have at least one reference electrode, to provide an accurate reference potential, supported on the board and it can be electrically connected to a electronic conducting pathway. Although it is possible to have one reference electrode present on the substrate and it is supported on the substrate and it is electrically connected to a electronic conducting pathway extending from the anode. The nonconducting substrate is a flat substantially thin ceramic substrate layer that has a patterned metallic layer provided on the ceramic substrate layer. The metallic layer can be formed on the substrate by depositing a metallic printing paste on the substrate to form electronic conducting pathways and the electrodes of the sensors and the electrode of a reference electrode. The metallic layer can be encapsulated with at least one layer of a chemically stable and moisture resistant encapsulant that provides electrical isolation of the electronic conducting pathways from the electrolyte and sample like blood. The wiring substrate as described can operate even after several months of storage. The thermistor provided on the ceramic substrate layer can be encapsulated with at least one substantially thin layer of a chemically stable and moisture resistant encapsulant.

The aforedescribed electrochemical sensor assembly can be used with a number of different types of sampling means including a single use or multiple use sampling or testing means. These sampling means can also be used with other types of electrochemical sensors or sensor means that have as a minimum the at least one sensor in a housing allowing for fluid contact with calibrant and fluid to be tested (sample) in a housing with an electrical circuitry means even as rudimentary as an electrical connection to an analyzing means having a microprocessor or microcomputer.

A single use, disposable sampling means useful with the electrochemical sensor assembly is a collector that is designed for association with at least one analyte sensor as the sensing means that is also particularly adapted for use with and/or connection to the Analyzer which can be battery powered. Such a fluid collection, sensing and calibrating apparatus has the calibrator, the sensing means, and the collector with suitable puncturing means for any seals of the sensing means where these components are arranged in fluid communication, where the collector provides for the flow of the calibration fluid from the calibrator to contact the sensing means that is associated with the collector.

The calibrator has at least one portion or section that is a base and holder adapted to hold (recess) at least one container of calibration fluid, (preferably the container is held in a vertical fashion).

The fluid collector is adapted for fluid communication with the container in the calibrator (needle) where the collector has a body portion defining a chamber at one end and has an actuating means slidable within the body portion at the other end to provide a variable volume for the chamber. At the end of the chamber that is opposite from the actuating means, the collector is adapted with connecting means for fluid engagement with means for withdrawing calibration and/or sample fluid.

The at least one sensing means is associated with the collector for fluid communication (needle) with the container and in fluid communication with the collection means so that when calibration fluid is withdrawn from the container by the actuating means of the collector and into the chamber where the fluid contacts the sensor. This fluid contacting association with the collector is through one or more fluid chambers of the collector itself or with one or more fluid chambers of a distinct sensor assembly, which can be the aforedescribed electrochemical sensor assembly, in fluid communication with the fluid chamber of the collector.

The means for withdrawing calibration fluid is attached to the collector either through the connecting means or through a housing for the sensing means that is affixed for fluid engagement to the collector by the connecting means. The calibration fluid has known levels of one or more analytes for calibrating the one or more sensors. The calibrator may be prepackaged with the collector and sensor for ready-use and may be readily disposable.

The calibrator with the container and the at least one sensing means as a distinct assembly and the collector are attachable for fluid communication in the order recited and along a longitudinal axis for fluid communication from the container to the chamber of the collector. When so engaged, the alignment can be vertical starting at the calibrator to maintain the container in a vertical position during fluid communication to contact the sensing means where the support for the alignment is provided by the holder of the calibrator. This support can be provided by an extended holder that engages at least a portion of the sensing means and/or collector so they are slidable within the holder or by an expanded base of the holder to securely retain the container and the sensing means and collector vertically attached to the container.

Also the chamber of the collector and the chamber of the sensing means assembly may be separated by a first fluid seal whereby the seal is the front or distal sealing means of the sensor housing. The first chamber of the collector defines a cylinder and has a piston slidable therein and the actuating means is the means for actuating said piston and also carries a first seal puncturing means. The housing of the chamber of the assembly for the sensing means of the present invention terminates at two ends with connecting means for fluid engagement one for means for withdrawing a sample of fluid and the other with the connecting means of the collector. The connecting means attachment to the chamber of the sensing means defines a connecting conduit for fluid communication. The connecting means or the chamber for connection with the withdrawing means is sealed by a second fluid seal. The calibrator having a body portion defining a cylinder open at one end for slidably receiving at least a portion of said body portion of said collector with the sensing means or sensor assembly also has a movable member that carries a second seal puncturing means. This movable member can be slidable in said cylinder of said calibrator to engage the sliding collector and to direct the collector toward the calibration solution in the container at the other end of the calibrator. The puncturing means can be used to puncture the second seal of the sensing means like the sealing means of the sensor assembly and any seal on the container in the calibrator to allow fluid flow from the container to come in contact with the sensing means and enter into the collector. The sealed container containing calibration solution is located and supported in the end of said cylinder of said calibrator opposite from the opening for the collector with the sensing means.

A portable, ready-for-use analyte measuring device for multiple sequential fluid samples may have a disposable cartridge, a fluid calibration means external to the cartridge and an analyzer. The cartridge has a housing, inlet for introduction of fluids into the housing, flow cell with sensing means, which is the aforedescribed electrochemical sensor assembly, in fluid connection with the inlet, at least one unidirectional valve in fluid connection with the flow cell, and a waste collection area or reservoir in fluid connection to the valve.

The housing of the cartridge has at least one opening to the exterior through a unidirectional fluid inlet. The interior of the housing has the aforedescribed electrochemical sensor assembly (hereinafter referred to as "Assembly") connected to the inlet for fluid flow into the Assembly. The connection is at an opening in the Assembly that forms a channel that runs through the Assembly to a second opening.

Generally, for the multi-use application, the electrochemical sensor assembly, the sensor containment area holds the at least one sensor that is comprised of at least one measuring electrode in sensing relationship to the channel so that the sensor sensingly contacts the fluids that flow through the channel. Sensors with membranes are hydrated for this sensing contact by the presence of a fluid that is at least a hydrating fluid in the Assembly to maintain the one or more sensors in a hydrated state for ready-to-use application of the cartridge. In ionic contact with the sensor is a reference electrode in the reference electrode containment area of the Assembly. The reference electrode is in conductive contact with a contained quantity of reference electrolyte for the number of multiple sequential tests for which the cartridge is capable.

The electric circuitry is attached for electrical conduction to the at least one sensor and at least one reference electrode. As in the electrochemical sensor assembly, the circuit is located remote from and electrically isolated from the fluid in the channel of the Assembly. The circuit has the capability of conveying signals from the at least one sensor, and for providing any required electrical power to the electrodes in the Assembly. The electrical circuitry is attached for electrical conduction to a signal conveyor which links the Assembly for electronic signaling to the exterior of the cartridge for electrical interface with the Analyzer.

At a second opening of the channel of the Assembly, there is connected for fluid flow at least one unidirectional valve to receive fluids from the Assembly. The one-way valve is arranged to maintain fluid in the Assembly to keep the sensor hydrated and to retard bi-directional flow of the fluids leaving the Assembly from the introduction of fluid at the inlet of the housing. To receive and retain fluids passed through the Assembly and the one-way valve, the cartridge has in fluid conveyance engagement with the unidirectional valve a waste reservoir.

The Analyzer of the system is electrically connected to the signal conveyor of the cartridge along with an electronic means for interpreting these signals and a display for displaying the results of calculations to determine the amount of analyte in a fluid. The electronic means interprets the signals sent from the sensor(s) for the calibrant and the multiple, sequentially-introduced samples having one or more analytes. This means also calculates the amount of the one or more analytes from these received signals. Additionally, the Analyzer has an encoded information reader for imputing encoded information for calibration.

In addition, the device for multiple use applications has external to the cartridge at least one container of calibration fluid which has a known amount of one or more analytes that are those analytes to be measured in the multiple samples introduced into the cartridge. Since the calibrating fluid is initially external to the cartridge, the calibrating fluid additionally is associated with an encoded information carrier having detailed information about the quantities of analytes in the calibrating fluid. Additionally, when the containers of calibration fluid are packaged with the cartridge, the encoded information carrier for the calibration fluid can have information about the one or more sensors in the electrochemical sensor assembly and their associated premeasured sensitivities to analytes to be measured. Otherwise with the cartridge packaged separately from the calibration fluid two encoded information carriers are used. One is for the calibration fluid and the other is for the sensor(s) in the cartridge having the respective aforementioned information for inputting into the analyzer through the information reader.

The method of using the above defined multi-use system involves a calibration fluid with associated encoded information carrier with encoded information about the calibrating fluid. The encoded information is read into the Analyzer's microcomputer or microprocessor through a reader. Also through the same or different encoded information carrier, encoded information about the one or more sensors- in the Assembly is inputted into the Analyzer through the reader. The calibration fluid is injected or introduced into the inlet of the cartridge to calibrate the sensor(s) in the Assembly. The calibration fluid displaces the hydration fluid in the channel of the Assembly and the hydration fluid flows through the unidirectional valve and into the waste reservoir. The at least one sensor electrically responds to the presence of the amount of the one or more analytes in the calibration fluid and signals this response to the Analyzer. The Analyzer uses this information and the information from the encoded information of the at least one sensor. At this point, the sample with an unknown value of a known analyte is introduced into the inlet of the cartridge to flow into the Assembly to contact the one or more measuring sensors. This forces the calibrant out of the Assembly through the unidirectional valve and into the waste reservoir. As with the calibration fluid, the sensor(s) respond and signal(s) are transmitted or conveyed to the Analyzer. The Analyzer interprets the signals and from the calibration information calculates the amount of the known analyte in the sample and displays it on the display means. Additionally, the Analyzer can have a printer to print a hard copy of the analysis.

In this method the performance characteristics of the one or more measuring sensors can be inputted into the Analyzer through the calibration fluid analysis and/or by means of inputting encoded information associated with the cartridge. This need only be done once for all of the multiple samples passed through the Assembly for a cartridge. When the cartridge is exhausted, it is disposed of and another cartridge is connected electrically to the Analyzer and the encoded information for this cartridge is inputted through the reader of the Analyzer for additional testing of samples.

The electrochemical sensor assembly with or without any use, delivery, and/or calibrating apparatus as described above can be in a gas impervious or hermetically-sealed layer. In any of these arrangements of the invention, the hydrating fluid of the Assembly or sensor means is a preconditioning fluid that is held in fluid contact with the one or more sensors of the sensor element by the housing of the sensor Assembly. The preconditioning fluid can be an activating fluid to maintain the sensors in an active state and/or a controlled-content fluid having a known amount of one or more analytes that are measured by the one or more sensors. When both the activating fluid and the controlled-content fluids are present, the activating fluid is in sealed contact with the one or more sensors and the controlled-content fluid is in contact with the Assembly sealed in the sealed layer to equilibrate with the one or more sensors to maintain the sensor in a precalibrated state. The sealed contact is provided by the housing where some portion of the housing is gas permeable over a period of time. This allows an equilibration of gas between the controlled-content fluid and the activating fluid in fluid contact with the one or more sensors.

With the multi-use system used for determining gas values in a liquid, the cartridge can be packaged in the sealed layer, and the atmosphere within the layer shaped like a bag is controlled with a known gas atmosphere as the controlled-content fluid. The containers of calibration fluid may be packaged with the cartridge or in a separate layer as a package which may or may not be gas impervious. Additionally, gas impervious layer as packaging may be used with controlled-content fluid for containers of additional hydrating fluid or flush fluid for the sensors. The encoded information carrier for the cartridge and the calibrating fluid can be located on the exterior of the sealed layer.

### Brief Description of the Drawings

Figure 1 is a side elevational view of the general arrangement of the sensor assembly.

Figure 2 is a bottom view of the housing of the sensor assembly taken along line 2-2 of Figure 1, where the channel contains fluid.

Figure 3 is a sectional elevational view of the sensor assembly along lines 3-3 of Figure 1.

Figure 4 is a side elevational of the first or top section of an alternative embodiment to that shown in Figure 1 taken along a line similar to that of lines 4-4 of Figure 2 without the back portion of the sensor assembly and Without the sensor board occupying the internal space.

Figure 5 is a bottom view of the housing taken along lines 2-2 of Figure 1 without the back of the sensor assembly similar to that shown in Figure 4.

Figure 6 is a sectional view of the distal end of the sensor assembly taken along lines 6-6 of Figure 5.

Figure 7 is a forward end view of the housing.

Figure 8 is a sectional view through the sensor assembly taken along line 8-8 of Figure 5.

Figure 9 is a front sectional elevational view of the back portion of the sensor assembly.

Figure 10 is a bottom view of the back portion taken along lines 10-10 of Figure 9.

Figure 11 is a forward end view of the back portion taken along lines 11-11 of Figure 9.

Figure 12 is a front elevational view of the contour of the channel formed along the longitudinal axis on the interior of the housing.

Figure 13 is a top view of the contour of the channel taken on line 13-13 of Figure 12.

Figures 14-17 is a cross sectional view of the channel at various portions along its length.

Figure 18 is a top plan view of the housing of the sensor assembly taken along line 2-2 of Figure 1, where the channel does not contain any fluid.

Figure 19 is a top planar view of one side of a wiring substrate, having electrodes and a thermistor.

Figure 20 is a planar view of the other side of the wiring substrate of Figure 19 having a resistor and a heater that traverses the board and a number of leads through the substrate from the side depicted in Figure 19 to provide an external electrical connection from the substrate.

Figure 21 is a planar view of the one side of a wiring substrate having an arrangement of three analyte sensors with two reference electrodes and a thermistor with accompanying patterned and layered circuitry.

Figure 22 is a planar view of the one side of a wiring substrate having one sensor and a thermistor axially aligned and one reference electrode spaced apart from that axis and having accompanying patterned and layered circuitry.

Figure 23 is a block diagram of the monitoring means and its connection with the electronic wiring substrate.

Figure 24 is a cross sectional view of a calibrator that can be used with various collector designs as shown in Figures 25-27.

Figures 25-27 show the calibrator in use with several different designs for the collector where the collectors are in various states of insertion into the calibrator.

Figure 28 is a cross sectional view of a calibrator that contains the collector with the sensing means and the puncturing means, which are not shown in cross sectional view, in an original non-vertical arrangement in separate recesses or wells in the calibrator.

Figure 29 is a cross sectional view of a syringe and calibrator wherein all of the several seals are intact and unpunctured.

Figure 30 is a cross sectional view of the syringe and calibrator of Figure 29 wherein the syringe has been moved with respect to the calibrator; all of the several seals are punctured and the calibration solution may be withdrawn from its container.

Figure 31 is a cross sectional view of the calibrator of Figure 29 along line 3-3 at the end retaining the container of calibration fluid.

Figure 32 is a cross sectional view of the calibrator and collector with the sensing means of Figure 29 along line 4-4 where they are held together in a longitudinal alignment by a fastening means.

Figure 33 is an elevational view of the top of the cartridge attached to the analyzing means.

Figure 34 is an orthogonal view of the top, front and side of the cartridge detached from the analyzer.

Figure 35 is a side view of a package with a cut-away showing a side view along lines 3-3 of Figure 33 of the cartridge inside the package.

Figure 36 is a sectional view of the side of the cartridge taken along the lines 3-3 as is Figure 33.

Figure 37 is a sectional view of the cartridge from the opposite side from that of Figure 36.

Figure 38 is plan view of the back of the analyzer along lines 7-7 of Figure 33.

Figure 39 is a plan view of the bottom of the analyzer.

Figure 40 is a electric block diagram of the analyzer.

Figure 41 and 42 are front views of two different embodiments of the wrapped sensor element of the present invention with a cut-away sectional view to display the interior of the envelope and the contents of the interior including the sensor.

Figure 43 is a front view of the wrapped sensor element of the present invention with a cut-away sectional view to display the interior of the envelop and the contents thereof including the sensor in a preferred arrangement for the single use application with the calibrator and the collector.

Figures 44 and 45 are elevational views of the packages of the calibration means, where for Figure 44 the package has vials of fluids, and where for Figure 45 the package has fluids in delivery assemblies.

Figure 46 is a graph of nanoamps along the ordinate and seconds along the abscissa showing the performance of the activated sensor device of the present invention.

### Detailed Description and Preferred Embodiment of the Invention

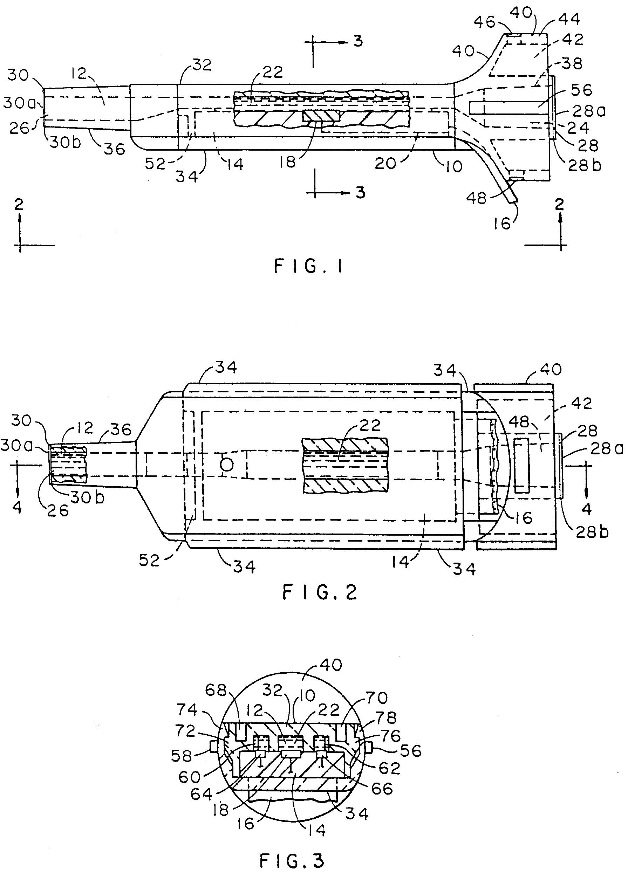

In the side elevational view of Figure 1, the general arrangement of the sensor assembly is shown. Housing 10 is made of any fairly rigid moldable material such as rigid thermoplastic polymers although thermosetting polymers can also be used. A suitable example is a methyl methacrylate styrene butadiene terpolymer and rigid plastics such as polyesters like polyethyleneterephthlate or polycarbonate or blends or alloys thereof and other similar materials known to those skilled in the art. The housing 10 can be any basic geometric shape suitable for containing a channel 12 and sensor element 14. The number of parts comprising the housing can range form 1 to a plurality, but two parts are preferred. A single part housing is at least that which sufficiently provides the channel for fluid communication with the one or more sensors 18 on sensor element 14. In this arrangement the sensor element 14 can actually form one side of the housing. The housing also supplies an opening for an electrical attachment means 16 for electric attachment to the electrical circuit means of sensor element 14. The sensor element 14 has at least one sensor 18 with a hydrophilic membrane where part of the sensor is electrically connected to an electric circuit means 20 and both the sensor 18 and electric circuit means 20 are on a nonconducting substrate of sensor element 14. The sensor 18 is located on element 14 and channel 12 and element 14 are arranged in housing 10 in a manner so that sensor 18 and channel 12 can be in fluid contact with each other when channel 12 is filled with a hydrating fluids 22.

Housing 10 has at least one and preferably two openings, 24 and 26, arranged along channel 12 at different locations from each other in relation to sensor element 14. This arrangement allows hydrating fluid 22 that is subjected to fluid pressure from either opening to flow across sensor element 14 and contact the one or more sensors 18. Channel 12 can have any shape that allows for laminar flow of fluid through it in the vicinity of the one or more sensors 18 along channel 12. Also, the openings 24 and 26 are sealed by a substantially moisture impervious seal 28 and 30, respectively. The opening 26 can serve as an inlet to or outlet from housing 10 that is preferably formed by conical tip 36. Also, the housing at the other end of the sensor element 14 from opening 26 can have a flared end 40 encompassing opening 24 that is formed by tip section 38, which preferably has a cylindrical exterior and a conical interior. The tip 38 is surrounded by flared end 40 which has an inner annular space 42 between the external rim 44 of the flared end 40 and the external surface of tip section 38. The openings 24 and 26 for housing 10 shown in Figures 1 and 2 are preferably aligned in the same plane and along the same axis at opposite ends of the channel 12 so channel 12 passes longitudinally through the housing along the same central axis. This arrangement provides sufficient support of the channel by the housing to receive and/or expel fluid through the channel with pressurized movement. Preferably, the sections 36 and 38 of housing 10 are at opposite ends of the housing 10 and contain portions of the channel 12 along with openings 24 and 26. The flared end 40 can also have one or more external ribs like 50 for ease of manipulation or handling of the housing 10. The attachment of sections 32 and 34 can be assisted by a guiding member and guiding slot shown together in Figure 1 as 52

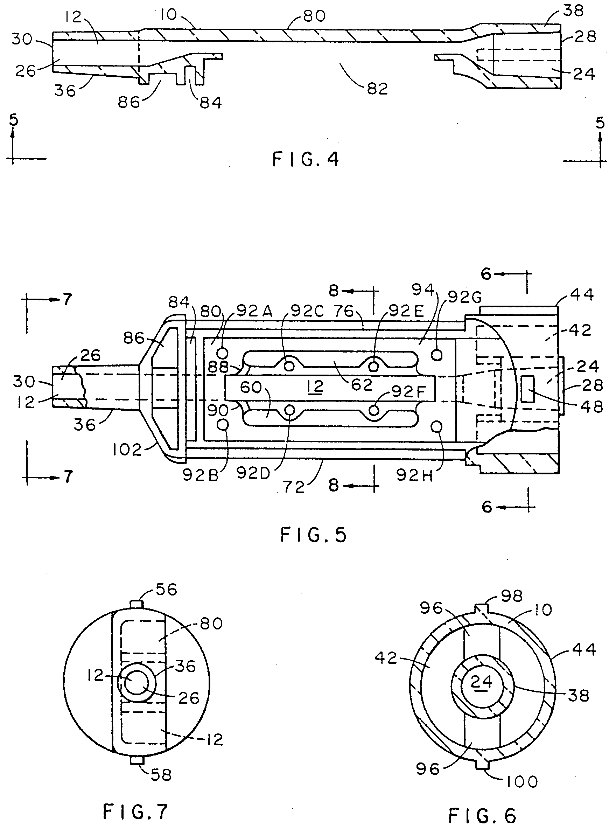

Tip sections 36 and 38 allow for connection or coupling to a device to provide fluid pressure, rapid fluid flow, or suction to cause the fluid with an analyte, for example, to be measured to pass, preferably in non-capillary action or flow, in measuring contact with the one or more sensors 18. The tip section 38 distally located from tip section 36 can be similar to tip section 36 as shown in Figure 4 or can be adapted and preferably is adapted to connect with a distal or needle-end end of a syringe. The distal end of the syringe fixedly engages the housing 10 through the annular space 42 and fixedly engages with the housing through attaching means 46 and 48. Preferably, the shape of tip 36 is of a standard outer diameter to allow for connection to sample gathering means or fluid withdrawing means such as needles or tubing or conduit from catheters or tubing in multi-sequential analyzing equipment. Most preferably, the shape is suitable for Leur attachment either slip or lok (lock) to a sample gathering means not shown in Figure 1 such as a needle for a syringe.

Housing 10 preferably has one section 32 and another section 34 which have matched attachment means (not shown in Figure 1 but shown in subsequent Figures) for connection to each other. Sections 32 and 34 fixedly engage to form the housing 10 having one or more internal spaces (a portion of which is shown in Figures 4, 5 and 7 as 82) for placement of sensor element 14. The internal space 82 need not be of any particular geometric configuration just so long as sensor element 14 fits into the space. The internal space 82 and sensor element 14 are preferably of matched configuration and are preferably generally rectangular. Preferably, one section 32 comprises a substantial portion of housing 10 as shown in Figure 4 and the other section 34 is a cover for the back of sensor element 14 occupying internal space 82 of Figure 4. With this arrangement and with the internal space having dimensions that closely match those of the sensor element 14 for a snug fit of the latter into the former, the sections 32 and 34 can assist in providing electrical isolation between the hydrating fluid 22 and the electric circuit means 20. The former is at least in channel 12 and the latter is on sensor element 14. Preferably, section 32 has the tip sections 36 and 38 and all of the flared end 40 and forms a portion of channel 12 and any other channels that are present. The remaining portions of the channel 12 or other channels are formed by sensor element 14 occupying the internal space so that the surface with one or more sensors as 18 actually forms a wall of the channel 12 as shown in Figure 1. Any arrangement or configuration other than that shown in Figure 1 can be used that allow the two sections 32 and 34 to engage and form housing 10 with one or more internal spaces for placement of sensor element 14 so that the sensor 18 is in fluid contact with hydrating fluid 22 that is in channel 12.

Similar numerals are used throughout the drawings to denote the same feature in each of the drawings. The bottom view of sensor housing 10 shown in Figure 2 along lines 2-2 of Figure 1 highlights the preferred matched configuration of sections 32 and 34 in a top and bottom relationship. Section 34 preferably matchedly engages section 32 through guide member and slot 52 of sections 34 and 32, respectively, shown better in subsequent drawings from the inner surface of bottom 34. In Figure 2, the slot of 52 is shown in phantom as is channel 12 except for the cutaway portion showing hydrating fluid 22. Also shown in phantom is electrical cable means 16 as it enters housing 10. Slot 48 clearly shown in Figure 2 provides for fixed attachment with a syringe not shown in Figure 2 at the distal or outlet end of housing 10. Preferably, as shown in Figure 2 an electrical insulating means 56 is present. The electrical insulating means 56 can be any material that can occupy spaces between housing sections 32 and 34 and sensor element 14 other than the one or more channels and electrical attachment means 16 to assist in providing for electrical isolation. The insulation can restrict any contact between any hydrating fluid 22 and the electric circuit means 20 to reduce the possibility of any short circuits or leakage current. This material should have the following characteristics: an insulation factor of around 10¹⁴ ohms /cm² and substantially impervious to moisture and preferably curable at a temperature of less than around 60 degrees Centigrade. Nonexclusive examples of a suitable material include: epoxy polymer, modified epoxy molding compound such as brominated epoxies, epoxy molding compounds, polyimides, unmodified polyimides like PMDA-ODA and BTDA ODA-based polyimides, Poly(amide-imide) polymers, modified polyimides having modification from diamic acid additives, siloxane polyimides, and high temperature polymers like silicone polymers, and polyarylene ether polymers. A particularly suitable material is a bisphenol A epichlorohydrin type epoxy polymer like that available from the Hysol Division of The Dexter Corporation in Industry, California 91749 under the trade designation EE4207.

Seals 28 and 30 in Figures 1, 2, 4 and 5 can be and preferably are substantially impervious to air, and they may be comprised of a single layer or multilayer laminate. A suitable single layer material includes metal foil that is capable of sealing by a polymeric material that can be heat-treated or RF (radio frequency) treated for sealing. The multilayer laminate material ordinarily has an interior layer of polymeric material and outside this layer a metal foil layer. A typical laminate can have two or more layers and may have an additional outer polymeric layer to facilitate abrasion resistance or printing on top of the metal foil layer. A nonexclusive example of the metal foil is aluminum. A three layer laminate suitable for the seal of the present invention can have from the exterior surface to the interior layer the following: 1) nylon, polyester, polyethylene or polypropylene, 2) aluminum foil, and 3) an inner heat sealable polymeric layer such as polyethylene, polypropylene, polyvinylidene chloride or nylon. A nylon-foil-polypropylene laminate of, i.e., 17 grams per square meter nylon, 32 grams per meter squared aluminum, 45 grams per meter squared polypropylene or of a suitable example is a polyfoil-polylaminate which is a three-layer composite having an aluminum foil intermediate layer and an inner and outer layer of polypropylene. The seals are puncturable and preferably can and form a seal that can withstand gamma radiation sterilization. The seals preferably have at least two sections -- an one section 28a and 30a away from the mouth or opening of the channel and another section 28b and 30b in contact with the housing 10 to seal the openings 24 and 26, respectively of the channel 12. The "a" seal sections can be at least an air impervious metal foil, preferably aluminum, and the "b" seal sections can be an adhesive material. Preferably, the seals 28 and 30 are a paper-backed aluminum foil coated with a clear heat sealable coating. The coating can be a blend of a high molecular weight ethylene and vinyl acetate copolymer. A nonexclusive example of a suitable material is an aluminum foil having a heat seal polyester film available under the trade designation "Foilseal 3-6" available from Selig Sealing Products, Inc. of 17w745 Butterfield Road, Oakbrook Terrace, Illinois 60181. Such materials can have a gas transmission for oxygen that is nil and a water vapor transmission which ranges from around 0.005 to 0.059 GS (grams)/CSI(100in²)/24 hours at 90 percent relative humidity. Such materials provide a seal that when securely attached across the openings 24 and 26 of the housing 10 provide substantial imperviousness to air. These values are obtained on a Permatran-W6 for water transmission and an Ox-tran 1000 for oxygen transmission, and both pieces of equipment are available from Mocon, Modern Controls, Inc., 4220 Shingle Creek Parkway, Minneapolis, Minnesota 55430. The thickness of the seals 28 and 30 can range from an overall thickness of around 25.4 µm (1 mil) to around 254 µm (10 mils) with the heat seal coating ranging in thickness from around 12.7 µm (0.5 mil) to around 101.6 µm (4 mils) and more preferably from around 12.7 µm (0.5 to) around 50.8 µm (2 mils) and the aluminum foil ranging in thickness from around 2.5 µm (0.1) to around 203.2 µm (8) and more preferably from around 7.6 µm (0.3) to around 50.8 µm (2 mils).

Alternatively, seals 28 and 30 can have an adhesive material as the "b" section which is a thermoplastic resin suitable for hot melt deposition or extrusion lamination. Suitable examples of these thermoplastic resins include resins known as the so-called hot-melt type adhesive, such as polyethylene, an ethylene/vinyl acetate copolymer (EVA) or a partially saponified EVA. For instance, a graft copolymer can be used that is a 20 to 60 percent saponification product of an ethylene/vinyl acetate copolymer (EVA) having a vinyl acetate content of 15 to 45 percent by weight as a trunk polymer and a polymer of an unsaturated carboxylated acid in a quantity of 0.1 to 10 percent by weight of the partially saponified EVA as a branch polymer. Also, the seals 28 and 30 can be a composite of an aluminum/polypropylene film with a heat sealable resin such as a polyamide, polyolefin, and saturated polyesters. When sealing to adhere the resin to the plastic surface and thereby adhere the seal to channel 12 is performed by heat sealing, any induction sealing or any heat sealing method known to those skilled in the art can be used. The method of sealing depends to a degree on any securing means used to maintain the seals 28 and 30 in a snug relationship to the tips 36 and 38, respectively. The seals 28 and 30 can have any shape suitable for covering completely openings 24 and 26 and providing for a snug fitting with the flat surface of the rims of the tips 36 and 38. Preferably, the seal is in the form of a disc having a diameter similar to the diameter across the opening and tip for attachment to the tip rim to cover the opening 24 and 26.

The sectional elevational view of the sensor apparatus shown in Figure 3 is along lines 3-3 of Figure 1. As shown in this figure, on housing 10 ridges 56 and 58 can be present for ease of handling. Flared end 40 is shown from this view from the front of the apparatus. The electrical attachment means 16 is shown as a cable extending from the bottom of housing 10. This view shows the preferred embodiment of the invention having a plurality of channels. In addition to channel 12, the housing 10 and sensor element 14 forms two additional channels 60 and 62 which also can and preferably do contain hydrating fluid 22. As shown in Figure 3, channel 12 is in fluid contact with sensor 18 which is on sensor element 14. Also shown in fluid contact with channels 60 and 62, respectively, are electrodes or sensors 64 and 66. Also as shown in Figure 3, there may be and preferably are present two longitudinal slots 68 and 70 which are in the top section 32 of housing 10. These slots are for mold enhancements for plastic molding of the housing 10 to assist in obtaining flat external and internal surfaces for a larger housing section 32. The ridges 56 and 58 are on housing section 34 which securely fastens to housing section 32 by matching fastening means 72 on top section 32 and 74 on bottom section 34. Mirror image fastening means are present on the opposite side of housing 10. These fastening means can be on the sides of housing 10; one on each interior side of bottom section 34 and two on the top section 32 of housing 10 where one is on each side of housing 10.

In Figures 1-3 and the remaining figures, the sensor element 14 can have one or more sensors like sensor 18 having one or more hydratable membranes known to those skilled in the art. Preferably, sensor element 14 is a nonconducting substrate with an electrical circuit means 20 electrically connected to at least one sensor 18 through at least one electrode. Generally, the nonconducting substrate can be a glass or ceramic including sheet or chip or nonconducting substrate like nonconducting polymers or commercially available frit that can be used as the substantially smooth flat surface for the nonconducting substrate. Nonexclusive examples include borosilicate glass as is known to those skilled in the art for producing thick film or layered circuits. A nonexclusive but preferred example of which includes a ceramic base having around 96% A1203 such as that available commercially from Coors Ceramic Company, Grand Junction, Colorado. Generally, the electrical circuit means 20 is any electrical circuit means known by those skilled in the art. Both the sensor 18 and the electrical circuit means 20 can be prepared from any number of well known layered circuit or integrated circuit technologies, as for example, thick film, thin film, plating, pressurized laminating and photolithographic etching, and the like, however, the thick film technique is preferred. A suitable sensor element is that described in U.S. Patent 5046436 and titled, "Sensor Assembly for Measuring Analytes in Fluids, which is commonly assigned. The at least one sensor 18 can be a potentiometric or amperometric sensor, in that the former has one electrode and the latter has two, both an anode and a cathode. In the situation where the sensor 18 is potentiometric, an additional electrode is usually present as a reference electrode. Any reference electrode known to those skilled in the art can be used. The potentiometric or amperometric sensor preferably has a hydrophilic polymeric membrane and the sensor preferably has an aqueous-based electrolyte with suitable ionized chemical species like those in silver/silver chloride, calomel and mercury sensors or electrodes. Suitable examples of such membranes that may be present in electrochemical sensors for use in determination of blood gases are described in U.S. Patents 3,088,905; 3,912,614; 4,133,735; and 4,454,007 and European patent specifications 0015075 and 0027385 and the article in the journal entitled "Medical and Biological Engineering Computing", 1978, Vol. 16, pages 599-600. The publications describe blood gas detectors requiring the presence of membranes and a number of useful or potentially useful membrane materials. Suitable nonexclusive examples of a hydrophilic polymeric membrane include polyvinylchloride and modified polyvinylchloride and any similar hydrophilic hydratable polymeric membrane known to those skilled in the art.

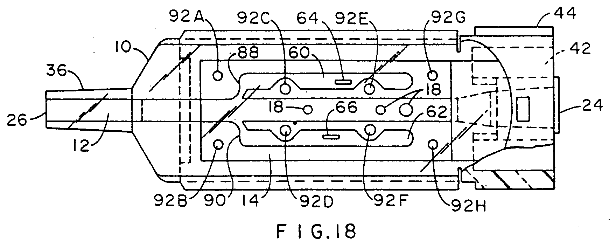

In addition to channel 12 having fluid contact with a sensor, sensor 18 on the substrate of sensor element 14 as shown in Figure 3, the sensor assembly of the present invention may have a plurality of channels. The arrangement of the channels and the sensors is such that when a plurality of channels and a plurality of electrodes for the sensor or sensors are present at least one channel can be in fluid contact with at least one sensor. With this type of arrangement, the one or more side channels are in fluid contact with at least channel 12 or with another channel connected to channel 12 so that each of the one or more side channels can provide fluid contact with the at least one electrode associated with that channel. This sensor or electrode to channel relationship is shown in Figures 3 and 18, while the channel to channel relationship is shown in Figure 5. In Figure 3 channels 60 and 62 are formed by the one housing section 32 and the adjacent positioning of sensor element 14 in the internal cavity or space formed by the joined two housing sections 32 and 34 in a fashion similar to the aforementioned formation of channel 12. Sensors or electrodes but preferably reference electrodes 64 and 66, respectively, are in contact with channels 60 and 62. Preferably, these channels contain fluid like hydrating fluid 22 as contained in channel 12 or any fluid known in the art to act as an electrolyte for the reference electrode. Preferably, the potentiometric and/or amperometric sensors are located in channel 12 and only the reference electrodes are located in the one or more additional or side channels 60 and 62 and most preferably each reference electrode is located in a separate side channel as shown is Figure 18.

The electric circuit means 20 is shown in Figure 1 by line 20, which connects to electrical attachment means 16 preferably by a cable 16 electrically connecting to the nonconducting substrate of sensor element 14. Cable 16 can be any suitable electronic multiple conductor with suitable leads to carry analog signals and preferably not binary signals. Preferably, the cable is a ribbon-type cable with a plurality of wires in one tape-like strip to provide the sensor element 14 with electrical communication from the at least one sensor 18. The connection of the electrical circuit means or cable 16 to the nonconducting substrate of 14 is in a manner to communicate electrically with at least the one or more sensors 18 or electrodes but to avoid contacting the hydrating fluid 22 which may cause short circuits or current leakage.

The hydrating fluid 22 is any liquid suitable for maintaining the membrane of sensor 18 in a non-dried state. For instance, the liquid will have some amount of water although a minor quantity of organic liquids may also be present. Preferably, the liquid is a stable liquid for storage ranging from a short time (days or weeks) to prolonged periods of time of several months. Preferably, the liquid is an aqueous solution that is isotonic with any electrolyte in the one or more sensors. More preferably, the hydrating fluid 22 is also isotonic to act as the electrolyte for any reference electrodes that may be present on the sensor element 14 as reference electrodes 64 and 66 as shown in Figure 3. A suitable example of a hydrating fluid 22 is an aqueous solution comprising: disodium hydrogen phosphate, potassium dihydrogen phosphate, sodium bicarbonate, and sodium chloride. Such a solution can have a varying range of amounts for the individual constituents but most preferably for the aforelisted salts the amounts are in millimoles per kilogram of water in the order listed as follows: 4.8, 13, 22, and 12.5. The quantity of hydrating fluid 22 in channel 12 or the plurality of channels is at least that which is sufficient to cover or remain in contact with the one or more sensors. For example, seals 30 and 28 of Figures 1 and 2 could be in channel 12 rather that at the opening so as to maintain the hydrating fluid 22 in contact with the one or more sensors. In this situation the seals 28 and 30 would be more plugs rather than foil-backed seals.

Figure 4 shows an alternative embodiment of housing 10 to that depicted in Figures 1 and 2 in that tip section 38 is a mirror image or near mirror image of tip section 36 at the other end of the housing. Hence, the difference between the embodiment of Figure 4 and that of Figure 1 is that the housing 10 with the near mirror image of tip sections 36 and 38 does not have the flared end 40 of Figure 1. Figure 4 is a side section without the back portion 34, the sensor element 14, and electrical attachment means 16 as shown in Figure 1 and the electrical insulating means 54 shown in Figure 2. Figure 4 has at least one channel 12 with openings 24 and 26 sealed by seals 28 and 30. With this view the internal space 82 is shown where sensor element 14 is placed. Since the sensor element 14 can have any geometric shape, the internal space should be of a matching shape to accommodate the sensor element 14 and to minimize the possibility of gaps that would allow hydrating fluid to leak into contact with the electrical circuit means 20 or cable 16. Also shown in Figure 4 is the slot 84 which is a portion of the slot and tab assembly shown in Figure 1 as 52. This slot assists in aligning the attachment of back 34. As shown in Figure 2, the slot 52 runs transversely across the bottom of the upper section 32 of housing 10. In addition the void space 86 of Figure 4 can be present or absent depending on the molding process for producing the sensor assembly.

Figure 5 is another bottom view taken along a transverse line 5-5 of Figure 4. Here, internal space 82 is of adequate dimensions to hold a comparably dimensioned sensor element (not shown) to completely fill the space to provide walls for not only channel 12 but also channels 60 and 62. Preferably, channels 60 and 62 are joined to channel 12 through joining sections 88 and 90. As an alternative embodiment, although not shown in Figure 5, similar joining sections can be present at the other end of channels 60 and 62 connecting to channel 12. The difference between channels 60 and 62 and channel 12 is that the former are side channels which do not flow through the housing 10 but are joined for fluid flow to the main channel 12. The diameters of the channels can be the same or the diameter of channels 60 and 62 can be smaller than that of channel 12.

As shown in Figure 18, the plurality of channels preferably associate with the electrodes and sensors so that the one or more sensors 18 of sensor element 14 are positioned in channel 12, while other sensors 18 or electrodes are on sensor element 14 for positioning in side channels 60 and 62. With this arrangement it is preferred that sensors for measuring the partial pressure of oxygen and electrodes or sensors for measuring the partial pressure of carbon dioxide and for measuring pH are in channel 12, while channels 60 and 62 each have a reference electrode 64 and 66, respectively. When hydrating fluid 22 is in the channels 60 and 62, the fluid in channel 12 is more easily displaced with the sample fluid to be measured relative to the fluid 22 in channels 60 and 62. Therefore, reference electrodes 64 and 66 are measuring as a reference a known fluid for comparison for the measurement of the sample fluid in channel 12.

The housing 10 has a plurality of attachment ports to assist in holding the sensor element 14 in place in the internal space 82 and to assist in attachment between top section 32 and bottom section 34. The preferred rectangular shape of internal space 82 is shown in Figure 5. The number of ports 92 can range from around 2 to around 8 although higher numbers can be present and the ports 92 can range in geometric configuration from circles to slots to square and the like for mechanical or chemical attachments. The mechanical attachments can be plastic or metal rivets or fasteners like screws and the chemical attachment can be adhesives, preferably curing adhesives such as a UV-curing adhesive. Figure 5 shows eight ports (92A - 92H). The preferred number of eight ports are arranged such that two of the ports, 92C and 92E, are positioned between channel 12 and side channel 62 and two ports, 92D and 92F, are positioned between main channel 12 and side channel 60. The other four ports are located outside of the channel array and preferably near the corners of the internal space 82 so that ports 92A and 92B are at the two corners of one end of internal space 82 and ports 92G and 92H are located at the other two corners of internal space 82. The ports 92 extend from the internal surface 94 of housing member 32 to the external surface 80 shown in Figure 4 of housing section 32 to provide for placement of the attachment means from the outside or external to housing 10 and into the internal space 82 when it contains the sensor element 14.

Also shown in Figure 5 is inner annular space 42, in phantom, slot 48, opening 24 and seal 28. At the distal end of the housing 10, the tip 36 is shown with a partial cutaway showing channel 12 that is sealed with seal 30 over opening 26. Also a void 86 is shown in Figure 5 which is present to decrease the amount of material used and to reduce the weight of the sensor assembly. The void 86 can extend completely through the housing sections 32 and 34, respectively, of housing 10. The attachment means of member 32 for engagement with member 34 are shown in Figure 5 at 72 and 76 as longitudinal ridges along the exterior sides of the planar portion of housing member 10.

Figure 6 is a sectional view of the proximate end of the sensor assembly showing opening 24 of channel 12. The channel extends into the flared end section 40 of the sensor assembly to form tip 38 for channel 12. Between tip 38 and rim 44 of flared end 40 is the inner annular space 42. Extending beyond annular space 42 into housing 10 in the direction of internal space 82 is detent 96 on both external sides of tip 38 for formation of the more or less planar portion of housing 10. Extending outwardly from and normal to the peripheral surface of flared end 40 are projections 98 and 100. These projections extend outwardly a short distance and preferably in the same plane so they continue along the same longitudinal axes of housing 10 as ridge members 56 and 58 shown in Figure 3. These projections assist in handling of the sensor assembly when it is connected to a syringe (not shown). As previously discussed, the inner annular space 42 accepts a Leur fitting for attachment as depicted in Figure 6 to a conventional syringe.

Figure 7 shows the distal end of sensor housing 10 in a sectional view. The tip section 36 provides a housing for channel 12 which has opening 26 at this distal or receiving end. Also shown are ridges 56 and 58 which extend from housing 10. As mentioned for and shown in Figure 6, the projections 98 and 100 are extensions of these ridges since Figures 6 and 7 are the opposite ends of the housing 10 rotated in the same horizontal plane 180°. The internal space 82 is shown above and below and to one side of channel 12. Tip section 36 extends beyond the shoulder region 102 of the essentially planar section of the housing 10 as shown in Figure 5.

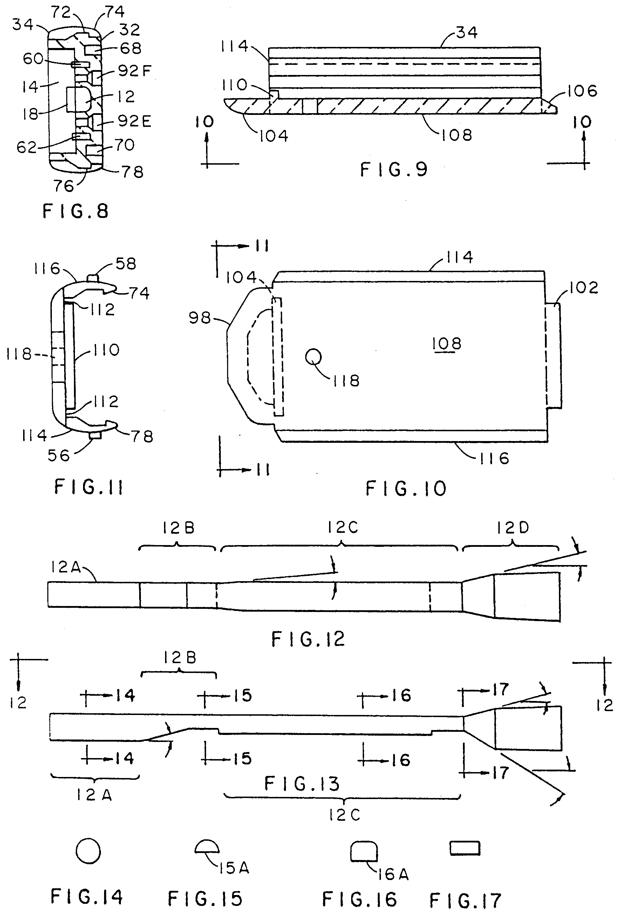

Figure 8 shows a sectional view along line 8-8 of Figure 5 of the housing section 32 of the sensor assembly. Member 32 has a portion of internal space 82 which is completed upon attachment of housing member 34 as shown in Figure 1. Figure 8 is rotated 90° in the clockwise direction in the same plane in regards to Figure 3. Also shown are fastening ports 92F and 92E. The fastening ports 92F and 92E are interspersed one above and one below channel 12. Above port 92F and below 92E are secondary channels 60 and 62, respectively. Fastening means 72 and 76 of housing member 32 are shown in engaged fashion with fastening means 74 and 78 of housing member 34 for engagement of the two housing members 32 and 34. Another section of the slots 68 and 70 that extend longitudinally along the exterior surface of housing member 32 as depicted in Figure 4 as 80 is shown in Figure 8.

Figures 9, 10 and 11 show the housing member 34 of the sensor assembly.

Figure 9 is the side section elevational view of the back portion of the housing 10 having a tongue section 104 which when attached to member 32 covers void space 86 as shown in Figure 5. When housing members 32 and 34 are attached, tongue 104 conforms to the shape of the shoulders 102 of the upper section as more clearly shown in Figure 10. Also, lip 106 extends from the exterior surface 108 of housing member 34 so that when member 34 engages member 32 a slot-like opening is available for cable attachment means 16 for electronic communication of the sensor assembly. Tab 110 is for mating attachment to slot 84 of housing member 32. Tab 110, which is a portion of assembly 52 shown in Figure 1, projects preferably at around a 90° angle from the interior surface 112, shown in Figure 11 of member 34. The tab 110 slidably engages or fills slot 84 which both traverse the longitudinal axis of members 34 and 32, respectively, as a guide for engagement of these members. Of course, the tab and slot arrangement can be any guiding mechanism arranged in any fashion and at any location on members 32 and 34 to assist in their engagement as long as there is no interference with the sensor element 14.

The tab 110 and slot 84 and lip 106 for the cable slot are preferably located where shown in the Figures. This arrangement allows for the cable attachment means 16 to associate with the housing 10 at or near the proximate end of the housing 10 and travel to around the distal end of the housing for attachment to the electric circuit means 20. This way the cable can travel a substantial distance of the longitudinal axis of the internal space 82 to retard the loss of heat from the sensor element 14 through the cable.

As shown in Figure 9, member 34 has a side element laterally extending at around a 90° angle from each side of internal surface 112A of member 34. One of these sides is shown in Figure 9 as 114A, while a mirror image laterally extending side 116A is behind side 114A in Figure 9 as shown in Figure 11. The laterally extending sides 114A and 116 extend in connecting fashion to engage the housing member 32 preferably on its lateral sides. As shown, lateral side 114A has a ridge which is ridge 56 of Figure 3 that travels the longitudinal axis of the lateral side and that extends outwardly preferably at an angle of around 90° from side 114A.

Figure 10 is a bottom view of member 34 taken along lines 10-10 of Figure 9 showing tongue member 104 and lip member 106 both beginning at one lateral side and extending transversely almost or to the other lateral side. In addition, hole 118A is in the back for insertion of chemical electronic insulation means 54. Tab 110 is shown in phantom and the laterally extending sides are approximately normal to the plane of exterior surface 108.

As shown in Figure 11, a forward end view of member 34 taken along lines 11-11 of Figure 9, tab 110 projects laterally and approximately normal to the plane of surface 108 in this view. The laterally extending sides 114A and 116A each have the attachment means 74 and 78 to matchedly engage the attachment means 72 and 76, respectively, on member 32. Also, the ridges 56 and 58 are shown extending from laterally extending sides 114A and 116A, respectively. Also shown in phantom is hole 118A.

Figure 12 shows channel 12 as it longitudinally passes through housing 10 in a plan elevational view of the contour of channel 12 formed along the longitudinal axis on the interior of the housing. The contours shown in Figures 12 and 13 are depicted in phantom and Figures 1 and 2, respectively. Figure 12 is a top view of the channel taken on lines 13-13 of Figure 13. The 12a portion indicates that portion of the channel passing from the tip 36 in Figure 1. Portion 12b is that part of the channel which passes through the shoulder section, 102 of Figure 5, of housing 10 into the interior cavity. Portion 12c is that part in the internal space 82 of housing 10 formed from housing member 32 and the sensor element 14 occupying space 82 in between top member 32 and bottom member 34. Preferably, cable attachment means 16 is in between sensor element 14 and housing member 34. Portion 12c preferably has a wider diameter than that of 12a or 12b although it could have the same diameter. Portion 12d is that section of the housing enclosed by tip 38 passing from the internal space 82 into the flared end 40 of the housing. This portion preferably has a wider diameter for coupling to a fluid collection pressure device such as a syringe.

The side elevational view of Figure 13 again shows portion 12a, where a cross section along line 14-14 is shown in Figure 14. As shown in Figure 14, the channel 12 at this location has a circular cross section and is the portion formed by the tip 36. As shown in Figure 13, the slope of the bottom of the channel 12 increases as the bottom ascends to the bottom level as shown in Figure 13 around the cross sectional line 15-15. The cross sectional view shown in Figure 15 along a line 15-15 in Figure 13 details a flat bottom portion of the channel 12 for a portion of 12b. Here, the channel 12 has a flat bottom as the channel 12 enters she planar section of the housing 10. Portion 12c extends along the longitudinal axis of the planar section of the housing and has a cross section as shown at Figure 16, which is along line 16-16 of Figure 13. At cross sectional line 17-17 of Figure 3, the bottom is notched by the detent 96 of Figure 6 to provide for coupling to the device like a syringe. After the point in Figure 13 at line 17, the diameter of the channel 12 widens again. The constriction and widening of the diameter of the channel 12 is for the purpose of obtaining an opening that is a minimum diameter for accepting a standard Leur fitting like that on a syringe. The portion of the channel 12 shown in Figure 13 is that which is preferably formed by the housing section 32. Preferably, the other housing section 34 forms little, if any, of the portion of the channel 12. The beginning of portion 12d has the cross section shown in Figure 17 just as the channel exists the planar section into the flared end section 40 of the housing 10. A cross section of the end of the channel 12 is shown in Figure 6 formed by the tip 38.

The sensor assembly is prepared by placing the sensor element 14 with one or more sensors 18 having unhydrated membranes, preferably three sensors 18, one for measuring the partial pressure of oxygen, another for measuring the partial pressure of carbon dioxide and a third for measuring the pH of fluids, preferably fluids like blood. The sensor element 14 is placed in the internal space 82 of housing member 32 as shown in Figures 1, 2, 4 and 5. The electrical attachment means 16 is electrically connected to sensor element 14. When the sensor element 14 is placed in internal space 82, this electrical connection is preferably at the distal end although it could be at the proximate end of housing 10. The cable stretches along the length of the sensor element 14 between the element 14 and the housing member 34 and exits housing 10 at the near proximate or exiting end 40. The attachment of the cable 16 to the nonconducting substrate of the sensor element 14 can be by any attachment means known to those skilled in the art for attaching cables to substrates for electronic circuits. Additionally, it is preferred to have a foam pad between the electrical attachment means 16 and housing member 34 so that there is uniform compression of sensor element 14 to the bottom of housing section 32. Housing member 34 is aligned with housing member 32 through the tab-and-slot arrangements 110 and 84 and preferably snapped through attachment means 72, 74, 76 and 78 as shown in Figure 8.

An adhesive that is curable by ultra-violet light is placed in at least some of the ports 92a through 92h and also preferably along the interior surface of the housing member 32 by wicking. Any suitable adhesive known to those skilled in the art of joining polymeric parts to glass or ceramic substrates can be used, but it is preferred to use an ultraviolet light curable adhesive that is substantially water insoluble in the cured state. A nonexclusive example of a suitable material is the UV curable epoxy adhesive P/N 10033 available from an electronic materials vendor. Also, this adhesive may be used with about 0.005 percent by weight polychrome blue organic dye to highlight the details of the adhesive. Before placing the housing with the adhesive in a UV-curing zone to cure the adhesive, it is preferred to allow the adhesive to wick within the internal opening 82 along the surface of housing member 32. The wicking of the adhesive within the cavity is preferably on both sides of the side channels. Preferably, the quantity of adhesive that is used allows for wicking lengthwise along the bottom of section 32 on both sides of channel 12 and under the channel so the bead of adhesive is near continuous on both sides of the channel 12. The curing can occur in any commercially available UV-curing oven with or without a conveyor. After curing the wicked adhesive, the housing 10 is cooled to ambient temperature. Preferably, now the UV-adhesive cured by ultraviolet light is placed in the ports and again placed in the UV-curing oven.

After the joining of the housing members with the adhesive, the electronic isolation means, preferably an epoxy, that is cured at room temperature and atmospheric pressure is filled through hole 118 in member 34 as shown in Figure 10 into the internal space 82 that is not already occupied. To increase the rate of cure, the housing is preferably placed and maintained in an oven for about two hours at 60°C. After the electronic insulating material has hardened or cured, as in the case of an epoxy material, the housing 10 can be pressure tested at an air pressure of around 6.9·10⁴ Pa (10) to around 10.4·10⁵ Pa (15 psi).

Upon joining of the housing members to contain the sensor element 14 and cable 16, one opening of the channel 12 is sealed which can be either opening 24 sealed with seal 28 or opening 26 sealed with seal 30 by a heat sealing but preferably an induction sealing process. After the sealing of one end, the hydrating fluid 22 is added to channel 12 and any side channels to fill substantially all of the channels although small amounts of air bubbles can be tolerated in the channels but preferably the channels are filled to capacity. The remaining opening of the housing is sealed with the other seal through a heat sealing process but preferably an induction sealing process.

The sealing of seals 28 and 30 to channel 12 depends on the presence or absence of any mechanical attachment means such as caps or the like and the type of thermoplastic adhesive polymer. When the cap is present, either the heat or induction sealing process can be used and any cap known to those skilled in the art for covering an opening in a plastic vessel can be used such as a screw cap or a snap cap. With the use of screw or snap caps, the seals 28 or 30 can be placed in the cap, and the cap applied to one of the openings 24 or 26 of the channel 12. When the cap is absent, induct on sealing should be used to avoid the escape of any hydrating fluid (22) (gas) from or the influx of gas into the channel 12. In general, the sealing needs to overcome the hurdle of adhering the seal to a plastic or polymeric substrate in a possibly moist environment since there may be moisture or liquid on the surface of tips 36 and 38 after the addition of the hydrating fluid.

With the capped channels a plurality of housings 10 can be heat or induction sealed. The heat sealing temperature and the pressure applied by the cap can vary depending on the type of heat sealable resin that is used with seals 28 and 30. In general, however, sufficient results are obtained by conducting the heat sealing at a temperature higher than the softening or melting point of the heat sealable resin and the pressure is sufficient if it doesn't cause excessive or substantial flow of heat sealable resin away from the area to be sealed. For heat sealing of a polypropylene heat sealable resin, the seal pressure by the screw-type cap is in the range of 2 to 5 kilograms per centimeter² (Kg/cm²) for the temperature of heat sealing in the range of 180°C to 280°C. For a polyamide, like Nylon 12, heat sealable resin the pressure is in the range of 2 to 7 Kg/cm² for the temperature of sealing of around 200°C to 300°C. For polytetramethylene terephthalate the seal pressure is around 2 to 7 Kg/cm² for the sealing temperature in the range of 220°C to 320°C. The time required for heat sealing varies depending on the thickness of the heat sealable resin layer.

Generally, the heat sealing is conducted for a time sufficient to perform melting and bonding of the sealable resin, for example 0.1 to 5 seconds. The heat sealing operation can be performed in an operation comprised of one stage or two or more stages. In the latter case, the same or different temperature and pressure conditions as those aforementioned can be adopted at these stages. The formed sealed area is cooled, if necessary, under application of pressure by optional means to form a sealed area with good sealing efficiency. For instance, immediately after completion of the heat sealing operation, the heat sealed area in which the resin is still in the softened or molten state is pressed by two positively cooled press bars whereby the resin is solidified. Although any operation known to those skilled in the art to cool and harden the adhesive polymer can be used.

For induction sealing, generally any induction sealing process known to those skilled in the art of induction sealing can be used. A nonexclusive example of a suitable process involves placing the housing 10 with seal 28 or 30 in place over the opening 24 or 26, respectively, of the channel 12 on the flat surface of the rim of one of the tips 36 or 38, respectively. With the seal in place over the opening, that end of the housing with the seal over the opening is held with the application of pressure against a region where it is exposed to high-frequency electromagnetic waves. A suitable piece of equipment is that available from Giltron, Inc., Medfield, Massachusetts 02052, referred to as Foil Sealer Induction Heat Sealer, Model PM1. The aluminum foil of the seal is locally heated to a point whereby it heats and melts the adjacent resin layer. The melted resin layer adheres to the top horizontal surface of the rim of the tip that surrounds the opening. The hydrating fluid is placed in the channel in the aforementioned manner and the other seal is placed over the other opening at the other tip and subjected to induction heating in the same manner to seal the other end.

When the sensor assembly needs to be sterilized, the sensor assembly with the sealed channel can be sterilized by gamma-sterilization or pasteurization sterilization. A nonexclusive example of a pasteurization technique that can be used with the sterilizable container of the present invention is heating one or more of them at a temperature of around 70°C for eight hours. The gamma-radiation sterilization can occur with the use of any gamma-sterilization equipment known to those skilled in the art.

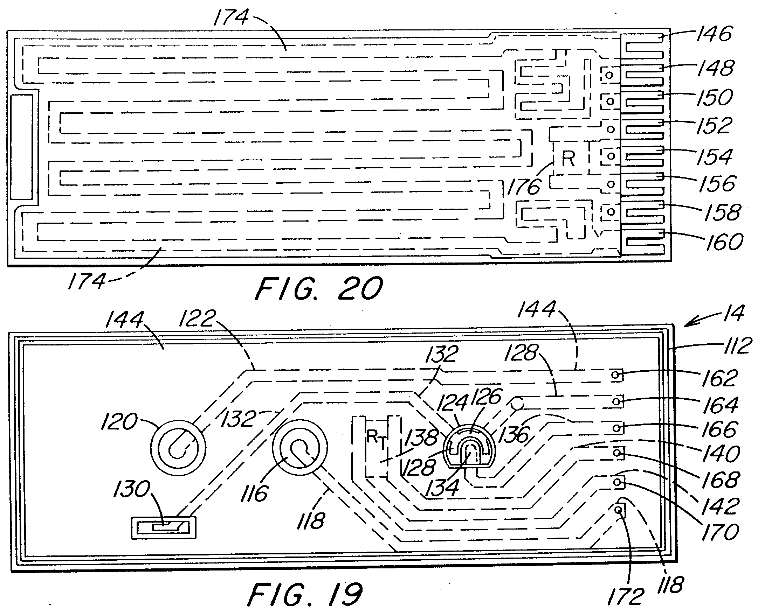

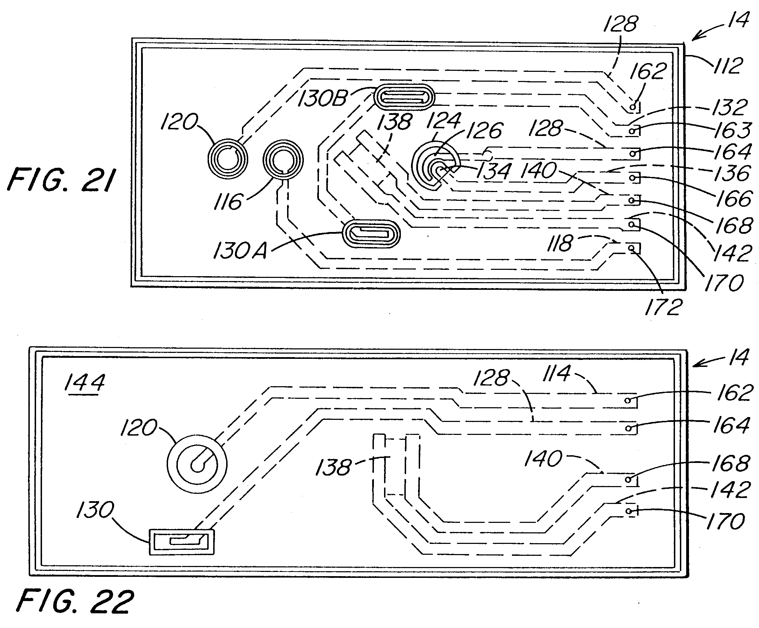

A nonexclusive example of the sensor element is shown in Figures 19-22 with the preferred sensor element 14 shown in Figure 21. The following description of these figures new reference numbers are used for components that may have been previously discussed in regards to the aforedescribed electrochemical sensor assembly.

Figure 19 is a top planar view of one side of the wiring substrate, hereinafter referred to as "board" with at least one electrochemical sensor 14 of the present invention, where the components have particular shapes. Any other shapes than those shown in Figure 19, that are known to those skilled in the art for the particular components, can be used.

The improved wiring board 14 may be produced from any number of well known layered circuit technologies, as for example, thick film, thin film, plating, pressurized laminating and photolithographic etching, however, the thick film technique is preferred.

The substrate 112 on both sides of the board 14 is any glass or ceramic including sheet or chip or nonconducting substrate like wood or nonconducting polymers or commercially available frit that can be used as the substantially smooth flat surface of the substrate layer 112. Nonexclusive examples include borosilicate glass as is known to those skilled in the art for producing thick film or layered circuits. A nonexclusive but preferred example of which includes a ceramic base having around 96% A1203 such as that available commercially from Coors Ceramic Company, Grand Junction, Colorado.

The substrate layer 112 is essentially flat and any substrate known to those skilled in the art for forming printed wiring circuits can be used. It is preferred that the composition of the substrate can endure the presence of electrolyte that has a pH in or over the range of 6 to 9 and remain unaffected for a substantial period of time.

As can best be seen in Figure 19, the board 14 is provided with a number of electrodes and more particularly, electrodes useful in the measurement of blood gas oxygen, carbon dioxide and pH. The board 14 is also provided with a thermistor 138 and resistor arrangement 176 to indicate the temperature at any time on the board 14 as well as reference electrodes for establishing an accurate reference potential, all of which will be described in further detail below.

On the substrate layer 112 is a patterned metallic layer 114 with a number of extensions which act as the electronic conducting pathway between a voltage or current source external to the board 14 (not shown) and each of the components. The extensions constitute the transmission section, where each extension has a component at its end. The several extensions also have the ability to transmit changes in voltage from the components of the board 14 to the Analyzer (not shown in this Figure).

The pH sensing electrode 116 is located at the end of extension 118; the carbon dioxide sensing electrode 120 is located at the end of extension 122; the oxygen sensor 124 is provided with an anode 126 located at the end of extension 128; the reference electrode 130 is located at the end of extension 132 which extends from anode 126 of the oxygen sensor 124; the oxygen sensor 124 is further provided with a cathode 134 which is located at the end of extension 136; thermistor 138 is located at the end of extensions 140 and 142.

As shown in Figure 20, the patterned metallic layer 114 has metallic external leads 146-160 on the other side of the substrate 112. Although the external leads 146-160 are shown on the opposite side of the substrate 112, they can also be on the same surface as their associated metallic lead patterns and components.

External leads 148-158 are conductively associated with the components on the Figure 19 side of the substrate layer 112 and external leads 146 and 160 are in metallic electrical conducting contact with a thick film heater 174 which is provided on the Figure 20 side of the substrate layer 112. The heater 174 traverses the board in a serpentine fashion to provide a grid of heat to the non-electrically conducting substrate and its function will be described below.

External leads 152 and 156 are in metallic electrical conducting contact with a resistor 176 which is also provided on the Figure 20 side of the substrate layer 112. The resistor 176 is in a half-bridge relationship with the thermistor 138 and, as such, it commonly shares external lead 152 with the thermistor 138; thermistor 138 also being in metallic electrical conducting contact with external lead 152. The function of the thermistor 138 and resistor 176 arrangement will be described below.

The patterned metallic layer 114 is formed by printing pastes deposited onto a substrate in the desired pattern to act as ohmic conductors. Nonexclusive examples of suitable heat resisting metals include; noble metals such as platinum (Pt), ruthenium (Ru), palladium (Pd), rhodium (Rh), iridium (Ir), gold (Au) or silver (Ag) or other metals traditionally used as Clark cells and other ISE's and mixtures thereof. A nonexclusive but preferred example of a suitable paste is a silver paste of the type produced and available from Electro-Science Laboratories, Inc. under the trade designation ESL 99112.

The metallic layer 114 is dried to produce the above noted patterned conductive pathways 118, 122, 128, 132, 136, 140 and 142 of Figure 19. Any method known to those skilled in the art for producing a sufficient thickness of metallic tracing can be used. Preferably pathway 128 has ground 129.

Preferably, the silver pastes are oven dried and fired at a high temperature in a furnace. Firing can be accomplished at a temperature in the range of around 800°C to 950°C for a period of around 1 to 20 minutes. With this procedure, the thickness of the layer of the metallic conducting tracing is usually in the range of around 0.927 mm (.0005) to 0.025 mm (.001 inches.) Although the aforementioned are preferred conditions, general conditions for obtaining a proper thickness can be used where the thickness can generally range from about 0.01 mm (.0004) to 0.04 mm (.0015 inches).

The aforementioned conductive patterns are encapsulated with a glass ceramic mixture or a ceramic insulating material such as alumina or spinel. This encapsulation can range from a total encapsulation to encapsulation except at the end of the metallic pattern.

The aforementioned electrodes are preferably produced by one of the layered circuit techniques. This involves leaving the respective shaped ends uncovered while the metallic patterns are completely covered by the encapsulant. The encapsulation of the metallic patterns can range from encapsulating each from the other to a sufficient degree for electrical insulation of the conductive patterns and any conductive layers from each other.

As shown in Figure 19, the encapsulant can extend across the whole board from edge to edge as generally shown at numeral 144. Preferably, the thickness of the encapsulant layer 144 is that which is adequate to seal the underlining metallic layer and to provide insulation for the metallic patterns. Preferably, the thickness of the layer is around 120-130 microns.

A preferred glass ceramic mixture useful as the encapsulant is the type produced and available from Electro-Science Laboratories, Inc. under the trade designation ESL 4904.

The several electrodes may be masked during the encapsulation to keep them suitably uncovered for the addition of active materials (e.g. polymer liquids and pre-cut dry film membranes) over the appropriate electrodes on the surface of the substrate layer 112.

This process involves masking the electrodes by the use of polymer film coating on the screen used to screen print the encapsulant. This leaves the underlying silver exposed to form the electrodes for active materials. It is also possible to use multiple layers of the metallic conductive layer or encapsulant.

Preferably, the glass composition for the encapsulant 144 as with the substrate 112 is selected to possess good chemical stability and/or moisture resistance and to possess high electrical insulation resistance. Also, the metallic and encapsulant materials are selected so that they can endure the presence of an electrolyte in a similar manner as the substrate composition.

The geometry of the several electrodes could be made by a laser beam to carve or cut or trim the electrode, however, they are preferably prepared by the aforementioned layered circuit technique.

The serpentine formed heater 174 and the resistor 176 on the Figure 20 side of the board may be prepared by a number of commercially available techniques, however, they are preferably thick film devices prepared by the aforementioned layered circuit technique.

Holes 162-172 may be drilled by a laser through the substrate 112 to conductively connect the metallic extensions 118, 122, 128, 140, 142 and 136 traced on the Figure 19 side of the substrate layer 112 with their respective metallic external leads 148-158 on the Figure 20 side of the substrate layer 112. In general, these openings 162-172 are produced by the focused laser beam drilling a hole by heating a small volume of material to a sufficiently high temperature for localized melting and/or vaporization.

The external leads 146-160 may be produced on the other side of the side of the substrate layer 112 with the same paste and firing as that done for aforementioned metallic patterns. The metallic external leads 146-160 are in metallic electrical conducting contact with the various components on each side of the board. As before mentioned external leads 146 and 160 are in metallic electrical conducting contact with the heater 174 and external leads 152 and 156 are in metallic electrical conducting contact with a resistor 176 which commonly shares external lead 150 with the thermistor 138; thermistor 138 also being in metallic electrical conducting contact with external lead 150. External lead 158 is in metallic electrical conducting contact with the CO₂ sensing electrode 120; external lead 148 is in metallic electrical conducting contact with the pH sensing electrode 116; external lead 148 is in metallic electrical conducting contact with the CO₂ sensing electrode 120; external lead 152 is in metallic electrical conducting contact with the anode 126 of the oxygen sensor 124, the anode 126 having an electrical ground 129; external lead 152 is also in metallic electrical conducting contact with the reference electrode 130 which is located at the end of extension 132 which extends from anode 126 of the oxygen sensor 124, external lead 154 is in metallic electrical conducting contact with the cathode 134 of the oxygen sensor 124;

The holes 162-172 have been drilled through the substrate layer 112 and when the metallic layers are screened such electrical connections are formed. Alternatively, the metallic external leads 146-160 can be produced and preferably are produced by a very high powered carbon dioxide laser. This can be accomplished by the supplier of the nonconducting substrate and in this case the metallic layer is added to the substrate so each conducting pathway electrically connects with an external lead.

As described above, the process of masking the electrodes by the use of polymer film coating on the screen is used to screen print the encapsulant. This leaves the underlying silver exposed to form the electrodes for active materials. It is also possible to use multiple layers of the metallic conductive layer or encapsulant and the outer layer of the encapsulant may be solvent or thermoplastically bondable and may include polymers, as for example, acrylates or polyvinyl chloride as the major component in the encapsulant. The purpose of the outer coating or encapsulant is to enhance bonding of the active materials and, in particular, to provide a reliable surface for the attachment of the liquid or solid film type membrane materials.

Each of the sensing electrodes are fabricated to perform their specific task and may be selected from many commercially available electrode components. The pH electrode 116, CO₂ electrode 120 and the oxygen sensor 124 are each fabricated with a membrane which maintains their respective electrolytes in a fluid tight manner in the cavities or openings in which the electrodes are positioned.

The pH electrode 116 and the CO₂ electrode 120 may be similar in regards to the circuit geometry and electrolyte and may be provided with membranes suitable for the particular characteristic being measured.

For pH electrode 116, for example, the use of cation permeable and particularly hydrogen ion permeable membrane may be used. A number of such cationic exchange materials may be utilized, as for example, membranes fabricated from copolymeric vinyl ethers as manufactured by E.I. duPont under registered trademark NAFION.

The membrane for the CO₂ electrode 120 may be fabricated from a wide range of commercially available carbon dioxide permeable polymeric materials. The electrolytes of the pH electrode 116 and the CO₂ electrode 120 are bound by their respective membranes.

The membrane for the oxygen sensor 124 may be fabricated from a polymeric material such as polystyrene in an organic or inorganic solvent. The oxygen permeable electrolyte of the oxygen sensor 124 bathes the anode 126 and cathode 134 to provide electrical ionic contact between the two. The electrolyte can be any electrolyte known to those skilled in the art for Clark Cell as, for example, a saline solution based on potassium chloride or sodium chloride.

The anode 126 of the oxygen sensor 124 is electrically grounded at 129 to assure that the electrolyte potential does not change and that the opening to the electrolyte is held at some voltage which is the same as the anode potential so that the electrolyte is grounded in the electrode configuration.

The reference electrode 130, which is located at the end of extension 132 and which extends from anode 126 of the oxygen sensor 124, provides a highly stable reference potential. This reference potential provided by the reference electrode 130 facilitates accurate measurement of the blood gases. The reference electrode 130 may be fabricated from a number of suitable materials known to those skilled in the reference electrode art such as a silver and silver chloride composite using the aforementioned layered circuit technique.

The thermistor 138 is a thick film thermally sensitive resistor whose conductivity varies with the changes in temperature. The thermistor 138 may be fabricated from a number semi-conductive materials as, for example, oxides of metals. The thermistor 138 may be formed and applied to the substrate layer 112 by the use of the aforementioned layered technique. The temperature coefficient of the thermistor 138 is large and negative and is used to sense the temperature of the board 14 at all times when the board 14 is coupled to its associated electronic module (not shown). It is operated at relatively low current levels so the resistance is affected only by the ambient temperature and not by the applied current.

As before described, external leads 152 and 156 are in metallic electrical conducting contact with a thick film resistor 176 which is provided on the Figure 20 side of the substrate layer 112. The resistor 176 is in an half-bridge relationship with the thermistor 138 and, as such, it commonly shares external lead 152 with the thermistor 138; thermistor 138 also being in metallic electrical conducting contact with external lead 152. The half-bridge circuit configuration is a voltage divider and generates a ratiometric output to the module. This is important for it allows the actual resistance values to float and results in highly consistent and accurate temperature sensing and control of the board 14 on a board to board basis. Accuracy and consistency of the resistor 176 and thermistor 138 arrangement is achieved by calibrating the board 14 by laser trimming of the resister 176 to produce zero volts at 37°C. The laser beam is precisely deflected across the thick film resistor 176 to produce the desired temperature voltage relationship. A current is applied at external leads 150 and 152 by the module until zero volts is achieved. This gives a linear output so that the temperatures can be measured other than 37°C from the slope of the line from the calibration at room temperature and 37°C. The resister 176 has essentially zero temperature coefficient and, accordingly, may be placed without any adverse effect on the sensing capability of the associated thermistor 138, on the Figure 20 side of the board 14 with the heater 174.

Accurate sensing of the ambient temperature of the board 14 is required to precisely control the heater 174 to ultimately maintain, within a narrow distribution of temperatures, the desired operating surface temperature on the Figure 19 or sensor side of the board 14.

Placement of the thermistor 138 is another important aspect. As can be seen in Figure 19, the thermistor 138 is placed in the same plane and in close relation to the sensors 116, 120 and 124 to thereby accurately sense the ambient temperature at or near such sensors.

This physical placement of the thermistor 138 allows for the rapid adjustment of the heater 174 by the module to maintain the desired operating temperature. The thermistor 138 resistor 176 arrangement provides temperature measurement accuracy of within 25°C.

This physical placement of the thermistor 138, so close to the sensors, requires that it be correctly fabricated to ensure that it is electrically isolated from the electrolytes of the several sensors. The encapsulant for the thermistor 138 must be thick enough to accomplish the electrical isolation yet thin enough so as not to lose any response time.

The heater 174, provided on the Figure 20 side of the board 14, rapidly and accurately produces the necessary heat in response to any temperature change sensed by the thermistor 138; the thermistor 138 and the several sensors 116, 120, and 124 all being in the heated region produced by the heater 174.

Thick film heaters are not generally considered to be rapid response devices and their heat output tends to take a relatively long time, in terms of electronic devices, to change. To improve the responsiveness of the heater 174, it is powered by a controlled DC voltage whereby the heater is regulated by a combination of proportional (P), integral (I) and/or derivative (D) controls, preferably PID control thereby reducing the amount of overshooting or undershooting by the heater of a predetermined temperature. This not only increases the responsiveness of the heater 174 but also allows for better overall thermal control including avoiding the heater 174 from overshooting or undershooting the desired temperature.

The timing sequence for the production of the heat by the heater 174 to the several sensors is provided by the natural state of power supplied to the board 14 when it is connected to the Analyzer. This same power will also produce the read-out from the measurements by the sensors of the blood gas oxygen, carbon dioxide and pH. This timing sequence facilitates a room temperature calibration of the board 14; a elevated temperature calibration at 37°C and then the measurement of the blood gas oxygen, carbon dioxide and pH.

Prior to any measuring of the blood gases by the several sensors 116, 120 and 124, all or part of the board 14 may be exposed to or stored with a calibration liquid, with the several sensors being exposed to the fluid. To measure the blood gases, the several sensors 116, 120, 124 are brought in contact with the volume of the blood sample to be measured. The volume of the blood sample may be quite small, ranging from as small as a few microliters.

Figures 21 shows the preferred embodiment of the substrate of the present invention where two reference electrodes 130A and 130B are present in offline alignment to the alignment of the sensors 120, 116 and 124 and thermistor 138. The axial alignment shown in Figure 21 allows the sensors 116, 120, 124 to be in contact with a sample in a chamber covering their alignment, while the reference electrodes 130A, 130B can be in contact with reference fluid or electrolyte in another chamber placed in fluid contact with the reference electrodes. Any alignment pattern can be used that separates the reference electrode from the sensors in the aforedescribed manner. The other components of the wiring board are as described for the other figures.

Figure 22 shows a broader aspect of the invention where only one sensor 120 is present with one reference electrode 130. If the sensor 120 does not require a reference electrode as in the case of most amperometric electrodes, the reference electrode need not be present.

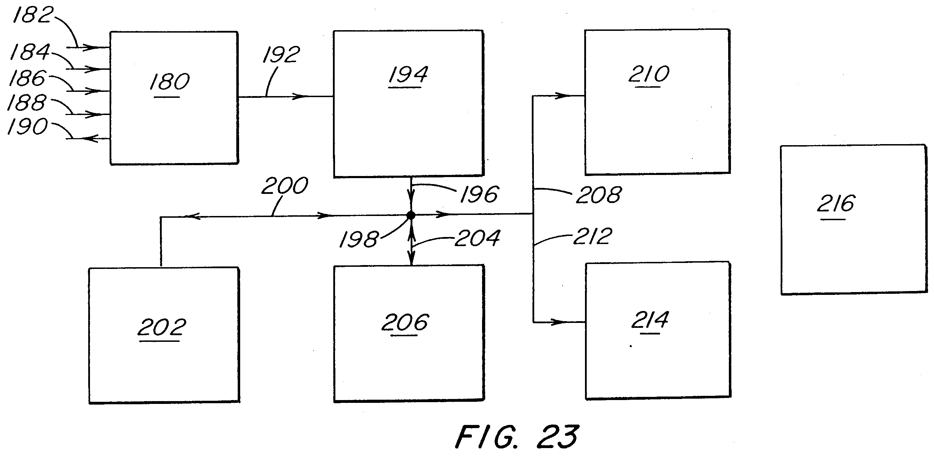

Figure 23 shows the preferred connection with the Analyzer for the electrical wiring board 14. The analog input processing unit of the monitoring means interfaces with the electrical wiring board 14 by electrical connections such as a conducting wire or cable or ribbon cable that allows signals from the one or more sensors and thermistor to be conveyed to the monitoring means. Also the electrical connection allows for electrical current to be supplied to the heater and resistor on board 14 and for any current or voltage that may be needed by the one or more sensors on the board 14. The electrical connections can be separate but are preferably individual connections in a bundle connector or ribbon cable. Connection 182 carries current to the amperometric oxygen sensor 124 of Figure 21. Respectively, connections 184, 186, 188, and 190 carry signal and/or supply current or voltage to: the pH sensor 116, the carbon dioxide sensor 120, the thermistor 138, and heater 174 shown in Figures 19, 20 and 21, respectively. The processing unit 180 is electrically connected by 192 to a 12 bit analog to digital converter 194 which is electrically connected by 196 to line 200 at point 198. Unit 202 is a date/time circuit and battery backup random access memory device. Line 200 serves as a buss of sorts since two way communication occurs between unit 202 and unit 206 which is also connected to line 200 by connection 204 through point 198. Unit 206 is an 8 bit microprocessor. Line 200 also connects to line 208 that is connected to a printing unit 210A and to unit 214A that is a display unit through connection 212. Unit 216 is a battery and charger assembly that provides battery power to the monitoring means. Although a particular arrangement for the functional units of the monitoring means has been specifically set forth, variations are possible that may delete one or more of the functional units. As long as the processing unit 180 and converter unit 194 are present when analog signals are used, and a processor is functionally tied into these units and power is supplied and a read out can be obtained, the monitoring means is usable with the board 14.

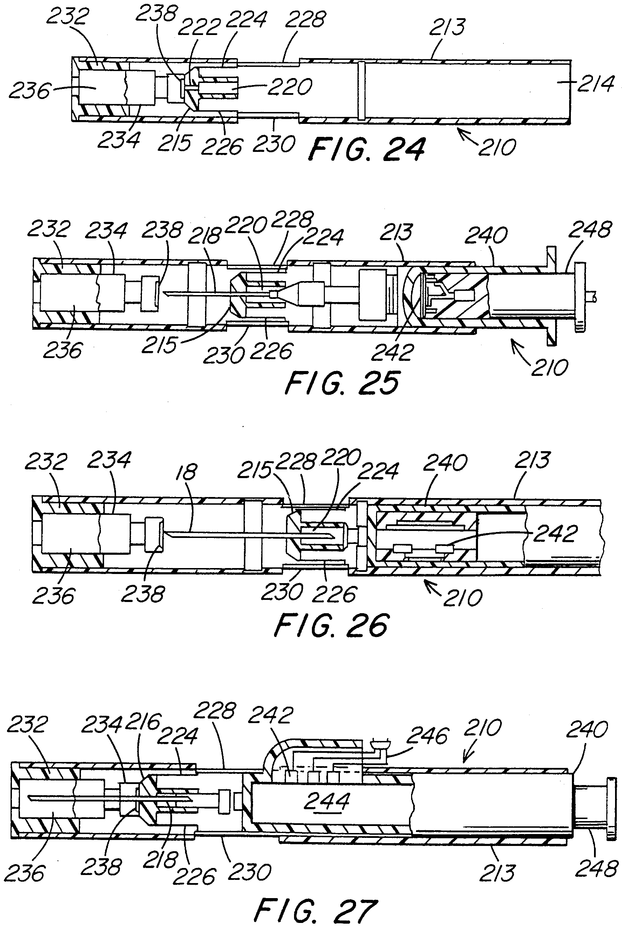

As shown in Figure 24, a calibrator 210 includes a body portion defining a cylinder 213 having an opening 214 at one end for receiving at least a section of the body portion of any type of collector/sensor, as shown in Figure 25 as 240. A movable member 215 that is slidable within cylinder 213 is adapted for receiving a seal puncturing means 218, which, for example, may be in the form of a single or double ended, hollow, rigid stainless steel needle. The receipt of the puncturing means 218 is in column space 220 that extends longitudinally from the movable member 215. The member 215 has hole 222 to fixedly engage the needle 218 which can be provided by the calibrator 210 or with a collector/sensor 240. Also the movable member has one or more and preferably two resilient fingers 224 and 226 which resiliently engage slots 228 and 230 provided in the wall of the calibrator 210 to positionally hold the movable member 215 at the start and end point of its travel within the calibrator 210. The travel of the movable member 215 within the cylinder 213 is limited by one or more inwardly facing projections 232 formed on the interior surface of the cylinder 213.

A sealed container 234 containing calibration solution 236, with characteristics that are described infra is supported in the end of the cylinder 213 of the calibrator 210, opposite the opening receiving a collector 240. The container may be a vial with any puncturable cap 238 known to those skilled in the art sealing the mouth of the container 234. Suitable containers depending on the type of calibration fluid include glass vials with an elastomeric stopper or with an induction seal with a snap-cap and plastic containers with similar or different caps or stoppers.

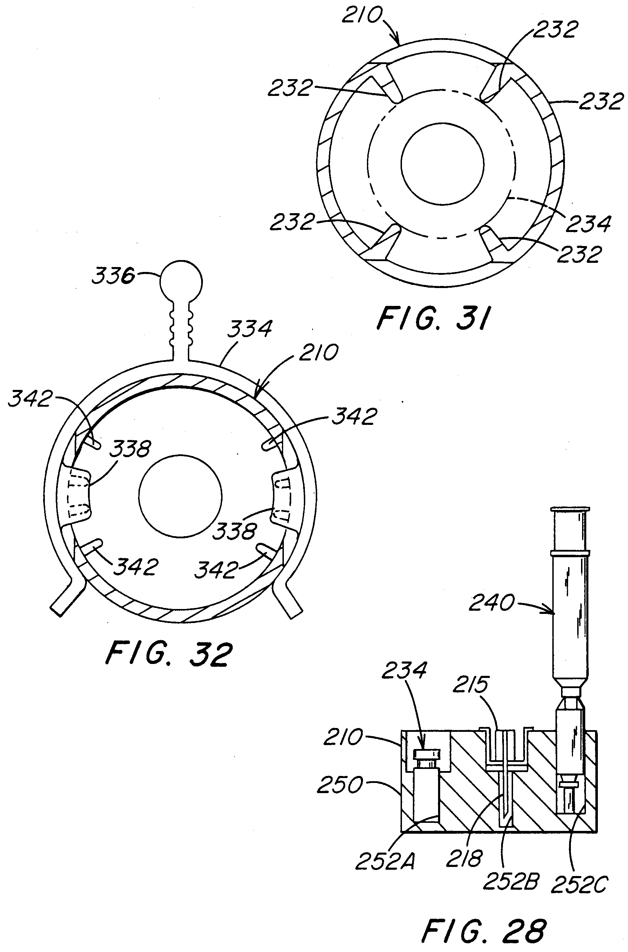

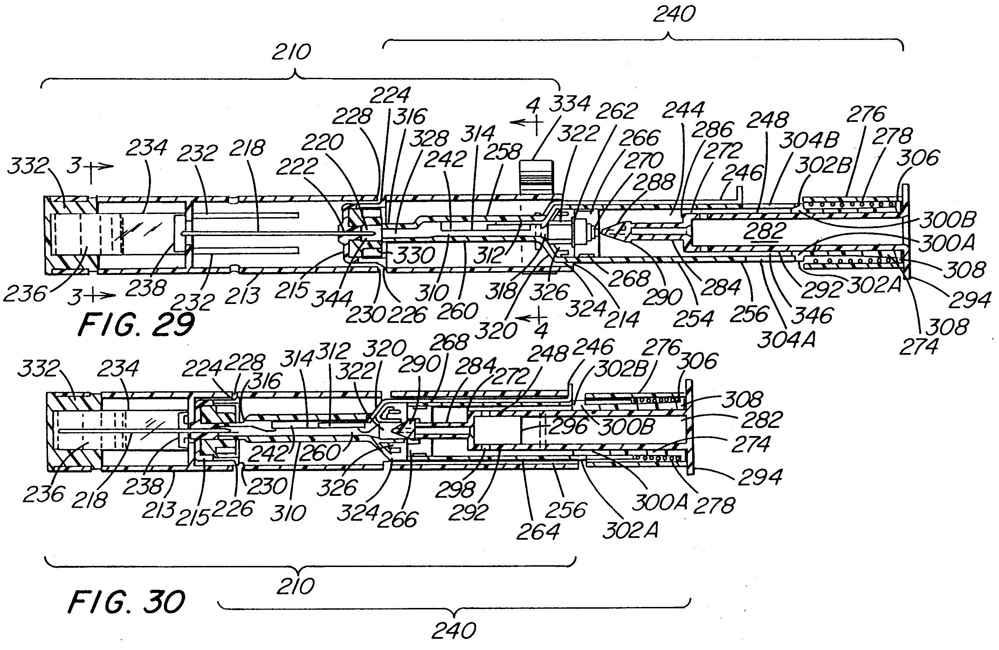

As shown in Figure 25, a collector generally shown as 240 slidably engages the calibrator 210 through opening 214. The collector can be a standard syringe with a needle where the syringe has one or more sensing means 242 positioned in a chamber at the distal end of the collector 240. The chamber is the aforedescribed electrochemical sensor assembly of Figures 1-18. Movable member 215 engages the needle 218 of the collector/sensor 240 as it moves toward the vial 234. The needle 218 of the collector/sensor 240 is directed to the piercable top of vial 234 and pierces the top to be in fluid contact with the calibrant 236 in the vial 234. With the collector/sensor 240 in fluid contact with calibrant 236 the plunger which is activator 248 is retracted to pull the calibrant through needle 218 into chamber 244 to contact the sensing means 242 to calibrate the sensing means as shown in Figure 27. The type of sensing means 242 and the calibration thereof are more fully discussed for Figures 28 and 30.

Figures 26 and 27 show two different types of collector/sensor 240 engaging movable member 215 where the member 215 has the needle 218 for fixedly engaging the collector to act as the needle for the collector/sensor 240. Also these collector/sensor units can be integral units with the at least one sensing means 242 located in the chamber 244 for a collector like a syringe. The sensing means can be located on the side wall of the syringe-like collector 240 so the sensing means can detect the amount of the analyte in the calibrant and send electrical signals by an electrical cable means 246. The calibration, the collector/sensor 240 can be removed from the calibrator 210 by retraction of a reciprocating actuator 248 of the collector 240. The calibrant 236 can be expelled and sample drawn into the collector/sensor 240 or a larger volume of the sample fluid by several orders of magnitude can be drawn into the collector/sensor 240 with the calibrant still present in the chamber 244 of the collector 240. With this latter approach the at least one sensing means 242 would read the values of the analyte in the sample since the calibrant would be so diluted by the sample.

Figure 28 depicts an alternative embodiment of the present invention where the calibrator 210 is a nontubular or noncylindrical device. Here calibrator 210 has a body portion that is a holder 250 having three recesses or wells 252 A, B, and C that extend from a surface of the holder 250 into the holder. These recesses can be parallel or approximately parallel to each other. One recess, for example, 252A holds the calibrant container 234 while another like 252B holds the member 215 and the other 252C holds the collector with the sensing means 240. The recesses can be cylindrical holes in the holder or they can be holes contoured to the matching shape of the object they are to hold. For example, the recess 252C for the collector 240 can have a contour to match and accept the contour of the collector where the direction of the contour can be any direction in the holder 250 just so the collector 240 is adequately held be the calibrator 210. This type of calibrator 210 can be used by removing the collector 240 and placing it vertically on top the member 215 for attachment. The member 215 is removed from recess 252B as part of the attached combination of the member 215 and the collector 240. This combination is placed vertically of the container 234 in recess 252A and the piercing member 218 of member 215 is used to at least puncture the seal or cap 238 of the container 234. With the retraction of a reciprocating actuator 248 of the collector 240 the calibrant fluid 236 is drawn to contact the sensing means 242 and into the chamber 244. Any of the collectors 240 depicted in Figures 24-29 can be used with this type of calibrator.

Referring now to Figures 29 and 30 depicting the preferred embodiment of the present invention, there is shown a fluid sample collection and detection device 240 received in a calibrator 210 in different stages of relative insertion. Device 240 is a sensor/collector that is hereinafter referred to as the "sensor/collector", and the calibration device is hereinafter referred to as the "calibrator". Figure 29 shows the preferred embodiment of the present invention where the collector 240 is a syringe, and where the sensor/collector/calibrator 240/213 are as they might be in their prepackaged stage of insertion (packaging not shown). Figure 30 shows the preferred embodiment at the stage of insertion of the sensor/collector 240 into the calibrator 210 just before the withdrawal of the calibration solution 236 from its container 234, all as will be further described below.

The sensor/collector 240 has a body portion 254 comprised of a collector portion 256 and a sensor portion 258, which is the preferred embodiment of the aforedescribed electrochemical sensor assembly of Figures 1-18. The collector 256 has a first fluid communicating chamber 244 and the sensor portion 258 has a second fluid communicating chamber 260 that is channel 12 of Figures 1 and 2. Other than for a first fluid seal 262 therebetween, that is seal 28 of Figures 1 and 2, the two chambers are juxtaposed for fluid communication between these chambers. The body portion 254 may be made from a suitable plastic material such as a suitable clear styrene plastic.

The first chamber 244 defines a cylinder 264 with a piston 266 slidable therein. The piston 266 is resilient and may be made of plastic or elastomeric material, as for example clear polystyrene or polycarbonate. The piston 266 and the cylinder 264 are circular in cross section and the piston 266 is positioned on a seat 268 provided at one end of the first chamber 244 nearest the second chamber 260. The piston 266 is provided with a centrally disposed, axially extending aperture 270 that has an expanded diameter after an initial narrower diameter to form lip 272.

The piston 266 is actuated by a reciprocating actuator 248 which includes a actuating means 274 reciprocally mounted in a spring biased sleeve 276. The sleeve 276, which may be made of polystyrene or like material known to those skilled in the art, is slidably mounted on the syringe body portion 254.

A spring 278, which may be in the form of a stainless steel helical spring, is disposed in an annular groove 280 in the sleeve 276 and provides a biasing force counter to the slidable movement of the sleeve 276 and its associated actuator toward the piston 266. As an alternative to the spring, the reciprocal actuator can be hand operated as indicated in Figures 25 and 27, to a mechanical stop or operated pneumatically or with a stepper motor.

The reciprocal actuator 248, which can be a hollow member defining a cavity 282 therein, is provided with a first shaft portion 284 terminating at one end in a raised portion 286 and at the other end in the skirt portion 288 of a first puncturing means, in the form of a puncturing head 290, formed on the end thereof. The reciprocal actuator 248 is further provided with a second shaft portion 292 terminating at one end in a generally planar, annular grasping means 294 and at the other end in the raised portion 286. When the reciprocal actuator 248 allows for venting action, a porous absorbent and expandable material 296, such as white porous polyethylene, can be disposed in the cavity 282 to occlude and retard the venting action upon contact of material 296 with fluid from the chamber 244 that may enter the cavity 282 through aperture 298 provided in the wall of the second shaft portion 292 during operation of the collector 240 and calibrator 210.

As best seen in Figure 29, the biasing force of the spring maintains a clearance between the seal 262 and the puncturing head 290 of the reciprocal actuator 248 when the collector 240 and calibrator 210 are in their prepackaged stage of insertion. Further, the grasping member 294 of the reciprocal actuator 248 abuts the proximate end of the sleeve 276 and maintains the position of the reciprocal actuator 248 with respect to the spring biased sleeve 276 until overridden by pushing the grasping member 294 and moving the reciprocal actuator 248 into the cylinder 264. The spring biased sleeve 276 is guided, for movement with respect to the collector body portion 254, through resilient fingers 300A and 300B at the end of actuating means 274 that projects into the cylinder 264. These fingers extend in a direction in a parallel plane to the longitudinal axis of cylinder 264 and the walls of cylinder 264. The fingers end in projections 302A and 302B, respectively, that extend outward toward to the wall of cylinder 264. These projections travel in matching longitudinally extending slots 304A and 304B in the wall of the cylinder 264. These slots have a length sufficient to stop the movement of the actuating means 274 at a particular location in the cylinder 264. This distance is that which when translated to the reciprocal actuator 248 through the spring 278 riding on means 274 that connects to sleeve 276 at its proximate end to permit the puncturing head 290 to perforate seal 262 and engage piston 266. The reciprocal actuator 248 can have a range of sliding longitudinal movement within the cylinder 264 before head 290 contacts the piston 264 and before stopping at the proximate end of the cylinder 264. The reciprocal actuator 248 has annular lip 272 at the distal end of second shaft portion 292 to retard the movement of the actuator 248 out of the cylinder 264 at its proximate end. The movement of actuating means 274 out of the cylinder 264 is retarded by projections 302A and 302B of flexible fingers 300A and 300B stopping at the proximate end of longitudinal slots 304A and 304B, respectively. This limitation of movement is translated to the reciprocal actuator 248 by lip 272 abutting the distal end of the actuating means 274 in cylinder 264. The actuating means 274 is attached by one or more and preferably two fastening means 306 at its proximate end to sleeve 276 at matching fastening means 308. These pairs of fastening means also serve as the proximate boundary for spring 278 within the means 274 and sleeve 276 arrangement. The distal boundary for spring 278 is established by the projections 302A and 302B.

The piston 266 is slidably restrained on the shaft portion 284, by and between the skirt portion 288 and the raised portion 286 for lost motion between the piston 266 and the reciprocal actuator 248, whereby the piston 266 will remain stationary on the shaft portion 284 when not engaged by the skirt portion 288 or the raised portion 286.

The sensing means 242 or the one or more sensors 18 of Figure 1 and preferably the sensor element 14 of Figure 21 is located in sensor portion or electrochemical sensor assembly 258 (also shown in Figures 1-18). Elements of Figures 24-29 that are like those of previous figures have the same reference number as those elements in the previous figures but with the letter I following the reference number for the sake of clarity. The housing 310 (10 in Figures 1 and 2) of the electrochemical sensor assembly also provides two openings for channel 12 of Figures 1 and 2, where one can be an inlet at connecting conduit 36I and one can be an outlet at the conical expansion 38I. One of these openings is for fluid communication with the first fluid chamber 244 of collector 240 while the other is for fluid communication with the calibrator 210. The conical expansion 36I allow for connection or coupling to a device to provide fluid pressure or suction to cause the fluid with the analyte to pass in measuring contact with the one or more sensors 18I. Most preferably, the conical shapes of 36I and 38I are suitable for Leur-Lok attachment to a sample gathering means not shown in Figure 29 such as a needle for a syringe.

The openings of channel 12I as shown in Figures 29 and 30 are preferably aligned in the same plane and along the same axis at opposite ends of channel. This arrangement provides sufficient support of the channel 12I by the housing to receive and/or expel fluid through the channel 12I with reciprocal movement from the piston 266 on the reciprocal actuator 248 of the collector 240.

The electrochemical sensor assembly may be formed integral with the syringe body portion 254 or may and preferably does fixedly attach to the collector body portion 254 by means of interlocking fingers 324 provided on the cup-like end 326 of the collector body portion 254. The fingers each engage a complementary groove provided on the interior surface of the flared end 320 of housing 310. In addition or in lieu of the fingers, the housing 310 and body portion 254 may be adhesively connected. The electrochemical sensor assembly has the seals 26I and 28I to maintain the storage fluid in channel 12I. Preferably, this sealing is at the inlet and outlet openings of the channel 12I. Preferably, a storage fluid 22I is sealed in the channel 12I by seal 28I between channel 12I and 244 and seal 28I at conical tip 36I. Seal 28I preferably is located in channel 12I after any sensors but before the end of channel 12I that is close to the collector 240. At the distal end of the sensor assembly 258 from the connection with the collector portion 254, the assembly 258 terminates in a connecting conduit 36I centrally disposed in a hub 322, and this connecting conduit 36I is preferably fluidly sealed by the second fluid seal 26I at or near the end of channel 12I.

The calibrator 210 is provided for calibrating the electrode assembly 258 to insure that the measurement of the analytes is accurate. Basically, the calibration of the three sensors of sensor element 14I involves contacting the sensors with a solution of a predetermined analyte value like electrolytes, pH, pO2 and pCO2 values. The outputs of the sensors are measured and calibration coefficients are measured to use with software algorithms, all of which can be conventional and known in the art, for example, as described in U.S. Patent No. 4,734,184.

The calibrator 210 includes a body portion defining a cylinder 213 open at one end for receiving at least a portion of the sensor assembly 258 and/or collector body portion 254. A movable member 215, carrying a second seal puncturing means, which may be in the form of a double ended, hollow, stainless steel needle 218, is slidably received in the cylinder 213. The movable member 215 is provided at least one but preferably two resilient fingers 224 and 226 which resiliently engage slots provided in the wall of the calibrator 210 to positionally hold the movable member 215 at the start and end point of its travel within the calibrator 210. The travel of the movable member 215 within the cylinder 213 is limited by inwardly facing projections 232 formed on the interior surface of the cylinder 213.

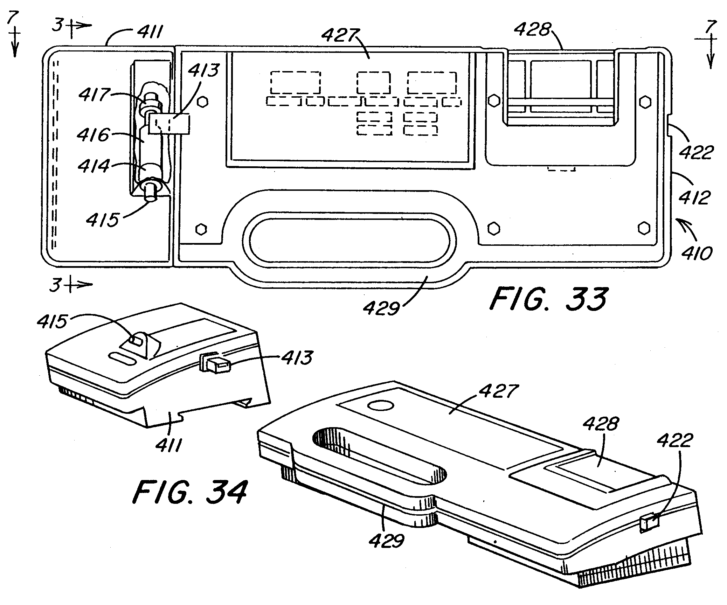

A sealed container 234 containing calibration solution is supported in the end of the cylinder 213 of the calibrator 210, opposite the opening for receiving the sensor collector unit 240 in a similar manner as shown for Figures 24-27. If container 234 is plastic when the calibration fluid is used as a standard for the analysis of gases in a fluid, the plastic container can be coated with a suitable barrier coating to essentially eliminate any O₂ or CO₂ permeability of the plastic container to retain the integrity of the calibration solution. The container 234 preferably is seated and centrally received in a cup member 332 that is centrally aligned in the calibrator 210 by a plurality of inwardly facing projections 232 formed on the interior surface of the cylinder 213. The cup member 332 may be made from a suitable plastic material and is sealingly received by the cylinder 213 of calibrator 210. This arrangement is shown in a cross-sectional view in Figure 31.

The calibration fluid 236 contained in container 234 and the storage fluid 22I contained in the second fluid communicating chamber or channel 12I and possibly in the connecting conduit 215 to be in fluid communication with the one or more sensors can be an analyte-containing fluid as aforementioned. Alternatively, the storage fluid 22I can be and preferably is an activating fluid. An activating fluid can equilibrate with the one or more sensors that are in an active state in the sensor assembly in order to precondition an active sensor. The analyte-containing fluid can be a gas, liquid or combination of a gas and liquid depending on the state of the analyte that is to be detected by the sensor. For a nonexclusive example, when the analyte is a blood gas such as oxygen and/or carbon dioxide, the storage fluid as an analyte-containing fluid 22I can be a gas. One or more of these gases alone or in combination with each other or with inert gases can purge the second fluid communicating chamber or channel 12I after the sensor assembly 258 has one seal in or over an opening of the channel 12I. After the purging, the second seal is sealed over the unsealed opening of the channel 12I. When the analyte-containing fluid is a combination of gas and a liquid, such a fluid can be produced with the requisite quantity of the gas by any method known to those skilled in the art. For example, such a fluid can be a tonometered fluid produced by any of the commercially available tonometers like the one available from Instrumentation Laboratory under the designation IL237 or by any method known to those skilled in the art like the techniques shown in preparing tonometered buffered solution or whole blood described in the article entitled "Quality Control in Blood pH and Gas Analysis by Use of a Tonometered Bicarbonate Solution and Duplicate Blood Analysis in Clinical Chemistry", Vol. 27, No. 210, 1981, pages 1761-1763. For such fluids the liquid can be an aqueous solution that is buffered and contains oxygen and carbon dioxide for use in blood gas measurements. Such solutions can be prepared in accordance with U.S. Patent 3,681,255.

An example of an equilibrated or tonometered fluid as fluid can result from contact of the buffered liquid solution with the carbon dioxide containing gas which can include a mixture of carbon dioxide with one or more inert gases. An inert gas is one which does not react with the buffer solution to change the pH. This would destroy the predictability of a final pH value. Also, inert gas is one that does not react with any of the ingredients in the calibration fluid 236 or storage fluid 22I. Nonexclusive examples of inert gases are nitrogen, argon and other similar gases normally found in the air. This includes the noble gases such as neon, argon, krypton, xenon, helium and the like. It is preferred to use as the equilibrating gases for blood gas analysis a mixture of carbon dioxide and nitrogen or carbon dioxide with oxygen and nitrogen. Two nonexclusive examples include: 1) around 5 percent carbon dioxide with oxygen making up the balance of the gas in the fluid, and 2) around 7 volume percent carbon dioxide and around 10 volume percent oxygen and the balance is nitrogen.

For storage fluid 22I that is activating fluid for thick film sensors with one or more hydratable membranes, it is most preferred that this fluid is a hydrating fluid 22I which is chiefly an aqueous fluid with an effective composition to hydrate at least to a partial degree but better to a substantial degree the hydrophilic polymeric membranes. Some sensors with hydratable membranes may be stored dry but they need the hydration of their membranes for the existence of an active state. When the sensor assembly 258 has at least one such sensor, the storage fluid 22I is the activating fluid. Storage fluid 22I is in fluid contact with the sensor by the sealed presence of the fluid in channel 12I. When fluid 22I is hydrating fluid, which it preferably is, any liquid is suitable that can maintain the membrane(s) of the one or more sensors in a non-dried state. For instance, the liquid will have some amount of water although a minor quantity of organic liquids may also be present. Preferably, the liquid is a stable liquid for storage ranging from a short time (days or weeks) to prolonged periods of time of several months. Preferably, the liquid is an aqueous solution that is isotonic with any electrolyte in the one or more sensors. More preferably, the fluid 22I as a hydrating fluid is also isotonic to act as the electrolyte for any reference electrodes that may be present in the sensor assembly 258. A suitable example of a hydrating fluid is an aqueous solution of salts. The quantity of hydrating fluid 22I in channel 12I or any plurality of channels associated with channel 12I is at least that which is sufficient to cover or remain in contact with the one or more sensors. Generally, the storage fluid 22I usually does but may not completely fill the channel 12I of the housing. A nonexclusive example of a suitable process for placing the requisite quantity of analyte-containing or activating fluid 22I in contact with the sensor assembly 258 was previously described for the electrochemical sensor assembly of Figures 1-18.

The seals 238, 26I and 28I may be different or the same in the form of a rubber, plastic, or metal foil and adhesive seals that are at least impervious to liquids and preferably substantially impervious to gaseous fluids. For measurement of gaseous analytes the seals are preferably substantially impervious to gas. Although seal 238 can be any of the aforementioned materials for seals 26I and 28I, a suitable example for seal 238 is a paper-backed aluminum foil coated with a clear heater RF (radio frequency) sealable coating. The coating is preferably a blend of a high molecular weight ethylene and vinyl acetate copolymer, available under the trade designation "SANCAP" available from Sancap, 215(16)1 Armor Street NE, Alliance, Ohio 44601 for seal 238. The sealing process can be similar for all three seals.

The calibrator 210 may also be provided with clamping means 334 which may be in the form of a substantially C-shaped clamp which clampingly engages at least portion of the exterior surface of the calibrator 210. As shown in Figure 32, the clamp 334 includes a outwardly grasping means 336 outwardly projecting from substantially C-shaped portion of the clamp 334 for grasping and positionally holding the calibrator 210 by the operator. The clamp 334 also includes a plurality of inwardly facing projections 338 formed on the interior surface of the substantially C-shaped portion of the clamp 334 and in registration with a plurality of slots 340 provided in the wall of the calibrator 210 for centrally aligning the sensor collector 240 with respect to said calibrator 210 such that the longitudinal axis of the sensor collector 240 is coincident with the longitudinal axis of the calibrator 210. Additional inwardly facing projections 342 may be formed on the interior surface of the cylinder 213 to further assist in aligning said sensor/syringe 240 with respect to said calibrator 210.

The above description of the sensor/collector 240 and calibrator 210, as before mentioned, is the stage of relative insertion during the prepackaged state. When this assembly is removed from its package, it will be connected to the associated monitoring device by means of the connecting cable 246. At this point the stages that will now occur can be described as: (i) puncture the several seals 238, 26I and 28I; (ii) aspirate the calibration solution from the container 234; (iii) remove the calibrator 210 from the sensor/syringe 240; (iv) attach a hypodermic needle or catheter, and (v) draw blood. Figures 29 and 30 illustrate steps (i) and (ii) and the remaining steps require no further illustrations.

As can be best seen in Figure 30, the sensor/syringe 240 and the calibrator 210 are progressively moved, one relative to the other, the puncturing of the first fluid seal 28I, the second fluid seal 26I and the seal 238 of the sealed container 276 containing calibration solution occurs. Additionally the container 234 can have a cap 239 covering the seal 238, and the cap can be metal like aluminum or a plastic snap-cap.

To initiate this action, the sleeve 276 and the actuator 274 are moved as a unit by the operator toward the piston 266 until the puncturing head 290 is urged into and through the aperture 270 in the piston 266. The piston is now captured on the reciprocal actuator 248, for movement therewith, by the skirt portion 288 thereof. Concurrently with this movement, the sensor/syringe 240 is progressively moved relative to the calibrator 210 and the hub portion 38I, projecting from the syringe body portion 254, is received in centrally disposed cavity 344 in the movable member 215. A portion of the needle 218 punctures the second fluid seal 26I provided at the end of the channel 12I. The so engaged movable member 215, carrying the needle 218, is urged toward the container 234 by the movement of the sensor/collector 240 until the other end of the double-ended needle 218 punctures the seal 238 of the container 234.

At this point in the operation of the assembly, there is fluid communication from the container 234 through the needle 218, (conduit 316) channel 12I, as the second fluid chamber 260, and the first fluid chamber 244 and the calibration solution may be withdrawn from the container 276.

The above described movement of the sleeve 276, to engage the piston 266, compressed spring 278 and now the operator merely releases the sleeve 276 and the energy stored in the spring 278 will return the sleeve 276, the actuating means 274 and reciprocal actuator 248 to their original position. Such movement of the actuator 248 will move the captured piston 266 a first incremental distance in the first fluid chamber 244 to aspirate or withdraw the calibration solution from the container 234 into the second fluid chamber 260 and into intimate contact with the one or more sensors 18I. After the calibration of the one or more sensors 18I is achieved, the operator removes the sensor/syringe 240 from the calibrator 210 and attaches a hypodermic needle or a catheter (not shown) to the sensor/syringe 240 at its hub 216 for drawing the blood sample.

To take the blood sample, the operator then grasps the generally planar, annular grasping portion 294 provided at the end of the actuator 248 and pulls the actuator 248 from the sleeve 276 with enough force to dislodge the abutment of an annular rib 346 that may be located on the second shaft portion 292 with the end of the actuating means 274. This movement of the actuator 248 moves the captured piston 266 a second incremental distance in the first fluid chamber 244 to withdraw the blood sample from the patient into the second fluid chamber 260 and into intimate contact with the one or more sensors 18I for analysis. After the procedure is completed, the operator may appropriately dispose of the sensor/syringe 240 and calibrator 210.

The above-described lost motion relationship of the piston 266 with respect to the actuator 248 also reduces the possibility of any loss of fluid from the sensor/syringe 240 during use or disposal. As can be seen from the above description, the piston 266 should move in one direction only, namely, in a withdrawal mode and all the withdrawn calibration solution and blood sample remains in the syringe portion of 240. If the actuator 248 were inadvertently moved forward as if to expel fluid from the sensor/syringe 240, the actuator 248 would slidably move within the piston 266 at its first shaft portion 284 and the forward motion of the actuator 248 would not be transmitted to the piston 266.

Figure 33 shows the cartridge 411 and Analyzer 412 of the multiple use analyte measuring device 410. The cartridge 411 is a multiple use disposable unit which is electrically connected to the Analyzer 412 through electrical connection 413, which acts as a signal conveyor. The cartridge 411 as shown in Figure 33 shows the inlet 414 for placement of chemical fluids for measurement, preferably through injection, at 414, which has an associated cap 415 which is removably attached thereto. In the cutaway of the cartridge in Figure 1, there is shown the electrochemical sensor assembly of Figures 1-18 as a flow cell 416 having a uni-directional inlet 414 at the end proximate to cap 415 and uni-directional valve 417 at the distal end of the assembly 416. Electrical connection 413 can be a prong connector or a cable as shown in Figures 28-30 and those as known to those skilled in the art. If the cartridge 411 is not mechanically attached to Analyzer 412, the electrical connection 413 is a cable such as a ribbon cable for electronic communication between the assembly 416 and the Analyzer 412. In this manner, the analyte measuring device of the present invention is portable and has a sample receiving means, the cartridge 411, which is detachable from the Analyzer 412 and is capable of a multitude of tests after which it is disposable.

The cartridge 411 as shown in Figure 34 is detached from the Analyzer 412 giving a clear view of the electrical connector 413 as a prong connector. The cartridge 411 can have any shape to serve as a housing for the flow cell with its one or more sensors. Hereinafter, in the specification, the terms "one or more sensors" and "at least one sensor" will be referred to in the plural to include both the singular and the plural. The shape can be designed to match that of the Analyzer 412 or could be a cube or rectangular box with a ribbon cable attachment as electrical connector 413 to the Analyzer 412. The Analyzer 412 shown in Figure 34 has the slide-through encoded information reader 422, which is electrically connected as aforementioned to the microprocessor within the Analyzer 412. On the exterior, the Analyzer 412 has display area 427 for display of numerical values and functions performed by Analyzer 412. Also, the analyzer 412 can have a printer for a hard copy display of information electrically displayed in display area 427. The printer 428 can be any printer known to those skilled in the art that is small and compact. The display area 427 can consist of any set of LCD displays or the like known to those skilled in the art arranged for a convenient display of numerical values and functions performed by the Analyzer 412. The Analyzer 412 also can have handle 429 to aid in its portability.

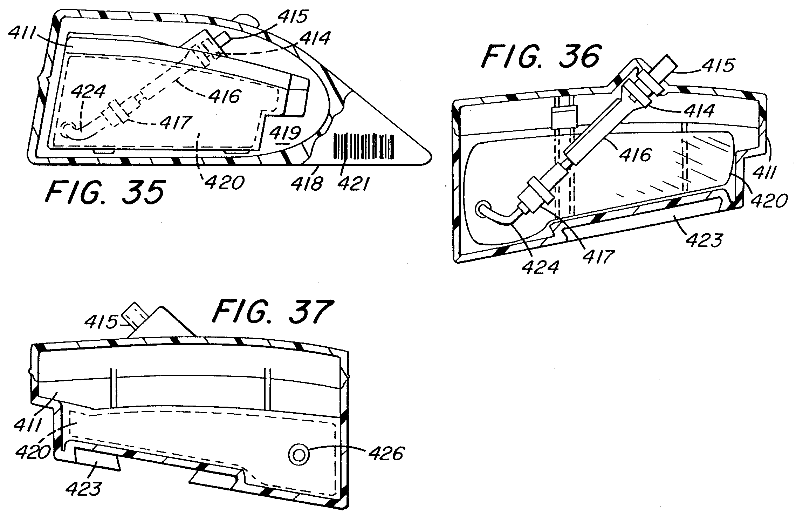

Figure 35 shows cartridge 411 inside a gas impermeable bag 418 to maintain the electrochemical sensor assembly 416 and the sensors therein within a certain atmosphere 419. Cartridge 411 is shown in a side view along the lines 3-3 of Figures 33 and 34. The cartridge has removable cap 415, which is preferably reusable, covering the uni-directional inlet 414 in fluid engagement with the sensor assembly 416 having at its distal end the uni-directional valve, 417, for fluid engagement with waste reservoir 420 as shown in Figures 36 and 37. Also, the cartridge 411 has an encoded information carrier 421 associated with it to carry information related to sensor values and information on any temperature sensor that can be present in the sensor assembly 416. The sensor values would include the performance characteristics including sensitivities of one or more of the electrodes for one or more of the analytes to be measured and different slopes for the function of the change in electronic values for changes in concentration of a particular analyte.

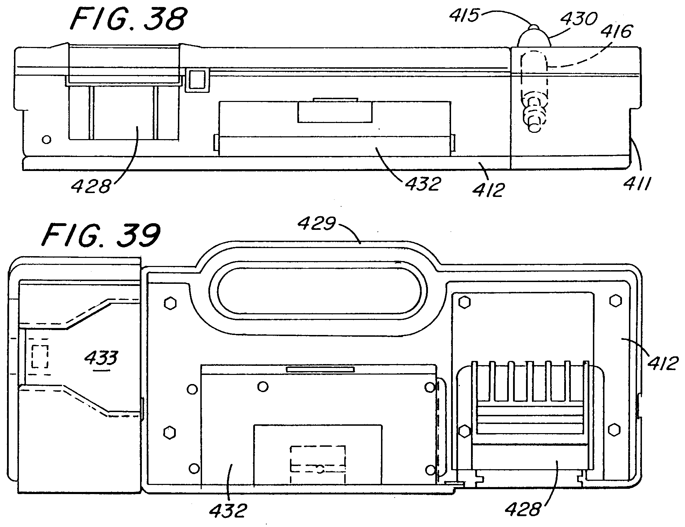

As shown in Figure 35, the orientation of the sensor assembly 416 is preferably a straight line orientation with the one-way inlet 414 and the one-way valve 417. Preferably, the orientation of the sensor assembly 416 is non-horizontal with respect to the horizontal plane passing through the width axis of the cartridge 411. Preferably, sensor assembly 416 has this orientation because of the arrangement of the electrodes with their electrolyte in the sensor assembly as more fully discussed for Figure 38, and for good flow through the channel of the sensor assembly 416. The latter allows for facile flushing of a previous sample from the multiple use sensor assembly before measurement of a subsequent chemical sample. The orientation of the sensor assembly 416 within cartridge 411 can be even horizontal with appropriate modifications of the electrodes in sensor assembly 416. For instance, the reference electrolyte for the reference electrode could be a gelled electrolyte.

The encoded information carrier 421 can be any means for containing information that can be inputted into the Analyzer 412. A few nonexclusive examples of encoded information carrier include bar codes, magnetic stripes, optical encoding, keyboarding, semi-conductor electronic memory, electromechanical punch cards, and touch memory chips. The latter are stainless steel, self-stick labels that read or write with a momentary contact. The touch memory chip is packaged in a coin-shaped MicroCan holder to withstand harsh environments. The simple conductive surfaces of its package are the conduit for error-free data transfer to other chips in the system in a direct chip to chip digital link. Preferably, the encoded information carrier 421 can be and is a bar code which contains information by having alternate elongated dark bars and light spaces to represent digital and alpha numeric codes. For example, the universal product code (UPC) is commonly used on retail products. The Analyzer 412 has a reader 422 for encoded information. For instance, when the information has a bar code, the bar code 421 can be on the bag or foil wrapper 418 which is passed through the reader or the reader passed over the bar code. Additionally, the encoded information carrier can be a sticker on the cartridge 411 or any other means so long as the information for the sensor can be inputted into the Analyzer 412.

A nonexclusive example of a bar code reader includes a wand having a light emitting diode shining through an aperture at one end of the wand. The wand can be held vertical relative to the bar code label 421 and is past directly from one end of the bar code label to the other. This is referred to as "scanning of the label". A photo-sensitive device, such as a photo-transistor, receives light that is emitted from the light emitting diode and is reflected by the light spaces between the dark bars in the label. The wand typically includes a single stage amplifier that amplifies the output signal produced by the photo-sensitive device. The signal referred to as the wand signal or analog signal typically has an amplitude of roughly 200 to 300 millivolts. Where the reader 422 is a wand-type reader, these signals could be conducted by means of a flexible cable extending from the upper end of the wand. The signal would go to wand conditioning circuitry that further amplifies and shapes the wand signal to produce a raw data signal that includes a sequence of pulses and intervals therebetween with a constant scan velocity of the widths of the pulses and intervals accurately correspond to the widths of the bars and spaces. The signal produced by the wand conditioning circuitry is inputted into a microprocessor system to execute algorithms for converting the raw data signal into binary numbers with one such binary number corresponding to each character in the bar code and each bit having a logical state (either a 1 or a 0) that corresponds to the width of the bar or space of the bar code label. Once the binary numbers are obtained, the characters represented thereby automatically can be obtained by the microprocessor with reference to its data information bank. Any bar code, information source and method for decoding same known to those skilled in the art can be used. Preferably, the bar code reader 422 is a stationary attachment on the Analyzer 412.

Figure 36 shows a sectional view through cartridge 411, where the section is taken in front of sensor assembly 416. The cartridge preferably has a recess 423 for mechanical attachment to Analyzer 412. Also, this view shows the inside of plug connector 413 for electrical attachment to the Analyzer 412. The cap on one-way inlet 414 is removable for multiple uses of the inlet. The inlet 414 is preferably adapted to receive a needle or a front end Leur fitting section of a syringe with the needle removed for injection of a fluid, preferably blood, for analysis of the analytes, preferably blood gases. Preferably, the inlet 414 includes a unidirection valve like a check valve to make the inlet unidirectional for flow into the sensor assembly 416. The sensor assembly 416 is in fluid communication with the inlet 414 and is connected at its distal end to one-way valve 417. Valve 417 has conduit 424 for connection with a waste reservoir 420.

Figure 37 is a sectional view of the other side of cartridge 411 from the side depicted in Figure 36 so that the sensor assembly 416 is shown in Figure 37 from a sectional view of Figure 35. The cartridge 411 has waste reservoir 420 which is preferably a biax nylon laminated to clear polyethylene bag having a continuous inner and outer seal. Preferably, the bag is double-edged and is bulk packed in cartridge 411 to be expandable. Looking through the clear bag 420, the reservoir port 426 is in view which is in fluid connection with conduit 424 to receive fluid from sensor assembly 416 through valve 417.

The electrochemical sensor assembly of Figures 1-18 is used with the cartridge 411 of Figures 33-37, As shown in Figures 3, 8 and 18, this is accomplished in the preferred embodiment by a having a plurality of channels. In Figure 18 the one channel, 12, is for the flow of fluids through the sensor assembly, and the additional channel or channels are for the reference electrode containment area, depicted by channels 60 and 62. Preferably, this area is formed by one additional channel for each reference electrode. Preferably, there are two reference electrodes in spaced apart relation to each other as shown at 64 and 66. The containment space can have the at least one reference electrode and its reference electrolyte in fluid contact therewith. The reference electrolyte is hydrating fluid 22, which can be similar to that which can occupy channel 12. Once again, the sensor board is 14 of Figure 3 and 110 of Figure 21. There is fluid contact between the fluid 22 in the channels 12 and the sensors 18 located along that particular channel 12. The reference electrode can be any reference electrode known to those skilled in the art, but preferably it is a bare wire or tracing of the electric circuitry as shown at 116 and 120 of Figure 19. The reference electrode containment area has sufficient electrolyte to be effective for the number of tests in the multiple test unit. Preferably, the area is in liquid junction communication with channel 436. The geometric design of the area can be any design that keeps the electrolyte in contact with the one or more reference electrodes during the number of multiple use tests, which can range from more than 1 up to around 75 or more separate tests. For instance, the design can be one that allows for use of the flow cell in any orientation in the cartridge 411 when the electrolyte is a gel. The sensor assembly of Figure 18 can have an angle that is an acute or obtuse angle in respect to this plane, as when the design of the area involves a channel for each reference electrode that are coplanar with channel 12. Preferably, this or these reference channels run parallel to channel 12 and intersect with channel 12 at the proximate end of the sensor assembly relative to the inlet 414.

Upon joining of the housing members to contain the sensor element 437 and cable 413, one opening of the channel 436 is coupled with a one-way check valve 414 or 417 and the hydrating fluid 434 is added to channel 436 and the reference containment area 443 to fill substantially all of the channels although small amounts of air bubbles can be tolerated in the channels but preferably the channels are filled to capacity. The remaining opening of channel 436 is coupled to a second one-way check valve and the sensor assembly is ready for positioning in cartridge 411 with the waste reservoir 420.
* Figure 38 is a view of the back of the cartridge 411 and Analyzer 412 in a view that is a 180 degree rotation about the elevational axis of the device shown in Figure 33. This view shows the cartridge housing 411 having a raised area 430 which encompasses the one-way inlet 414 that is attached to sensor assembly 416. The printer 428 is shown also in Figure 38 and area 432 is a battery supply and AC connection. It is preferred that the unit have both a DC supply and an AC connection although it is possible that the unit have just one or the other.

Figure 39 is the bottom of the Analyzer 412 with attached cartridge 411 of Figure 33 flipped 180 degrees about the longitudinal axis. This view shows the mechanical attachment 433 which forms a base plate of the cartridge 411 and Analyzer 412. The mechanical fastener 433 of the Analyzer 412 engages cartridge 411 through recess 423. The mechanical fastener can be any protruding element capable of attaching to and supporting a box-like cartridge which can develop a not inconsiderable weight when the waste reservoir 420 is full of fluid. Also shown in Figure 39 is the bottom of the printer 428, the handle 429 and the battery or power section 432. When the cartridge 411 has a full waste reservoir, the cartridge is removed from the mechanical fastener 433 of the analyzer 412 and discarded and a new cartridge is slidably engaged with mechanical fastener 433 through recess 423 of the cartridge 411 to provide additional tests.

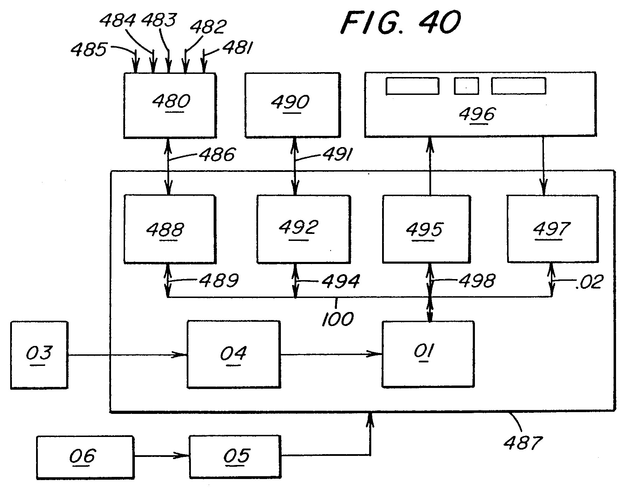

Figure 40 shows the block diagram of the functions and interrelationship of these functions for the Analyzer 412 with its electrical connection to the sensor assembly 416. The analog input processing unit 480 of the Analyzer 412 interfaces with the electric circuitry 450 by the electrical connector 413 to allow signals from the sensors 438 and any temperature detector 461 of previous Figures. Also the electrical connection allows for electrical current to be supplied to any heater and resistor on sensor printed wiring board and for any current or voltage that may be needed by the sensors on the board. The electrical connections can be separate but are preferably individual connections in a bundle connector or ribbon cable. Connection 481 can carry current to an amperometric oxygen sensor as shown in Figures 19-23. Respectively, connections 482, 483, 484 and 485 can carry signal and/or supply current or voltage to: the sensors, the thermistor, and heater shown in the previous figures. The microprocessing unit 480 can be electrically connected to the microcomputer 487 by 486 to the function of a 12 bit analog to digital converter 488. Converter 488 can be electrically connected by 489 to line 500 a type of buss line. By line 500 connections are made to Unit 501 which has is a date/time circuit and battery backup random access memory device and can be and preferably is an 8 bit central processing unit (CPU) microprocessor 501. In addition, the encoded information reader and drive circuit unit 490 is connected by line 491 to the microprocomputer 487 through Input/Output (I/O) port 492 for two way communication. The CPU is connected through I/O port 495 to the display and keyboard unit 496, and this unit is connected through I/O port 497 and through line 502 to line 500 for communication with the microcomputer 487. The CPU 501 is connected for external communication by RS232 and drive circuit unit 503 through serial port 504. The microcomputer 487 is connected to a power supply 505 and battery pack 506 for power. Although a particular preferred arrangement for the functional units of the Analyzer 412 has been specifically set forth, variations are possible that may delete one or more of the functional units. As long as the processing unit 480 and converter unit 494 are present when analog signals are used, and a processor is functionally tied into these units and power is supplied and a read out can be obtained, the Analyzer 412 is usable with the cartridge 411 having sensor assembly 416 of previous figures. Suitable software resides in the CPU to accomplish these connections and to perform the calibration and analysis of samples.

In the aforedescribed sections of the device of the present invention, the arrangement of the components preferably result in sensors for measuring the partial pressures of oxygen and of carbon dioxide and for measuring pH are in a main channel while one or more reference electrodes are in one or more side channels. When hydrating fluid 34 is in the channels, the fluid in the main channel is more easily displaced with the introduction through inlet 414 of sample fluid to be measured relative to the fluid in the side channels. Therefore, the reference electrodes are measuring as a reference a known fluid for comparison for the measurement of the sample fluid in the main channel.

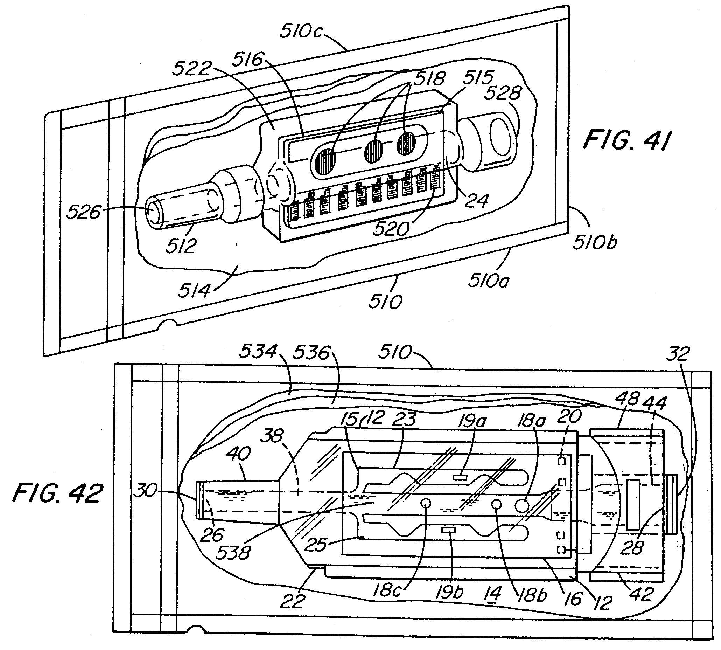

As shown in several figures, 35 and 41-46, the electrochemical sensor assembly in several embodiments can be wrapped in a hermetically sealable layer that is gas impermeable and diffusion tight. As shown in Figure 35, the cartridge 411 with the electrochemical sensor assembly can be placed in the wrapper. Also as show in Figures 41-46 respectively, the wrapper can have the sensor assembly by itself in two embodiments (41-42), the sensor assembly with the calibrator and collector (43), and vials of calibration fluid without and with flush solution for the multi-use embodiment of the invention (44-45).

This layer can be any single layer or multiple layer laminate-type material that can provide the characteristics of gas impermeable and diffusion tightness. Suitable multilayer material includes metal foil polymer laminate material that can be heat-sealed or RF (radio frequency) sealed to form a bag. The laminate material ordinarily has the interior layer of polymeric material and outside this layer a metal foil layer. The thickness of the inner polymeric or plastic layer is generally in the range of about 20 to around 80 microns. A typical laminate can have two or more layers but preferably has an additional outer polymeric layer to facilitate abrasion resistance or printing on top of the metal foil layer. A nonexclusive example of the metal foil is aluminum.

The three layer laminate suitable for the layer for a gas impervious wrapper or bag of Figures 41, 42, 43, 44 and 45 can have from the exterior surface to the interior layer the following: 1) nylon, polyester polyethylene or polypropylene, for example, 10 to 70 grams per meter² thickness for abrasion resistance, 2) aluminum foil, for example, 5 to 40 grams per meter² thickness, and 3) an inner heat sealable polymeric layer such as polyethylene, polypropylene, polyvinylidene chloride or nylon, i.e., of 5 to 25 grams per meter² thickness. A nylon-foil-polypropylene laminate of, i.e., 17 grams per meter² nylon, 32 grams per meter²; aluminum, 45 grams per meter²; polypropylene available under the trade name Sterilite NFP is suitable.

Another suitable example is a laminate having as an outer layer polyvinyl alcohol in an inner layer of a heat sealable polymeric material such as polyethylene, polypropylene, high-density polyethylene, polyester, a laminate consisting of non-stretch polypropylene and biaxially stretched polypropylene and an inner layer of non-stretch polypropylene and nylon as an intermediate layer and biaxially stretched polypropylene as an outer layer. Any of these heat-sealable polymeric films can be used as the inner layer with the polyvinyl alcohol. The hydroxyl groups of the polyvinyl alcohol are bonded with each other through hydrogen bonding providing polyvinyl alcohol with an extremely high impermeability (barrier property) to oxygen gas. The inner layer of the heat sealable polymeric film improves the heat sealability of the polyvinyl alcohol laminate. In addition to retard deleterious effects of moisture or water in the environment on the polyvinyl alcohol layer, the exterior layer over the polyvinyl alcohol layer is a third polymeric layer. Suitable examples for this layer include biaxially stretched polypropylene, polyester, biaxially stretched nylon and polyvinylidene chloride film. A suitable laminate layer of this type is available commercially under the trade designation EVAR from Kuraray, Ltd.

A still further example of a suitable example is a polyfoil-polylaminate which is a three-layer composite having an aluminum foil intermediate layer and an inner and outer layer of polypropylene. In the metal foil laminate layer, the thickness of the aluminum foil is generally in the range of at least 20 µm to about 30 µm. The inner polymeric layer can also be selected from low permeable thermoplast available from ICI Chemicals, polyvinylidene chloride available under the trade designation SARAN, polyacrylonitrile-copolyme available under the trade designation PANC and BARAX 210; polyethylene terephthalate available under the trade designation MYLAR, polyvinylfluoride available under the trade designation PVF and polyamide-6 available under the trade designation NYLON-6 and polyvinyl chloride. The PANC is available from Lonzag, 4002 Basel and is described as a copolymer of a high proportion of acrylonitrile about 72 percent by weight and a low portion of other monomers, that is thermoelastically workable up to a temperature of about 150°C.

In this description and in the accompanying claims, the term "equilibrating" is used in its art-recognized sense to mean that the gas and the buffer solution are maintained in contact with each other until such time as a state of equilibrium has been reached between the analyte in the controlled-content fluid and the analyte in the activating fluid.

The term "active state" for the sensor refers to the condition of the sensor that it is ready to detect analyte although calibration may be needed with conventional reference fluids.

"Preconditioned state" of the sensor refers to providing a sensor that is either in its active state and/or is in a diagnostic state, and/or is in a pseudo or precalibration state and/or is in a calibrated state. The diagnostic state is that where the sensitivity of the sensor can be tested initially on first using the sensor with a display device not shown in the drawings. With this diagnostic information, the sensor can be tested for its ability to function prior to using it in analysis equipment. The pseudo or precalibration state is when the output from a calibrant or reference sample with a predetermined amount of analyte is compared with an output reading on a subsequent calibration or reference sample and a particular range of the change in values is expected for good operability of the sensor. Calibrated state is when the sensor initially gives an output reading on display equipment of some value or values for a calibrant or reference sample with a predetermined amount of one or more analytes.

In Figures 41-43 similar numerals are used throughout the drawings to denote the same feature in each of the drawings.

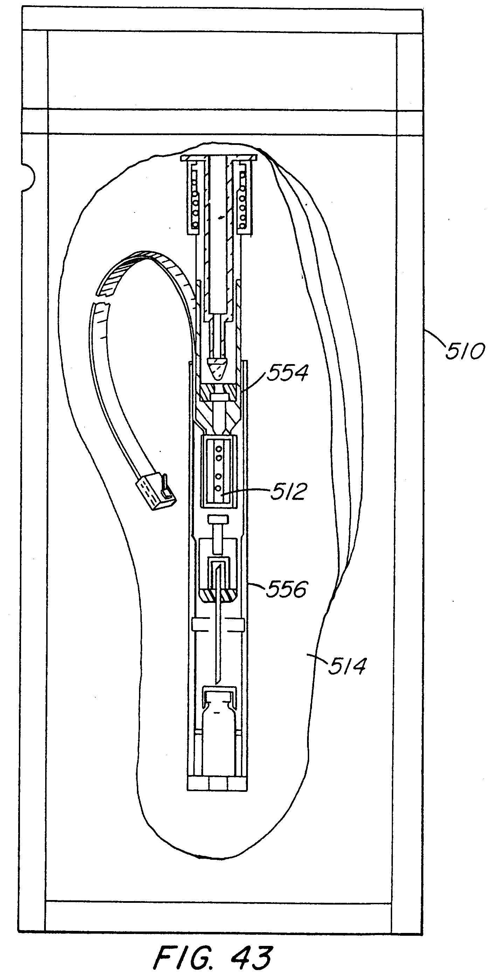

As shown in Figures 35 and 41-45, a bag of the layer is formed by any method known to those skilled in the art such as heat sealing or RF sealing. The size of the bag formed by the layer 510 will vary depending on the contents of the bag. For the sensor assembly of Figures 41-43, the size can range from 4 x 7 cm to 6 x 10 cm to larger dimensions when additional components are included in the bag other than the sensor assembly. For instance, the size of the bag can be from 6 to 15 cm wide to 10 to 20 cm long for Figure 43 and can include a tear strip or tearing notch or line of weakness or the like expedient to facilitate opening. The seals for the bag can be placed along each end of the layer folded on itself or for two layers facing each other with their heat sealable polymeric layers, the inner layer of each edge can be sealed. Heat seals for the material folded on itself would be along two edges and one folded side like 510a, 510b and 510c of Figure 41. For two pieces of material facing each other, three heat seals would be made -- one at each end like 510a through 510c. Typically, the heat seals 510a through 510c can be 9 to 10 mm wide. The heat seals are applied to the layer to allow for one open end of the bag. After the components are added to the bag the opening is heat sealed in the same manner as the other sides.

In Figures 35, 41-45, the preconditioning fluid 514 in the electrochemical sensor assembly 515 can be is an activating fluid to maintain the sensor or sensors in an active state. For example, a sensor may have a water vapor permeable polymeric membrane like a hydratable polymeric membrane with some portion of its electrolyte that is aqueous. In this situation, the sensor needs to be maintained in a hydrating fluid to be active. Some sensors with hydratable membranes may be stored dry but they need the hydration of their membranes for the existence of an active state. When the sensor assembly 512 has at least one such sensor at 518 that requires hydration for activity, the preconditioning fluid 514 is the activating fluid. Preconditioning fluid 514 is in fluid contact with the sensor by the sealed presence of the fluid within the bag formed by layer 510. In this manner the preconditioning fluid is between the gas impervious, diffusion tight layer and the sensor by its presence within the bag in fluid contact with the sensor.

Nonexclusive examples of such an activating fluid used as the atmosphere in the layer 510 are: moist air or air with a relative humidity greater than around 30 percent, or super-saturated moist air, or any similar moisture-containing or moisture-ladened inert gas. In addition to the fluid 514 occupying the environs between the sealed bag and the housing 522, the fluid can also occupy the channel 524 which may be sealed in a similar manner as discussed above for Figure 1-18 or may remain unsealed.

Figure 42 shows an embodiment where the preconditioning fluid 514 is an activating fluid or controlled-content fluid or both fluids by the presence of two separate fluids. When the fluid 514 is either one or the other, it is in fluid contact with the one or more sensors within a sealed housing 522. When both fluids are present the activating fluid will be referred to as 538 rather than 514. In this case, the preconditioning fluid 514 is the controlled-content fluid present outside the sealed housing 522 while the activating fluid 538 is present within the sealed housing. The controlled-content fluid 514 can equilibrate with the one or more sensors that are in an active state in the sensor assembly 522 in order to precondition an active sensor.

The controlled-content fluid can be a gas, liquid or combination of a gas and liquid depending on the state of the analyte that is detected by the sensor. For a nonexclusive example when the analyte is a blood gas such as oxygen and/or carbon dioxide and the controlled-content fluid 514 is a gas one or more of these gases alone or in combination with each other or with inert gases can purge the layer after the sensor assembly 512 is placed in the layer and prior to hermetic sealing. Also it is possible that the controlled-content fluid is just the inert gas when it may be desired to provide a zero quantity of the analyte in the fluid. When the controlled-content fluid 514 is a combination of gas and a liquid, such a fluid can be produced with the requisite quantity of the gas by any method known to those skilled in the art. For example, such a fluid can be a tonometered fluid produced by any of the commercially available tonometers as previously discussed for the vials.

An example of an equilibrated or tonometered fluid as fluid 514 can result from contact of the buffered liquid solution with the carbon dioxide containing gas which can include a mixture of carbon dioxide with one or more inert gases. An inert gas is one which does not react with the buffer solution to change the pH. This would destroy the predictability of a final pH value. Also, inert gas is one that does not react with any of the ingredients in the preconditioning fluid 514. Nonexclusive examples of inert gases are nitrogen, argon and other similar gases normally found in the air. This includes the noble gases such as neon, argon, krypton, xenon, helium and the like. It is preferred to use as the equilibrating gases for blood gas analysis a mixture of carbon dioxide and nitrogen or carbon dioxide with oxygen and nitrogen. Two nonexclusive examples include: 1) around 5 percent carbon dioxide with nitrogen making up the balance of the gas in the fluid and 2) around 7 volume percent carbon dioxide and around 10 volume percent oxygen and the balance is nitrogen.

The controlled-content fluid with the controlled amount of gas or equilibrated with gas is maintained in an environment which prevents the diffusion of gas or vapor into or out of the system to prevent any drifting of the partial pressure values and any change in pH value. Art-recognized apparatus for maintaining this fluid can be used and one such example is the aforementioned commercial tonometer.

In addition, Figure 42 has a different configuration for the housing and sensor element 515 from that of Figure 41. Layer 510 is shown in the form of a bag as in Figure 41 having the outer layer 534 and inner sealable polymeric layer 536. The sensor assembly 515 has the nonconducting substrate, one or more sensors 518, electrical circuitry 520, and housing 522 with inlet 526, outlet 528 and channel 524 as in Figure 41. The preconditioning fluid is sealed in the channel 524 and side channels 523 and 525 that connect with channel 524 in the housing 522. When the one or more sensors on the nonconducting substrate are in an active state, the preconditioning fluid can be the controlled-content fluid. One or more of the sensors on the non-conducting substrate may require something other than itself to be active, i.e. the presence of an additional component such as a hydrating fluid.

With the sensor assembly 512 with the sealed activating fluid 538 in fluid contact with the one or more sensors 518 in placed in the layered bag 510 with or without the calibrator/collector or the cartridge, the last unsealed edge or all of the edges are sealed at this time. The manner of sealing can be the heat or induction sealing methods

Generally, heat-sealing or RF sealing occurs by applying the heat or RF to the metal foil layer directly or indirectly which radiates the heat to the polymeric layer that is contacting another polymeric layer at the edge of the bag and the heat causes the polymeric layer to diffuse to seal the bag. The heat sealing is conducted for a time sufficient to perform melting and bonding of the sealable resin, for example 0.1 to 5 seconds. The heat sealing operation can be performed in an operation comprised of one stage or two or more stages. In the latter case, the same or different temperature and pressure conditions as those aforementioned can be adopted at these stages. The formed sealed area is cooled, if necessary, under application of pressure by optional means to form a sealed area with good sealing efficiency. For instance, immediately after completion of the heat sealing operation, the heat sealed area in which the resin is still in the softened or molten state is pressed by two positively cooled press bars whereby the resin is solidified. Although any operation known to those skilled in the art to cool and harden, the adhesive polymer can be used.

For induction sealing, generally any induction sealing process known to those skilled in the art of induction sealing can be used. A nonexclusive example of a suitable process involves using equipment that is available from Giltron, Inc., Medfield, Massachusetts 02052, referred to as Foil Sealer Induction Heat Sealer, Model PM1.

Figure 43 discloses an embodiment of the present invention where in addition to the presence of the sensor assembly 512 in the layered envelope 510 there is also present a sample collection means 554 and a calibrant delivery means 556. Most preferably, these means are affiliated with the sensor assembly 512 in a user friendly fashion as previously discussed for Figures 24-30. Also, it is preferred to have two preconditioning fluids one present in the bag. One fluid is an controlled-content fluid and other is an activating fluid since in the preferred embodiment at least one sensor is present that has at least one hydratable membrane.

When the hermetically-sealed sensor apparatus of the present invention needs to be sterilized, the hermetically sealed sensor can be sterilized by gamma-sterilization or pasteurization sterilization. For example, the hermetically sealed sensor apparatus as depicted in Figures 1-3 can be sterilized as an entire unit. A nonexclusive example of a pasteurization technique that can be used with the hermetically-sealed sensor apparatus of the present invention is heating one or more of them at a temperature of around 70°C for eight hours. The gamma- radiation sterilization can occur with the use of any gamma-sterilization equipment known to those skilled in the art. For pasteurization sterilization, the cooling rate should be such that in the total heat history given the channels is accomplished over an adequate period of time.

When the hermetically-sealed sensor apparatus with all of its contents undergo sterilization by gamma-radiation, initial oxygen concentrations can be altered for certain types of fluid compositions. The gas composition of the controlled-content fluid 514 in Figures 42 or 43 preferably contains little if any oxygen but some oxygen may be present in fluid 14 when gamma-sterilization is used. Gamma-sterilization consumes oxygen thereby reducing any oxygen initially present in fluid 514 or equilibrated into fluid 538.

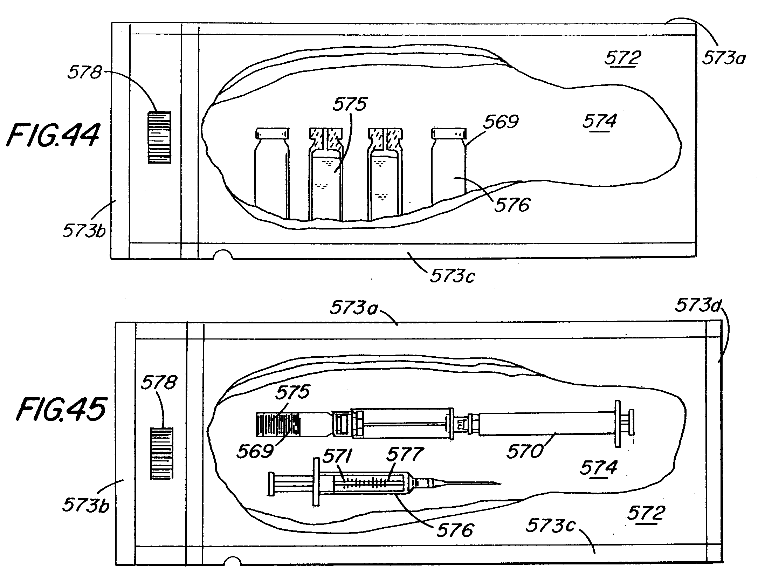

Figure 44 shows a package of vials or containers 569 and Figure 45 shows a package of vials or containers 569 and delivery devices 570 and 571 for the calibration of the multiple use sensor in the cartridge when the analytes to be measured are gases like blood gases. If the vial is gas impervious, the package does not have to be gas impervious. There are two situations where the package or wrapper is gas impervious. One is where the calibrating fluid or hydrating fluid is used as a flush between samples and the wrapper 572 is gas impervious to provide an atmosphere in the bag to equilibrate with the flush fluid. Another situation is where immediate containers for the calibrating fluid and/or flush fluid are not gas impervious. Wrapper 572 in Figures 44 and 45 can be the same type of material as those aforedescribed for wrappers for the sensor assembly.

In Figure 35 the controlled-content fluid 514 contacts the cartridge 411 with the sensors in the sensor assembly 416 by the fluid's sealed presence within the bag 418. In this manner the controlled-content fluid 514 is between the gas impervious, diffusion-type layer and the sensors by its presence within the bag 418 in fluid contact with the sensors through plastic containers such as the cartridge 411 and the sensor assembly 416. The controlled-content fluid equilibrates with the sensors that are in an active state in the presence of the hydrating fluid 34 in the flow cell 16 in order to precondition an active sensor in a similar manner as that for the sensor assembly 512 of Figures 41-43.

In a similar manner in wrapper 572, the controlled-content fluid 574 is in contact with the hydrating or flush fluid 572 or a calibrating fluid 575 in gas permeable containers 576. Preferably, the calibrating fluid 575 is in gas impervious containers like the sealed vials 569 or those previously discussed with for Figures 24-32 or glass ampules. Vials 569 can be glass vials with an opening that is heat-sealed with by a gas impervious material like those used for the bag 18. Preferably, the opening for vial 569 has the smallest practical diameter that allows for intentional ingress and egress of the fluid. The calibrating fluid 575 in vial 569 is a fluid containing a known set amount of the one or more analytes to be measured as previously discussed for Figures 24-32.

Figures 44 and 45 show the calibrating fluid 575 and flushing fluid 577 in packages 572 with the encoded information carrier 578. Figure 45 shows the calibrant and flushing solution with the encoded information carrier 578 of the preferred embodiment of the present invention where there is also present a calibrant delivery device 570 and a flushing fluid delivery device 571. Most preferably, carrier 578 like carrier 421 is affiliated with the package 572 in a user friendly fashion. Carrier 578 can be of a similar type as that of carrier 421 and contains information about the concentrations of the analytes in the calibrating fluid.

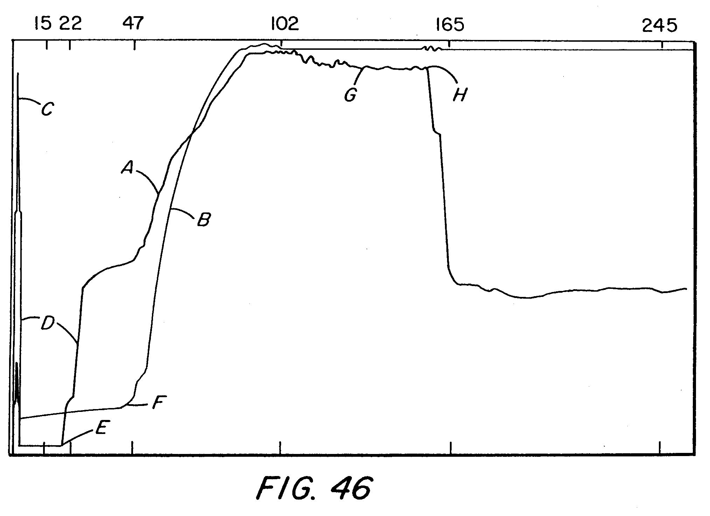

Figure 46 shows a graft of the nanoamp output of the sensor over time for the sensor in the sensor-collector-calibrator apparatus of the sensor apparatus of the present invention as shown in Figure 43, where the two preconditioning fluids are present. One fluid is the controlled-content fluid and the other is the activating fluid that is the hydrating fluid. A third fluid is present in the calibrator and it is the calibration fluid. The known-content fluid 514B can have a composition of around 5 percent carbon dioxide with nitrogen making up the balance of the gas in the fluid. The calibration fluid had known values of carbon dioxide and oxygen such as around 7 volume percent carbon dioxide and around 10 volume percent oxygen with the balance as nitrogen. The sensors in the sensor assembly included an oxygen, carbon dioxide and pH sensor with accompanying reference electrodes in the case of carbon dioxide and pH much like that shown in Figure 42. Nanoamps are along the ordinate while the time is along the abscissa.

Curve A is the nanoamp output and Curve B is the temperature of a heater present on the nonconducting substrate along with at least the oxygen sensor. The peak at C is when the cable 502 is plugged into a measuring device. The time period indicated as D is the time period that the equilibrated hydrating fluid which is the activating fluid in the sensor assembly 512 is read by the measuring instrument. Here, both the controlled-content fluid and the activating fluid are essentially devoid of and preferably do not contain any oxygen. The calibrant is introduced across the sensor at point E and the oxygen sensor begins to read the oxygen concentration in the calibration fluid 575 of Figure 43. The current output of the oxygen sensor tracks the oxygen until the heater clicks in at point F and heats the calibration fluid to a temperature similar to the temperature at which the unknown sample has when collected. For example, blood when collected has a body temperature of around 37°C. The current output from the sensor measuring the oxygen concentration of the calibration fluid stabilizes in about 30 seconds to give a value of the oxygen concentration at the desired temperature for the calibration fluid as indicated at range G in the curve. At point H the blood sample is introduced and the current output of the sensor in this case decreases to measure the oxygen concentration of the blood gas sample.

When the calibration fluid is introduced, the electronic measuring means detects both the carbon dioxide and the pH of the calibrant because the carbon dioxide equilibration with the known-content fluid can cause a change in the pH. The concentration of the carbon dioxide and the pH of the equilibrated hydrating fluid are known as is that for the calibrant in the vial 234 since the calibrant in the vial is not equilibrated with the known-content fluid 514B. There is no equilibration since the vial is hermetically sealed from the atmosphere in layer 510. When the calibrant is introduced at point E of the chart, the current signal changes by some known amount and this change is compared to a statistical average of changes occurring for numerous samples to ascertain if the change meets the minimum criteria for sensor operability. This tests the sensitivity of the sensor to determine if it is accurate. For utilizing a 1 point calibration in determining concentration values of samples, the determination and sensitivity of the sensor is important even in calibrating for the offset of the sensor from the origin of a relationship of current output over time as indicated Figure 46.

In this way the hydrating fluid that is equilibrated with the controlled-content fluid is used as a diagnostic check for adequate operability of the sensor. This is important in the use of portable sensors as a check for whether or not the sensor is properly operating.

In the situation of the sensor including an oxygen sensor, a carbon dioxide sensor and a pH sensor and two reference electrodes for the carbon dioxide sensor and pH sensor, a 1 point calibration can be performed. For a 1 point calibration, the slope of the line or the offset from the origin must be known. In order to determine the offset of the oxygen sensor, the sensor current in the absence of oxygen must be known. Therefore, the envelop layer 510 and its packaged sensor assembly with the preconditioning fluids are arranged so that the controlled-content fluid is free of oxygen or contains very little oxygen that may be consumed during gamma-sterilization and this controlled-content fluid is allowed to equilibrate across the housing material of the sensor assembly with the second preconditioning fluid that is the hydrating or activating fluid. After the unit is plugged into the measuring module, the unit looks at the current of the oxygen sensor. If no oxygen is present, the current is read by the unit as the offset. So the amount of current read when current is first plugged in indicates of the sensor is good or not. A standard is set for background current and the sensor unit is checked to see if the oxygen sensor meets the offset requirements of zero or essential zero.

The preconditioning fluid with the controlled-content fluid in the pouch layer 510 allows the test for the offset limit without the need of using difficult solutions such as sulfides and the like to determine the offset of the oxygen sensor. The offset for the oxygen concentration is measured before the calibrating fluid is in contact with the oxygen sensor and before heating, since the oxygen sensor has a temperature coefficient. With the known carbon dioxide value in the controlled-content fluid, the carbon dioxide and pH sensor can be calibrated. Both of these sensors have a certain sensitivity in millivolts per millimeters of mercury for carbon dioxide tension. This is determined via a statistical process by measuring many samples which are compared against the sensor being checked. The monitor means checks or predicts the millivolt change from the millivolts measured for the hydrating fluid and the calibrant fluid and this change should be within the statistical range of acceptable values or the sensitivity of the sensor is not within specifications.

The calibrant is introduced over the sensor when the sensor assembly with collection means and calibrating means or the cartridge is removed from the hermetically sealed envelope of layer 510. After removal, the unit is appropriately assembled and the calibrant is introduced over the sensor. In the time it takes to remove the sensor from the hermetically sealed envelope and introduce the calibrant, the unit was already plugged into the measuring module and the module was measuring the analytes carbon dioxide and the pH preferably in the preconditioning fluid that is the equilibrated hydrating fluid.

Although the invention has been described in some detail by the foregoing, it is to be understood that many variations may be made therein without departing from the spirit and the scope thereof as defined by the appended claims.

## Claims

1. An apparatus having a preconditioned sensor and electronic circuit, comprising:
sensor element (14) with a nonconducting substrate with at least one preconditioned electrochemical sensor (18) with a membrane and with electrical circuitry means (20) in electrical contact with the sensor (18) to convey electrical impulses from the sensor (18),
housing (10) having at least one part enclosing the sensor element (14) with a nonconducting substrate and forming a channel (12) in fluid contact with the at least one sensor (18) on the substrate element (14), wherein the channel (12) has two openings (24, 26) from the housing (10) for fluid flow through the channel (12) and that is in fluid contact with the at least one sensor (18) on the substrate of element (14) to allow fluid in the channel (12) to be in fluid contact with the sensor (18) where one opening (24, 26) is adapted for receipt of a fluid sample for measurement of an analyte by the sensor (18), and another opening (24, 26) is positioned in the channel (12) after the fluid sample contacts at least one sensor (18),
preconditioning fluid (22) occupying a substantial portion of the channel (12) to precondition the membrane of the one or more sensors (18),
seals (28, 30) that are substantially impervious to at least moisture placed in the channel to minimize the transport of preconditioning fluid (22) away from contact with the one or more sensors (18),
electrical attachment means (16) electrically associated with the circuit means (20) of the substrate of element (14) to convey the sensor signals from the apparatus for interpretation,
wherein the preconditioning fluid (22) is electrically isolated from the electric circuit (20) of the substrate of element (14) and the electrical attachment means (16).

2. Apparatus of claim 1 wherein the substrate of element (14) has at least one sensor (18) to detect one or more analytes in fluid wherein the analytes are one or more blood gases and the fluid is blood.

3. Apparatus of claim 1 wherein the substrate of element (14) is ceramic and has three sensors (18), one for measuring the partial pressure of oxygen, another for measuring the partial pressure of carbon dioxide and another of measuring the pH of a fluid like blood.

4. Apparatus of claim 1 wherein substrate of element (14) has the electrical circuit means (20) designed to enhance electrical isolation from the preconditioning fluid (22).

5. Apparatus of claim 1 wherein housing (10) has at least two sections (32, 34) and both sections have at least one set of attachment means (72, 74, 76, 78) for suitable attachment of the two sections (32, 34) to enclose the substrate of element (14) in the internal space (82), wherein the housing (10) has a channel (12) formed by one section (32, 34) of the housing (10) and the substrate of element (14) within the internal space between the sections (32, 34) or the housing.

6. Apparatus of claim 5 wherein the housing (10) has the channel (12) extending along a longitudinal axis in a near linear orientation from one end of the housing (10) to the other, wherein the channel (12) emerges from each end of the housing in tips (36, 38) for attachment to a sample collection device at one end and a sample disposal device at the other end.

7. Apparatus of claim 6 wherein the housing (10) includes one or more adhesive ports (92) filled with cured adhesive.

8. Apparatus of claim 1 wherein the housing (10) has one or more side channels (88, 90) fluidly connected to the channel (12) with openings (124, 126) from the housing (10) that are substantially full of preconditioning fluid (22).

9. Apparatus of claim 1 wherein the housing (10) has a slot for the ingress and egress of the electronic attachment means (16).

10. Apparatus of claim 1 wherein the preconditioning fluid (22) is a hydrating fluid (22) that is isotonic with the hydrated state of the submembrane of the sensor (18).

11. Apparatus of claim 1 wherein the seals (28, 30) are located one at each opening (24, 26) of the channel (12) from the housing (10).

12. Apparatus of claim 1 wherein the substantially impervious seals (28, 30) are also impervious to oxygen and carbon dioxide.

13. Apparatus of claim 1 wherein the seals (28, 30) have inner surface (28b, 30b) attached to the housing (10) covering the opening (24, 26) of the channel (12) and outer surface (28a, 30a), where the outer surface (28a, 30a) is metal and the inner surface (28b, 30b) is an adhesive type polymer, where said seal (28, 30) covers the opening of the channel (12) by the inner adhesive surface (285, 305) being stuck to at least a portion of the housing (10) surrounding opening of the channel, and wherein the seals (28, 30) are flexible generally circular disc having a generally circular periphery and a diameter such as to circumferentially seal the openings (24, 26) of the channel (12).

14. Apparatus of claim 1 wherein the seals (28, 30) are induction sealed to the housing (10) over the channel openings (24, 26).

15. Apparatus of claim 1 wherein the seals (28, 30) are heat sealed to the housing (10) over the channel openings (12).

16. Apparatus of claim 1, which includes an electrically isolating means (54) to isolate the electric circuit means (20) on the substrate and the hydrating fluid (22), where the electrically isolating means (54) occupies the internal space (82) not already occupied by other components, and wherein the electrically isolating means (54) is placed in the housing (10) through a hole (118) in the housing.

17. Apparatus of claim 1 wherein the apparatus is gamma-sterilized.

18. Apparatus of claim 16 wherein the electrical isolating means (54) is a hardened epoxy polymer.

19. Apparatus of claim ( 1 ) wherein the nonconducting substrate (112) with at least one preconditioned electrochemical sensor (18) and with electrical circuitry means (20) in electrical contact with the sensor (18) to convey electrical impulses from the sensor (18) is an improved electronic wiring board of sensor element (14) having a nonconducting substrate (112), thermistor (138) and at least one analyte sensor (18) supported, in close relation, one to the other, on the substrate (112) and a heater (174), also supported on the substrate (112), to provide heat in response to temperature sensed by said thermistor (138) to at least the region where said thermistor (138) and said blood gas sensor (18) are positioned on said board (14) to thereby control the temperature of said region of said board (14) within a narrow distribution of temperatures, and connecting means of circuitry means (20) supported on said board for connecting said board to an external electrical source.

20. Apparatus of claim 19 wherein the improved electronic wiring board comprises:
a nonconducting substrate (112) with electrical circuitry means (20) with one or more leads to and from the circuitry means (20) on the substrate,
rigid plastic member covering at least a portion of at least one surface of the substrate (112),
UV cured epoxy adhesive contacting the plastic and the substrate (112) to adhesively secure them.

21. Apparatus of claim 20 wherein the UV cured epoxy adhesive has a pattern of contact achieved by wicking the curable epoxy adhesive on one contacting surface.

22. The apparatus of claim 19 wherein said board (14) is manufactured using thick film layered circuit technique.

23. The apparatus of claim 19 wherein said thermistor (138) and said blood gas sensors are supported in the same plane on said board (14).

24. The apparatus of claim 19 wherein said blood gas sensors (18) includes at least one of the following: an oxygen sensor, carbon dioxide sensor, and a pH sensor.

25. The apparatus of claim 19 wherein said connecting means of circuitry means (20) includes plurality of external leads (146 -160), a resistor (176) is supported on said board (14) on the same side as said heater (174) and commonly connected to one of said external leads (146-160) with said thermistor (138), dividing the voltage therebetween.

26. The apparatus of claim 19 wherein the temperature coefficient of said thermistor (138) is negative or positive and the temperature coefficient of said resistor (176) is substantially zero, and where the divided voltage is proportional or inversely proportional to temperature, and this output is used to measure temperature.

27. The apparatus of claim 25 wherein said connecting means of circuitry means (20) further includes a plurality electronic conducting pathways (118, 122, 128, 132, 138, 140) individually and electrically connecting each of said sensors (18) and said thermistor (138) with external leads (146-160) provided on said board (14) at the end of said pathways (118, 122, 128, 132, 138, 140) and wherein said resistor (176) and said heater (174) are each electrically connected to external leads (146-160).

28. The apparatus of claim 19 wherein said heater (174) is powered by pulsed DC whereby said heater (174) is continually turned on and off thereby avoiding said heater (174) from overshooting or undershooting a predetermined temperature.

29. The apparatus of claim 24 wherein said blood gas sensors (18) are calibrated by laser trimming of said resistor (176).

30. The apparatus of claim 29 wherein said oxygen sensor (124) is an electrochemical cell and includes a anode (126) and a cathode (134), each connected to an external lead (154).

31. The apparatus of claim 30 wherein said oxygen sensor (124) includes an oxygen permeable membrane covering, in a fluid tight manner, an opening in said board (14) containing an electrolyte, said anode (126) being grounded on said board (14) to thereby assure that potential of said electrolyte is the same as the anode potential.

32. The apparatus of claim 31 wherein there is at least one reference electrode (64, 66), to provide an accurate reference potential, supported on said board (14) and is electrically connected to a electronic conducting pathway (118, 122, 128, 132, 138, 140).

33. The apparatus of claim 32 wherein there is one reference electrode (64, 66) supported on said board (14) and is electrically connected to a electronic conducting pathway (118, 122, 128, 132, 138, 140) extending from said anode (126).

34. The apparatus of claim 33 wherein said board (14) is a substantially flat ceramic layer (112) and includes a patterned metallic layer (114) formed on said substrate (112) by depositing a metallic printing paste on said substrate (112) to form electronic conducting pathways (118, 122, 128, 132, 138, 140) and the electrodes of said sensors (18) and the electrode of a reference electrode (64, 66).

35. The apparatus of claim 34 wherein said metallic layer (114) is encapsulated with at least one layer of a chemically stable and moisture resistant encapsulant (144).

36. The apparatus of claim 35 wherein said board (14) includes a thermistor (138) provided on ceramic substrate layer (112), said thermistor (138) is encapsulated with at least one substantially thin layer of a chemically stable and moisture resistant encapsulant (144).

## Patentansprüche

1. Vorrichtung mit einem vorkonditionierten Sensor und einem elektronischen Schaltkreis, enthaltend:
Sensorelement (14) mit einem nichtleitenden Substrat mit wenigstens einem vorkonditionierten elektrochemischen Sensor (18) mit einer Membran und mit Mitteln (20) zur Ausbildung eines Stromkreises in elektrischem Kontakt mit dem Sensor (18), um elektrische Impulse von dem Sensor (18) zu übermitteln,
Gehäuse (10) mit wenigstens einem Teil, das das Sensorelement (14) mit einem nichtleitenden Substrat einschließt und einen Kanal (12) ausbildet, der in Fluidkontakt mit dem wenigstens einen Sensor (18) auf dem Substratelement (14) steht, wobei der Kanal (12) zwei Öffnungen (24,26) aus dem Gehäuse (10) zum Fluidfluß durch den Kanal (12) aufweist und dieses in Fluidkontakt mit dem wenigstens einen Sensor (18) auf dem Substrat des Elementes (14) steht, um der Flüssigkeit in dem Kanal (12) zu ermöglichen, in Fluidkontakt mit dem Sensor (18) zu treten, wobei eine Öffnung (24,26) für die Aufnahme einer Fluidprobe zur Messung eines Analytes durch den Sensor (18) angepaßt ist und eine andere Öffnung (24,26) in dem Kanal (12) angeordnet ist, nachdem die Fluidprobe mit dem wenigstens einen Sensor (18) in Kontakt tritt,
Vorkonditionierungsfluid (22), das einen wesentlichen Teil des Kanals (12) besetzt, um die Membran des einen oder der mehreren Sensoren (18) vorzukonditionieren,
Dichtungen (28,30), die im wesentlichen undurchlässig mindestens in bezug auf die Feuchtigkeit, die sich in dem Kanal befindet, sind, um den Transport des Vorkonditionierungsfluids (22) weg von dem Kontakt mit dem einen oder den mehreren Sensoren (18) zu minimieren,
elektrische Anschlußmittel (16), die elektrisch mit den Mitteln (20) zur Ausbildung eines Stromkreises des Substrats des Elementes (14) verbunden sind, um die Sensorsignale von der Vorrichtung zur Auswertung zu übermitteln,
wobei das Vorkonditionierungsfluid (22) elektrisch von dem elektrischen Schaltkreis (20) des Substrats des Elementes (14) und den elektrischen Anschlußmitteln (16) isoliert ist.

2. Vorrichtung nach Anspruch 1, wobei das Substrat des Elementes (14) wenigstens einen Sensor (18) aufweist, um einen oder mehrere Analyte in einem Fluid zu detektieren, wobei die Analyte ein oder mehrere Blutgase sind und das Fluid Blut ist.

3. Vorrichtung nach Anspruch 1, wobei das Substrat des Elementes (14) aus Keramik ist und drei Sensoren (18) aufweist, einen zur Messung des Partialdruckes von Sauerstoff, einen anderen zur Messung des Partialdruckes von Kohlendioxid und einen anderen zur Messung des pH-Wertes eines Fluids wie Blut.

4. Vorrichtung nach Anspruch 1, wobei das Substrat des Elementes (14) Mittel (20) zur Ausbildung eines Stromkreises aufweist, die ausgebildet sind, um die elektrische Isolation von dem Vorkonditionierungsfluid (22) zu verstärken.

5. Vorrichtung nach Anspruch 1, wobei das Gehäuse (10) wenigstens zwei Sektionen (32,34) aufweist und beide Sektionen wenigstens ein Set von Verbindungsmittel (72,74,76,78) zur geeigneten Verbindung der beiden Sektionen (32,34) aufweist, um das Substrat des Elementes (14) in dem Innenraum (82) einzuschließen, wobei das Gehäuse (10) einen Kanal (12) aufweist, der durch eine Sektion (32,34) des Gehäuses (10) und durch das Substrat des Elementes (14) innerhalb des Innenraums zwischen den Sektionen (32,34) des Gehäuses ausgebildet ist.

6. Vorrichtung nach Anspruch 5, wobei das Gehäuse (10) einen Kanal (12) aufweist, der sich entlang der Längsachse in einer annäherend linearen Orientierung von einem Ende des Gehäuses (10) zu dem anderen erstreckt, wobei der Kanal (12) aus jedem Ende des Gehäuses in Spitzen (36,38) zum Anschluß an eine Probensammeleinrichtung an einem Ende und an eine Probenbeseitigungseinrichtung an dem anderen Ende ausläuft.

7. Vorrichtung nach Anspruch 6, wobei das Gehäuse (10) einen oder mehrere verklebte Eingänge (92), die mit gehärtetem Klebstoff gefüllt sind, enthält.

8. Vorrichtung nach Anspruch 1, wobei das Gehäuse (10) einen oder mehrere Seitenkanäle (88,90) aufweist, die mit dem Kanal (12) durch Öffnungen (124,126) aus dem Gehäuse (10), die im wesentlichen mit dem Vorkonditionierungsfluid (22) gefüllt sind, flüssigkeitsmäßig verbunden sind.

9. Vorrichtung nach Anspruch 1, wobei das Gehäuse (10) einen Schlitz für den Eintritt und Austritt der elektronischen Anschlußmittel (16) aufweist.

10. Vorrichtung nach Anspruch 1, wobei das Vorkonditionierungsfluid (22) ein hydratisierendes Fluid (22) ist, das isotonisch bezüglich des hydratisierten Zustands der Untermembran des Sensors (18) ist.

11. Vorrichtung nach Anspruch 1, wobei die Dichtungen (28,30) jeweils an jeder Öffnung (24,26) des Kanals (12) aus dem Gehäuse (10) angeordnet sind.

12. Vorrichtung nach Anspruch 1, wobei die im wesentlichen undurchlässigen Dichtungen (28,30) ebenfalls undurchlässig für Sauerstoff und Kohlendioxid sind.

13. Vorrichtung nach Anspruch 1, wobei die Dichtungen (28,30) eine innere Oberfläche (28b,30b), die mit dem Gehäuse (10) verbunden ist und die Öffnung (24,26) des Kanals (12) abdeckt, und eine äußere Oberfläche (28a,30a) aufweisen, wobei die äußere Oberfläche (28a,30a) aus Metall besteht und die innere Oberfläche (28b,30b) aus einem klebstoffartigen Polymer besteht, wobei diese Dichtung (28,30) die Öffnung des Kanals (12) mit der inneren klebenden Oberfläche (28b,30b) abdeckt, die wenigstens an einem Teil des Gehäuses (10), das die Öffnung des Kanals umgibt, aufgeklebt ist, und wobei die Dichtungen (28,30) flexible, im allgemeinen kreisförmige Scheiben mit einer im allgemeinen kreisförmigen Peripherie und einem Durchmesser derart sind, um die Öffnungen (24,26) des Kanals (12) umfänglich abzudichten.

14. Vorrichtung nach Anspruch 1, wobei die Dichtungen (28,30) mit dem Gehäuse (10) oberhalb der Kanalöffnungen (24,26) mittels Induktionserwärmung verschmolzen sind.

15. Vorrichtung nach Anspruch 1, wobei die Dichtungen (28,30) mit dem Gehäuse (10) oberhalb der Kanalöffnungen (12) mittels Wärme verschmolzen sind.

16. Vorrichtung nach Anspruch 1, die Einrichtungen (54) zur elektrischen Isolation, um die Mittel (20) zur Ausbildung eines Stromkreises auf dem Substrat von dem hydratisierenden Fluid (22) zu isolieren, aufweist, wobei die Einrichtungen (54) zur elektrischen Isolation den Innenraum (82) besetzen, der nicht bereits durch andere Komponenten besetzt ist, und wobei die Einrichtungen (54) zur elektrischen Isolation in das Gehäuse (10) durch eine Öffnung (118) in dem Gehäuse eingebracht werden.

17. Vorrichtung nach Anspruch 1, wobei die Vorrichtung durch Gammastrahlung sterilisiert ist.

18. Vorrichtung nach Anspruch 16, wobei die Einrichtungen (54) zur elektrischen Isolation ein gehärtetes Epoxypolymer sind.

19. Vorrichtung nach Anspruch 1, wobei das nichtleitende Substrat (112) mit wenigstens einem vorkonditionierten elektrochemischen Sensor (18) und mit Mitteln (20) zur Ausbildung eines Stromkreises, die in elektrischem Kontakt mit dem Sensor (18) sind, um elektrische Impulse von dem Sensor (18) zu übermitteln, eine verbesserte elektronische Verdrahtungsplatine mit einem Sensorelement (14) ist, das ein nichtleitendes Substrat (112), einen Thermistor (138) und wenigstens einen Analytsensor (18) aufweist, die in unmittelbarer Nähe zueinander auf dem Substrat (112) gehalten werden, und eine Heizvorrichtung (174) aufweist, die ebenfalls auf dem Substrat (112) gehalten wird, um Wärme als Reaktion auf die Temperatur, die durch diesen Thermistor (138) gemessen wird, wenigstens zu dem Bereich, in dem dieser Thermistor (138) und dieser Blutgassensor (18) auf dieser Platine (14) angeordnet sind, zuzuführen, um dadurch die Temperatur dieses Bereiches auf dieser Platine (14) innerhalb einer engen Temperaturverteilung zu regeln, und Anschlußmittel für die Mittel (20) zur Ausbildung eines Stromkreises aufweist, die auf dieser Platine gehalten werden, um diese Platine an eine externe Spannungsquelle anzuschließen.

20. Vorrichtung nach Anspruch 19, wobei die verbesserte elektronische Verdrahtungsplatine ein nichtleitendes Substrat (112) mit Mitteln (20) zur Ausbildung eines Stromkreises mit einer oder mehreren Leitungen zu und von den Mitteln (20) zur Ausbildung eines Stromkreises auf dem Substrat, ein steifes Plastikteil, das wenigstens einen Teil mindestens einer Oberfläche des Substrats (112) abdeckt, und einen UV gehärteten Epoxyklebstoff, der mit dem Plastikteil und dem Substrat (112) in Kontakt steht, um diese klebend miteinander zu verbinden, aufweist.

21. Vorrichtung nach Anspruch 20, wobei der UV-gehärtete Epoxyklebstoff ein Kontaktmuster aufweist, das durch Dochtwirkung des härtbaren Epoxyklebstoffs auf einer Kontaktoberfläche erreicht wird.

22. Vorrichtung nach Anspruch 19, wobei diese Platine (14) unter Verwendung einer Dickfilmschaltungstechnik hergestellt ist.

23. Vorrichtung nach Anspruch 19, wobei dieser Thermistor (138) und diese Blutgassensoren in derselben Ebene auf dieser Platine (14) gehalten werden.

24. Vorrichtung nach Anspruch 19, wobei diese Blutgassensoren (18) wenigstens einen der Folgenden enthalten: einen Sauerstoffsensor, einen Kohlendioxidsensor und einen pH-Sensor.

25. Vorrichtung nach Anspruch 19, wobei diese Anschlußmittel der Mittel (20) zur Ausbildung eines Stromkreises eine Vielzahl von externen Leitungen (146-160) beinhalten, ein Widerstand (176) auf dieser Platine (14) auf derselben Seite wie diese Heizvorrichtung (174) gehalten wird und allgemein mit einer dieser externen Leitungen (146-160) verbunden ist, wobei dieser Thermistor (138) die Spannung dazwischen teilt.

26. Vorrichtung nach Anspruch 19, wobei der Temperaturkoeffizient dieses Thermistors (138) negativ oder positiv ist und der Temperaturkoeffizient dieses Widerstands (176) im wesentlichen null ist und die geteilte Spannung proportional oder umgekehrt proportional zur Temperatur ist und dieser Output verwendet wird, um die Temperaturen zu messen.

27. Vorrichtung nach Anspruch 25, wobei diese Anschlußmittel der Mittel (20) zur Ausbildung eines Stromkreises weiterhin eine Vielzahl elektronischer Leitungswege (118,122,128,132,138,140) aufweisen, die individuell jeden dieser Sensoren (18) und diesen Thermistor (138) elektrisch mit externen Leitungen (146-160) verbinden, die auf dieser Platine (14) am Ende dieser Leitungswege (118,122,128,132,138,140) bereitgestellt werden, und wobei dieser Widerstand (176) und diese Heizvorrichtung (174) jeweils elektrisch mit externen Leitungen (146-160) verbunden sind.

28. Vorrichtung nach Anspruch 19, wobei diese Heizvorrichtung (174) durch gepulsten Gleichstrom betrieben wird, wobei diese Heizvorrichtung (174) kontinuierlich an- und ausgeschaltet wird, wodurch vermieden wird, daß die Heizvorrichtung (174) eine vorbestimmte Temperatur überschreitet oder unterschreitet.

29. Vorrichtung nach Anspruch 24, wobei diese Blutgassensoren (18) durch Lasertrimmen dieses Widerstands (176) kalibriert sind.

30. Vorrichtung nach Anspruch 29, wobei dieser Sauerstoffsensor (124) eine elektrochemische Zelle ist und eine Anode (126) und ein Kathode (134) aufweist, die jeweils mit einer externen Leitung (154) verbunden sind.

31. Vorrichtung nach Anspruch 30, wobei dieser Sauerstoffsensor (124) eine sauerstoffpermeable Membran aufweist, die in einer flüssigkeitsdichten Art und Weise eine Öffnung in dieser Platine (14) bedeckt, und einen Elektrolyten enthält, wobei diese Anode (126) auf dieser Platine (14) an Masse liegt, um dadurch sicherzustellen, daß das Potential dieses Elektrolyten dasselbe ist wie das Anodenpotential.

32. Vorrichtung nach Anspruch 31, wobei wenigstens eine Referenzelektrode (64,66) vorhanden ist, um ein genaues Referenzpotential bereitzustellen, und auf dieser Platine (14) gehalten wird und elektrisch mit einem elektronischen Leitungsweg (118,122,128,132,138,140) verbunden ist.

33. Vorrichtung nach Anspruch 32, wobei eine Referenzelektrode (64,66) vorhanden ist, die auf dieser Platine (14) gehalten wird und elektrisch mit einem elektronischen Leitungsweg (118,122,128,132,138,140) verbunden ist, der sich von dieser Anode (126) erstreckt.

34. Vorrichtung nach Anspruch 33, wobei diese Platine (14) eine im wesentlichen flache keramische Schicht (112) ist und eine metallische Schicht (114) mit einem Leitungsbild aufweist, die auf diesem Substrat (112) durch Abscheidung einer metallischen Druckpaste auf diesem Substrat (112) ausgebildet ist, um elektronische Leitungswege (118,122,128,132,138,140) und die Elektroden dieser Sensoren (18) und die Elektrode einer Referenzelektrode (64,66) auszubilden.

35. Vorrichtung nach Anspruch 34, wobei diese metallische Schicht (114) mit wenigstens einer Schicht aus einem chemisch stabilen und feuchtigkeitsbeständigen Einbettharz (144) verkapselt ist.

36. Vorrichtung nach Anspruch 35, wobei diese Platine (14) einen Thermistor (138) aufweist, der auf einer keramischen Substratschicht (112) vorgesehen ist, wobei dieser Thermistor (138) mit wenigstens einer im wesentlichen dünnen Schicht aus einem chemisch stabilen und feuchtigkeitsbeständigen Einbettharz (144) verkapselt ist.

## Revendications

1. Appareil présentant une sonde préconditionnée et un circuit électronique, comprenant :
un élément de sonde (14) présentant un support non conducteur avec au moins une sonde électrochimique préconditionnée (18) avec une membrane et avec un moyen à circuit électrique (20) en contact électrique avec la sonde (18), pour transporter des impulsions électriques provenant de la sonde (18),
un boîtier (10) dont au moins une partie englobe l'élément de sonde (14) présentant un support non conducteur, lequel boîtier forme un canal (12) permettant un contact du fluide avec la sonde (18) au moins présente sur l'élément de support (14), le canal (12) présentant deux ouvertures (24, 26) traversant le boîtier (10) pour un écoulement de fluide à travers le canal (12), et lequel canal permet un contact du fluide avec la sonde (18) au moins présente sur le support de l'élément (14), pour permettre au fluide situé dans le canal (12) d'être en contact avec la sonde (18), une ouverture (24, 26) étant adaptée pour recevoir un échantillon de fluide en vue de la mesure d'un analyte par la sonde (18), et une autre ouverture (24, 26) est disposée dans le canal (12) après que l'échantillon de fluide soit rentré en contact avec au moins une sonde (18),
un fluide de préconditionnement (22) occupant une partie essentielle du canal (12), pour préconditionner la membrane de la ou des sondes (18),
des joints d'étanchéité (28, 30) qui sont essentiellement imperméables au moins à l'humidité placée dans le canal, pour minimiser le transport de fluide de préconditionnement (22) hors du contact avec la ou les sondes (18),
un moyen de raccordement électrique (16) électriquement associé au moyen à circuit (20) du support de l'élément (14), pour transporter les signaux des sondes provenant de l'appareil, en vue de les interpréter,
le fluide de préconditionnement (22) étant électriquement isolé du circuit électrique (20) du support de l'élément (14) et du moyen (16) de raccordement électrique.

2. Appareil selon la revendication 1, dans lequel le support de l'élément (14) présente au moins une sonde (18) pour détecter un ou plusieurs analytes dans le fluide, les analytes étant un ou plusieurs gaz sanguins et le fluide étant du sang.

3. Appareil selon la revendication 1, dans lequel le support de l'élément (14) est en céramique et présente trois sondes (18), l'une pour mesurer la pression partielle d'oxygène, une autre pour mesurer la pression partielle de dioxyde de carbone et une autre pour mesurer le pH d'un fluide tel que le sang.

4. Appareil selon la revendication 1, dans lequel le support de l'élément (14) a son moyen à circuit électrique (20) conçu pour renforcer l'isolation électrique vis-à-vis du fluide de préconditionnement (22).

5. Appareil selon la revendication 1, dans lequel le boîtier (10) présente au moins deux tronçons (32, 34), et les deux tronçons présentent au moins un jeu de moyens de fixation (72, 74, 76, 78) en vue d'une fixation appropriée des deux tronçons (32, 34), pour qu'ils englobent le support de l'élément (14) dans l'espace intérieur (82), le boîtier (10) présentant un canal (12) formé par un tronçon (32, 34) du boîtier (10) et le support de l'élément (14) dans l'espace intérieur situé entre les tronçons (32, 34) du boîtier.

6. Appareil selon la revendication 5, dans lequel le boîtier (10) présente son canal (12) qui s'étend le long d'un axe longitudinal, dans une orientation presque linéaire depuis une extrémité du boîtier (10) jusqu'à l'autre, le canal (12) émergeant de chaque extrémité du boîtier par des pointes (36, 38) en vue de la fixation à un dispositif de collecte d'échantillon, à une extrémité, et à un dispositif d'évacuation de l'échantillon à l'autre extrémité.

7. Appareil selon la revendication 6, dans lequel le boîtier (10) comprend un ou plusieurs orifices adhésifs (92) remplis d'un adhésif durci.

8. Appareil selon la revendication 1, dans lequel le boîtier (10) présente un ou plusieurs canaux latéraux (88, 90) en communication d'écoulement avec le canal (12) par des ouvertures (124, 126) du boîtier (10), qui sont essentiellement remplis de fluide de préconditionnement (22).

9. Appareil selon la revendication 1, dans lequel le boîtier (10) présente une fente pour l'insertion et l'extraction du moyen de raccordement électronique (16).

10. Appareil selon la revendication 1, dans lequel le fluide de préconditionnement (22) est un fluide hydratant (22) qui est isotonique par rapport à l'état hydraté de la sous-membrane de la sonde (18).

11. Appareil selon la revendication 1, dans lequel les joints (28, 30) sont situés chacun à une ouverture (24, 26) du canal (12) provenant du boîtier (10).

12. Appareil selon la revendication 1, dans lequel les joints d'étanchéité essentiellement imperméables (28, 30) sont également imperméables à l'oxygène et au dioxyde de carbone.

13. Appareil selon la revendication 1, dans lequel les joints d'étanchéité (28, 30) présentent une surface intérieure (28b, 30b) fixée au boîtier (10) recouvrant l'ouverture (24, 26) du canal (12), et une surface extérieure (28a, 30a), la surface extérieure (28a, 30a) étant métallique et la surface intérieure (28b, 30b) étant un polymère de type adhésif, ledit joint d'étanchéité (28, 30) recouvrant l'ouverture du canal (12) par le fait que la surface intérieure adhésive (28b, 30b) est collée sur au moins une partie du boîtier (10) qui entoure l'ouverture du canal, et les joints d'étanchéité (28, 30) étant un disque flexible généralement circulaire présentant une périphérie généralement circulaire et un diamètre tel qu'il scelle périphériquement les ouvertures (24, 26) du canal (12).

14. Appareil selon la revendication 1, dans lequel les joints d'étanchéité (28, 30) sont scellés par induction sur le boîtier (10), au-dessus des ouvertures de canal (24, 26).

15. Appareil selon la revendication 1, dans lequel les joints d'étanchéité (28, 30) sont scellés à la chaleur au boîtier (10), au-dessus des ouvertures de canal (12).

16. Appareil selon la revendication 1, qui comprend un moyen d'isolation électrique (54) pour isoler le moyen à circuit électrique (20) sur le support et le fluide hydratant (22), le moyen d'isolation électrique (54) occupant l'espace intérieur (82) qui n'est pas déjà occupé par d'autres composants, et le moyen (54) d'isolation électrique étant placé dans le boîtier (10) par l'intermédiaire d'un trou (118) pratiqué dans le boîtier.

17. Appareil selon la revendication 1, dans lequel l'appareil est stérilisé par rayons gamma.

18. Appareil selon la revendication 16, dans lequel le moyen d'isolation électrique (54) est un polymère d'époxy durci.

19. Appareil selon la revendication 1, dans lequel le support non conducteur (112) présentant au moins une sonde électrochimique préconditionnée (18) et un moyen à circuit électrique (20) en contact électrique avec la sonde (18) pour transporter les impulsions électriques provenant de la sonde (18) est une plaque améliorée à circuit électronique de l'élément de sonde (14), qui présente un support non conducteur (112), un thermistor (138) et au moins une sonde d'analyte (18) qu'elle porte en positions proches l'une de l'autre sur le support (112) et un réchauffeur (174), également porté sur le support (112), pour fournir de la chaleur en réponse à une température détectée par ledit thermistor (138), au moins à la région dans laquelle ledit thermistor (138) et ladite sonde (18) de gaz sanguin sont disposés sur ladite plaque (14), pour ainsi contrôler la température dans ladite région de ladite plaque (14) à l'intérieur d'une étroite distribution de températures, et un moyen de connexion au moyen à circuit (20) porté sur ladite plaque, pour relier ladite plaque à une source électrique extérieure.

20. Appareil selon la revendication 19, dans lequel la plaque à circuit électronique améliorée comprend :
un support non conducteur (112) présentant un moyen à circuit électrique (20) comportant un ou plusieurs conducteurs conduisant et partant du moyen à circuit (20) disposé sur le support, un organe rigide en plastique couvrant au moins une partie d'au moins une surface du support (112), un adhésif en époxy durci aux UV étant en contact avec le plastique et le support (112), pour les fixer par collage.

21. Appareil selon la revendication 20, dans lequel l'adhésif en époxy durci aux UV présente un motif de contact obtenu en absorbant par effet de mèche l'adhésif en époxy durcissable sur une surface de contact.

22. Appareil selon la revendication 19, dans lequel ladite plaque (14) est fabriquée en recourant à une technique de circuit par stratification de films épais.

23. Appareil selon la revendication 19, dans lequel ledit thermistor (138) et lesdites sondes à gaz sanguin sont portés dans le même plan sur ladite plaque (14).

24. Appareil selon la revendication 19, dans lequel lesdites sondes (18) à gaz sanguin comprennent au moins l'une parmi une sonde d'oxygène, une sonde de dioxyde de carbone et une sonde de pH.

25. Appareil selon la revendication 19, dans lequel ledit moyen de connexion du moyen à circuit (20) comprend plusieurs conducteurs extérieurs (146-160), une résistance (176) étant portée sur ladite plaque (14), du même côté que ledit réchauffeur (174), et étant reliée en commun à l'un desdits conducteurs extérieurs (146-160), ledit thermistor (138) divisant la tension entre ceux-ci.

26. Appareil selon la revendication 19, dans lequel le coefficient de température dudit thermistor (138) est négatif ou positif, et le coefficient de température de ladite résistance (176) est essentiellement zéro, et lorsque la tension divisée est proportionnelle ou inversement proportionnelle à la température, cette sortie étant utilisée pour mesurer la température.

27. Appareil selon la revendication 25, dans lequel ledit moyen de connexion du moyen à circuit (20) comprend en outre plusieurs parcours électroniques conducteurs (118, 122, 128, 132, 138, 140) reliant individuellement et électriquement chacune desdites sondes (18) et ledit thermistor (138) aux conducteurs extérieurs (145-160) prévus sur lesdites plaques (14) à l'extrémité desdits parcours (118, 122, 128, 132, 138, 140), ladite résistance (176) et ledit réchauffeur (174) étant chacun reliés électriquement aux conducteurs extérieurs (146-160).

28. Appareil selon la revendication 19, dans lequel ledit réchauffeur (174) est alimenté par un courant continu pulsé, ledit réchauffeur (174) étant en permanence branché et débranché, pour ainsi éviter que ledit réchauffeur (174) dépasse par le haut ou par le bas une température prédéterminée.

29. Appareil selon la revendication 24, dans lequel lesdites sondes (18) à gaz sanguin sont calibrées par ajustement au laser de ladite résistance (176).

30. Appareil selon la revendication 29, dans lequel ladite sonde d'oxygène (124) est une cellule électrochimique et comprend une anode (126) et une cathode (134) qui sont chacune reliées à un conducteur extérieur (154).

31. Appareil selon la revendication 30, dans lequel ladite sonde d'oxygène (124) comprend une membrane perméable à l'oxygène recouvrant de manière étanche au liquide une ouverture de ladite plaque (14) qui contient un électrolyte, ladite anode (126) étant mise à la masse sur ladite plaque (14), pour ainsi assurer que le potentiel dudit électrolyte est le même que le potentiel d'anode.

32. Appareil selon la revendication 31, dans lequel il existe au moins une électrode de référence (64, 66), pour fournir un potentiel de référence précis, portée sur ladite plaque (14) et électriquement connectée à un parcours conducteur électronique (118, 122, 128, 132, 138, 140).

33. Appareil selon la revendication 32, dans lequel il existe une électrode de référence (64, 66) portée sur ladite plaque (14), et électriquement connectée à un parcours conducteur électronique (118, 122, 128, 132, 138, 140) partant de ladite anode (126).

34. Appareil selon la revendication 33, dans lequel ladite plaque (14) est une couche céramique (112) essentiellement plane, et comprend une couche métallique (114) configurée suivant un motif, formée sur ledit support (112) par dépôt d'une pâte métallique d'impression sur ledit support (112), pour former les parcours conducteurs électroniques (118, 122, 128, 132, 138, 140) et les électrodes desdites sondes (18), ainsi que l'électrode d'une électrode de référence (64, 66).

35. Appareil selon la revendication 34, dans lequel ladite couche métallique (114) est encapsulée au moyen d'au moins une couche d'un encapsulant (144) chimiquement stable et résistant à l'humidité.

36. Appareil selon la revendication 35, dans lequel ladite plaque (14) comprend un thermistor (138) prévu sur la couche de support céramique (112), ledit thermistor (138) étant encapsulé au moyen d'au moins une couche essentiellement mince d'un encapsulant (144) chimiquement stable et résistant à l'humidité.
